# EUROPEAN PATENT SPECIFICATION

(11) **EP 3 561 056 B1**
(45) Date of publication and mention of the grant of the patent: **25.10.2023**
(21) Application number: 17884396.7
(22) Date of filing: 21.12.2017
(51) Int. Cl.: C12N 15/09, A61K 38/55, A61K 45/00, A61K 45/06, A61P 9/00, A61P 19/02, A61P 27/02, A61P 29/00, A61P 43/00, C07K 16/38, C07K 19/00, C12N 1/15, C12N 1/19, C12N 1/21, C12N 5/10, C12P 21/02, C12Q 1/37

(54) **PEPTIDE FOR TREATING AGE-RELATED MACULAR DEGENERATION**
PEPTID ZUR BEHANDLUNG VON ALTERSBEDINGTER MAKULADEGENERATION
PEPTIDE POUR LE TRAITEMENT DE LA DÉGÉNÉRESCENCE MACULAIRE LIÉE À L'ÂGE

(30) Priority: 22.12.2016 JP 2016249020
(43) Date of publication of application: 30.10.2019
(73) Proprietor: Daiichi Sankyo Company, Limited, Tokyo 103-8426 (JP)
(72) Inventor: NISHIMIYA, Daisuke, Tokyo 103-8426 (JP); HASHIMOTO, Ryuji, Tokyo 103-8426 (JP); SATO, Toshiyuki, Tokyo 103-8426 (JP); KIMURA, Takako, Tokyo 134-8630 (JP); YAMASAKI, Atsushi, Tokyo 103-8426 (JP); INOUE, Tatsuya, Tokyo 103-8426 (JP)
(74) Representative: Marks & Clerk LLP
(86) International application number: PCT/JP2017/046044
(87) International publication number: WO 2018/117244

(56) References cited:
- WO-A1-2014/024914
- WO-A1-2014/024914
- WO-A2-2009/046405
- JP-A- 2006 515 836
- JP-A- 2009 544 317
- JP-A- 2014 515 012
- JP-A- 2015 501 149
- JP-A- 2016 513 109
- JP-A- 2016 513 125
- MARK E PENNESI ET AL: "Animal models of age related macular degeneration", MOLECULAR ASPECTS OF MEDICINE, vol. 33, no. 4, 11 September 2013 (2013-09-11), pages 487-509, XP028400783, ISSN: 0098-2997, DOI: 10.1016/J.MAM.2012.06.003 [retrieved on 2012-06-15]
- JOEL A. DIETZ ET AL: "Spink2 Modulates Apoptotic Susceptibility and Is a Candidate Gene in the Rgcs1 QTL That Affects Retinal Ganglion Cell Death after Optic Nerve Damage", PLOS ONE, vol. 9, no. 4, 3 April 2014 (2014-04-03), page e93564, XP55717943, DOI: 10.1371/journal.pone.0093564
- DATABASE Geneseq [Online] 10 April 2014 (2014-04-10), "Human SPINK2 gene, SEQ 14.", XP002799849, retrieved from EBI accession no. GSN:BBC39926 Database accession no. BBC39926
- Daisuke Nishimiya ET AL: "A protein scaffold, engineered SPINK2, for generation of inhibitors with high affinity and specificity against target proteases", Scientific Reports, VOL. 9, N.1, 7 August 2019 (2019-08-07), XP055707463, DOI: 10.1038/s41598-019-47615-5 Retrieved from the Internet: URL:http://www.nature.com/articles/s41598- 019-47615-5 [retrieved on 2020-06-22]
- ESPINOSA-HEIDMANN, DIEGO G. et al.: "Cigarette Smoke-Related Oxidants and the Development of Sub-RPE Deposits in an Experimental Animal Model of Dry AMD", Investigative Ophthalmology & Visual Science, vol. 47, no. 2, 1 February 2006 (2006-02-01), pages 729-737, XP055612342, ISSN: 1552-5783, DOI: 10.1167/iovs.05-0719

## Description

### Technical Field

The present invention relates to a peptide, a polynucleotide, a vector, a cell, a method for producing the peptide, a peptide obtained by the method, a composition comprising the peptide, a pharmaceutical composition comprising the peptide, the pharmaceutical composition for the treatment or prevention of various diseases comprising the peptide, use of the peptide for the treatment or prevention of various diseases, a method for treating various diseases comprising the step of administering the peptide, etc.

### Background Art

High temperature requirement A serine peptidase 1 (HTRA1) is a trypsin-like serine protease (PRSS11; Clan PA, family S1) and is constituted by an N-terminal domain consisting of an IGFBP-like module and a Kazal-like module, a protease domain and a C-terminal PDZ domain. HTRA1 belongs to the HTRA family including HTRA2, HTRA3, and HTRA4 and reversibly exhibits active and inactive structures, in the same way as the other HTRA molecules (Non Patent Literature 1 and 2). Its expression is maldistributed in the human body and found at relatively high levels in the cartilage, the synovial membrane, the placenta, and the like. It is known that HTRA1 cleaves many extracellular matrix constituents such as amyloid precursor protein, fibromodulin, clusterin, ADAM9, and vitronectin as substrates and is related to diseases typified by arthritis and bone calcification (Non Patent Literature 3, 4, 5, and 6). It is further known that if a HTRA1 promoter region has a gene polymorphism (rs11200638), HTRA1 transcription level is elevated. Genome-wide association analysis has also revealed that the polymorphism correlates strongly with age-related macular degeneration (hereinafter, referred to as AMD) (Non Patent Literature 7 and 8).

AMD is a chronic degenerative disease with aging and is characterized by loss of central vision. This disease is the leading cause of acquired blindness in the USA and is the fourth most common cause of acquired blindness following glaucoma, diabetic retinopathy, and retinitis pigmentosa in Japan (Non Patent Literature 12). The mRNA and protein levels of HTRA1 are elevated in the lymphocytes or retinal pigment epithelial cells of AMD patients (Non Patent Literature 13). It has also been reported that the expression of the HTRA1 protein is elevated in drusen or degenerated retinal pigment epithelial cells which are precursor lesions of AMD, or neovascular membranes (Non Patent Literature 11 and 14 to 16). It has also been reported that the HTRA1 protein is detected in the vitreous humor in association with retinal detachment, retinal vein occlusion, vitreous hemorrhage, macular hole, and the like, and its value is synchronized with an angiogenesis marker VEGF (Non Patent Literature 17). Furthermore, the decomposition of proteins constituting basement membrane, such as fibulin 5 or tropoelastin, and the fragmentation of the elastic layer of Bruch's membrane have been observed in HTRA1 transgenic mice (Non Patent Literature 9). However, it has not been directly shown that the inhibition of the protease activity of HTRA1 is effective or is not effective for treating the diseases described above and for protecting the retina.

SPINK2 (serine protease inhibitor Kazal-type 2) is a Kazal-like domain having three disulfide bonds and functions as a trypsin/acrosin inhibitor (Non Patent Literature 10). However, its relation to AMD has not yet been elucidated.

Mouse and rabbit models are known as animal models for evaluating AMD (Non Patent Literature 18, 19, and 20). As for rabbits, which are more preferred as models that can be extrapolated to human eye diseases (Non Patent Literature 21), the conventional models are used merely for observing abnormal findings in the retina and the choroid and have the difficulty of evaluating the abnormal functions of retinal pigment epithelial cells (RPE cells), etc. A further problem thereof is a period as long as 8 months required for model formation (Non Patent Literature 20).

### Citation List

### Non Patent Literature

Non Patent Literature 1: Truebestein L, et al., 2011, Nat Struct Mol Biol., Vol. 18 (No. 3): p. 386-8
Non Patent Literature 2: Eigenbrot C, et al., 2012, Structure, Vol. 20 (No. 6): p. 1040-50
Non Patent Literature 3: Grau S, et al., 2005, Proc Natl Acad Sci U S A., Vol. 102 (No. 17): p. 6021-26
Non Patent Literature 4: Grau S, et al., 2006, J Biol Chem., Vol. 281 (No. 10): p. 6124-29
Non Patent Literature 5: Hadfield KD, et al., 2008, J Biol Chem., Vol. 283, (No. 9): p. 5928-38
Non Patent Literature 6: An E, et al., 2010, Invest Ophthalmol Vis Sci., Vol. 51 (No. 7): p. 3379-86
Non Patent Literature 7: Yang Z, et al., 2006, Science, Vol. 314 (No. 5801): p. 992-93
Non Patent Literature 8: Tang NP, et al., 2009, Ann Epidemiol., Vol. 19 (No. 10): p. 740-45
Non Patent Literature 9: Vierkotten S, et al., 2011, PLoS One, Vol. 6 (No. 8): p. e22959
Non Patent Literature 10: Chen T, et al., 2009, Proteins, Vol. 77 (No. 1): p. 209-19
Non Patent Literature 11: Yang Z et al., Science, 2006, Vol. 314, No. 5801: p. 992-93
Non Patent Literature 12: Kimihiro Nakae, et al., 2007 Annual report of the Research Committee on Chorioretinal Degenerations and Optic Atrophy, Research on Measures for Intractable Diseases, the Ministry of Health, Labour and Welfare of Japan (2007)
Non Patent Literature 13: Black JR and Clark SJ, Genet. Med., 2016, Vol. 18, No. 4: p. 283-89
Non Patent Literature 14: Cameron DJ, et al., Cell Cycle, 2007, Vol. 6, No. 9: p. 1122-25
Non Patent Literature 15: Chan CC et al., Trans. Am. Soc., 2007, Vol. 105: p. 92-97
Non Patent Literature 16: Tuo J et al., 2008, Vol. 115, No. 11: p. 1891-98
Non Patent Literature 17: Ng TK et al., Invest. Ophthalmol. Vis. Sci, 2011, Vol. 52, No. 6: p. 3706-12
Non Patent Literature 18: Espinosa-Haidemann D.G, et al., Investigative Ophthalmology & Visual Science, 2006, Vol. 47 (No. 2): p. 729-37
Non Patent Literature 19: Pons M, et al., American Journal of Pathology, 2010, Vol. 177: p. 1198-1213
Non Patent Literature 20: Trivino A, et al., Experimental Eye Research, 2006, Vol. 83: p. 357-366
Non Patent Literature 21: Zernii E.Y, et al., CNS & Neurological Disorders-Drug Targets, 2016, Vol. 15: p. 267-291

### Summary of Invention

### Technical Problem

An object of the present invention is to provide a novel high temperature requirement A serine peptidase 1 (HTRA1) inhibitor.

### Solution to Problem

The present invention is defined as recited in the claims.

### Advantageous Effects of Invention

The peptide provided by the present invention, and a pharmaceutical composition comprising the peptide have HTRA1 inhibitory activity and are useful in the treatment or prevention, etc. of age-related macular degeneration.

### Brief Description of Drawings

[Figure 1(A)] Figure 1(A) is a diagram showing the results of comparing sequence similarity among human, mouse, rat, and monkey HTRA1. The broken line depicts an enzymatically active domain (204Gly to 364Leu).
[Figure 1(B)] Figure 1(B) is a diagram showing results of comparing sequence similarity among human, mouse, rat, and monkey HTRA1 (Cont.).
[Figure 2] Figure 2 is a diagram showing the results of evaluating the HTRA1 (cat) inhibitory activity of a HTRA1-inhibiting peptide by using the decomposition rate of a peptide substrate as an indicator. Panels A to C each show the evaluation results for each inhibiting peptide, and panel D shows the evaluation results for control wild-type SPINK2.
[Figure 3] Figure 3 is a diagram showing the results of evaluating the HTRA1 (full) inhibitory activity of a HTRA1-inhibiting peptide by using the decomposition rate of a peptide substrate as an indicator (panels A to C).
[Figure 4(A)] Figure 4(A) is a diagram showing the results of evaluating the HTRA1 (cat) inhibitory activity of a HTRA1-inhibiting peptide by using the decomposition of human vitronectin as an indicator. Analysis was conducted by Western blot using Human Vitronectin Antibody (R&D Systems, Inc.; MAB2349).
[Figure 4(B)] Figure 4(B) is a diagram showing the results of evaluating the HTRA1 (cat) inhibitory activity of a HTRA1-inhibiting peptide by using the decomposition of human vitronectin as an indicator (Cont.).
[Figure 5(A)] Figure 5(A) is a diagram showing the results of evaluating the cross-reactivity of a HTRA1-inhibiting peptide with each protease by using the decomposition of a peptide substrate as an indicator (part 1). For the name of each protease used and its concentration and the name of the substrate and its concentration, etc., see Example 3.
[Figure 5(B)] Figure 5(B) is a diagram showing the results of evaluating the cross-reactivity of a HTRA1-inhibiting peptide with each protease by using the decomposition of a peptide substrate as an indicator (part 2).
[Figure 5(C)] Figure 5(C) is a diagram showing the results of evaluating the cross-reactivity of a HTRA1-inhibiting peptide with each protease by using the decomposition of a peptide substrate as an indicator (part 3).
[Figure 5(D)] Figure 5(D) is a diagram showing the results of evaluating the cross-reactivity of a HTRA1-inhibiting peptide with each protease by using the decomposition of a peptide substrate as an indicator (part 4).
[Figure 6] Figure 6 is a diagram showing a HTRA1 (cat)/HTRA1-inhibiting peptide complex obtained by X-ray crystallography. The inhibiting peptide is bound to each molecule of a HTRA1 trimer formed by HTRA1 (cat).
[Figure 7] Figure 7 is a diagram showing a HTRA1 (cat)/HTRA1-inhibiting peptide complex obtained by X-ray crystallography as a monomer. The inhibiting peptide is bound to a region containing the active center of HTRA1 (cat).
[Figure 8] Figure 8 shows the amino acid sequence (SEQ ID NO: 54) of H2-Opt. N-terminal "Mca-I" means N-(4-methylcoumaryl-7-amide)-isoleucine, and C-terminal "(Dnp)K" means N epsilon-(2,4-dinitrophenyl)-lysine.
[Figure 9] Figure 9 is a diagram showing that the expression of HTRA1 was increased in the vitreous humor of a rat model of retinal damage induced by light exposure. Analysis was conducted by Western blot using Human HTRA1/PRSS11 Antibody (R&D Systems, Inc.; AF2916).
[Figure 10] Figure 10 is a diagram showing that a HTRA1-inhibiting peptide administration group of rat models of retinal damage induced by light exposure suppressed a decrease in nucleus count in an outer nuclear layer on a cross-section of the retina. n = 4 for a normal saline administration group, and n = 5 for the other groups.
[Figure 11] Figure 11 is a diagram showing the results of evaluating the HTRA1 (cat) inhibitory activity of five HTRA1-inhibiting peptide derivatives by using the decomposition rate of a peptide substrate as an indicator.

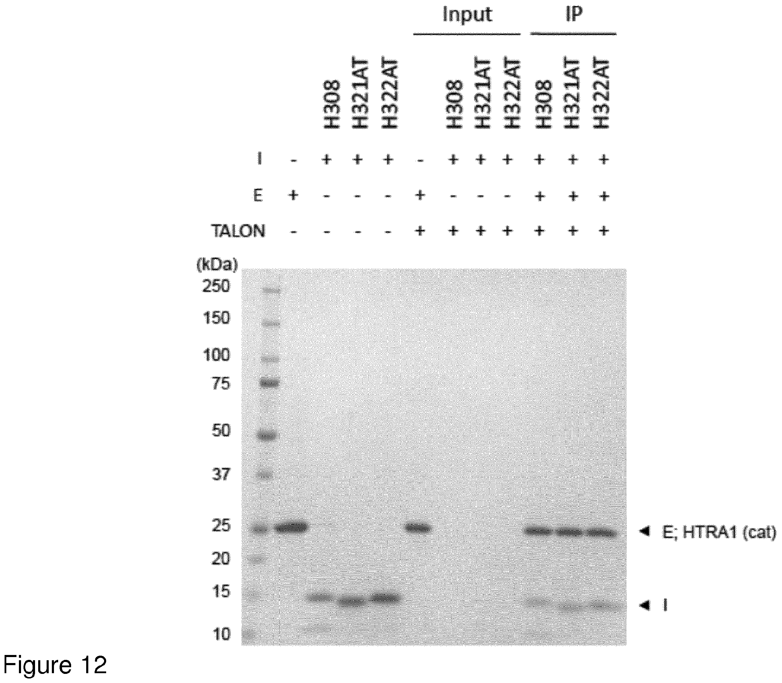

[Figure 12] Figure 12 is a diagram showing the results of evaluating the binding of three HTRA1-inhibiting peptides to HTRA1 (cat) by an immunoprecipitation method.
[Figure 13] Figure 13 shows the amino acid sequence (SEQ ID NO: 1) of human SPINK2.
[Figure 14] Figure 14 shows a nucleotide sequence (SEQ ID NO: 2) encoding the amino acid sequence of human SPINK2.
[Figure 15] Figure 15 shows the amino acid sequence (SEQ ID NO: 3) of peptide H218.
[Figure 16] Figure 16 shows a nucleotide sequence (SEQ ID NO: 4) encoding the amino acid sequence of peptide H218.
[Figure 17] Figure 17 shows the amino acid sequence (SEQ ID NO: 5) of peptide H223.
[Figure 18] Figure 18 shows a nucleotide sequence (SEQ ID NO: 6) encoding the amino acid sequence of peptide H223.
[Figure 19] Figure 19 shows the amino acid sequence (SEQ ID NO: 7) of peptide H228.
[Figure 20] Figure 20 shows a nucleotide sequence (SEQ ID NO: 8) encoding the amino acid sequence of peptide H228.
[Figure 21] Figure 21 shows the amino acid sequence (SEQ ID NO: 9) of peptide H308.
[Figure 22] Figure 22 shows a nucleotide sequence (SEQ ID NO: 10) encoding the amino acid sequence of peptide H308.
[Figure 23] Figure 23 shows the amino acid sequence (SEQ ID NO: 11) of peptide H321.
[Figure 24] Figure 24 shows a nucleotide sequence (SEQ ID NO: 12) encoding the amino acid sequence of peptide H321.
[Figure 25] Figure 25 shows the amino acid sequence (SEQ ID NO: 13) of peptide H322.
[Figure 26] Figure 26 shows a nucleotide sequence (SEQ ID NO: 14) encoding the amino acid sequence of peptide H322.
[Figure 27] Figure 27 shows the amino acid sequence (SEQ ID NO: 15) of peptide derivative H308AT.
[Figure 28] Figure 28 shows a nucleotide sequence (SEQ ID NO: 16) encoding the amino acid sequence of peptide derivative H308AT.
[Figure 29] Figure 29 shows the amino acid sequence (SEQ ID NO: 17) of peptide derivative H321AT.
[Figure 30] Figure 30 shows a nucleotide sequence (SEQ ID NO: 18) encoding the amino acid sequence of peptide derivative H321AT.
[Figure 31] Figure 31 shows the amino acid sequence (SEQ ID NO: 19) of peptide derivative H322AT.
[Figure 32] Figure 32 shows a nucleotide sequence (SEQ ID NO: 20) encoding the amino acid sequence of peptide derivative H322AT.
[Figure 33] Figure 33 shows the amino acid sequence (SEQ ID NO: 21) of peptide M7.
[Figure 34] Figure 34 shows a nucleotide sequence (SEQ ID NO: 22) encoding the amino acid sequence of peptide M7.
[Figure 35] Figure 35 shows the amino acid sequence (SEQ ID NO: 23) of peptide derivative H308_S16A.
[Figure 36] Figure 36 shows the amino acid sequence (SEQ ID NO: 24) of peptide derivative H308_D1G_S16A.
[Figure 37] Figure 37 shows the amino acid sequence (SEQ ID NO: 25) of peptide derivative H308_D1S_S16A.
[Figure 38] Figure 38 shows the amino acid sequence (SEQ ID NO: 26) of peptide derivative H308_D1E_S16A.
[Figure 39] Figure 39 shows the amino acid sequence (SEQ ID NO: 27) of peptide derivative H308_D1SLI_S16A.
[Figure 40] Figure 40 shows the amino acid sequence (SEQ ID NO: 28) of peptide derivative H321AT_D1S_S16A.
[Figure 41] Figure 41 shows the amino acid sequence (SEQ ID NO: 29) of peptide derivative H322AT_D1S_S16A.
[Figure 42] Figure 42 shows the general formula (SEQ ID NO: 30) of a HTRA1-inhibiting peptide. X₁ to X₁₁ each represent an arbitrary amino acid.
[Figure 43] Figure 43 shows an amino acid sequence (SEQ ID NO: 31) consisting of S tag and a linker.
[Figure 44] Figure 44 shows the amino acid sequence (SEQ ID NO: 32) of a C-terminal hexamer.
[Figure 45] Figure 45 shows the nucleotide sequence (SEQ ID NO: 33) of primer 1.
[Figure 46] Figure 46 shows the nucleotide sequence (SEQ ID NO: 34) of primer 2.
[Figure 47] Figure 47 shows the nucleotide sequence (SEQ ID NO: 35) of primer 3.
[Figure 48] Figure 48 shows the nucleotide sequence (SEQ ID NO: 36) of primer 4.
[Figure 49] Figure 49 shows the nucleotide sequence (SEQ ID NO: 37) of primer 5.
[Figure 50] Figure 50 shows the nucleotide sequence (SEQ ID NO: 38) of primer 6.
[Figure 51] Figure 51 shows the nucleotide sequence (SEQ ID NO: 39) of primer 7.
[Figure 52] Figure 52 shows the nucleotide sequence (SEQ ID NO: 40) of primer 8.
[Figure 53] Figure 53 shows the nucleotide sequence (SEQ ID NO: 41) of primer 9.
[Figure 54] Figure 54 shows the nucleotide sequence (SEQ ID NO: 42) of primer 10.
[Figure 55] Figure 55 shows the nucleotide sequence (SEQ ID NO: 43) of primer 11.
[Figure 56] Figure 56 shows the nucleotide sequence (SEQ ID NO: 44) of primer 12.
[Figure 57] Figure 57 shows the nucleotide sequence (SEQ ID NO: 45) of primer 13.
[Figure 58] Figure 58 shows the nucleotide sequence (SEQ ID NO: 46) of primer 14.
[Figure 59] Figure 59 shows the nucleotide sequence (SEQ ID NO: 47) of primer 15.
[Figure 60] Figure 60 shows the nucleotide sequence (SEQ ID NO: 48) of primer 16.
[Figure 61] Figure 61 shows the nucleotide sequence (SEQ ID NO: 49) of primer 17.
[Figure 62] Figure 62 shows the nucleotide sequence (SEQ ID NO: 50) of primer 18.
[Figure 63] Figure 63 shows the nucleotide sequence (SEQ ID NO: 51) of primer 19.
[Figure 64] Figure 64 shows the nucleotide sequence (SEQ ID NO: 52) of primer 20.
[Figure 65] Figure 65 shows the amino acid sequence (SEQ ID NO: 53) of human HTRA1 (full).
[Figure 66(A)] Figure 66(A) is a diagram showing the results of evaluating the HTRA1 (cat) inhibitory activity of HTRA1-inhibiting peptides by using the decomposition rate of a peptide substrate as an indicator.
[Figure 66(B)] Figure 66(B) is a diagram showing the results of evaluating the HTRA1 (full) inhibitory activity of a HTRA1-inhibiting peptide by using the decomposition rate of a peptide substrate as an indicator.
[Figure 67] Figure 67 is a diagram showing results of evaluating the HTRA1 (cat) inhibitory activity of a HTRA1-inhibiting peptide by using the decomposition of human vitronectin as an indicator. Analysis was conducted by Western blot using Human Vitronectin Antibody (R&D Systems, Inc.; MAB2349).
[Figure 68(A)] Figure 68(A) is a diagram showing the results of evaluating the cross-reactivity of HTRA1-inhibiting peptides with each protease by using the decomposition of a peptide substrate as an indicator (part 1).
[Figure 68(B)] Figure 68(B) is a diagram showing the results of evaluating the cross-reactivity of a HTRA1-inhibiting peptide with each protease by using the decomposition of a peptide substrate as an indicator (part 2).
[Figure 68(C)] Figure 68(C) is a diagram showing the results of evaluating the cross-reactivity of a HTRA1-inhibiting peptide with each protease by using the decomposition of a peptide substrate as an indicator (part 3).
[Figure 68(D)] Figure 68(D) is a diagram showing results of evaluating the cross-reactivity of a HTRA1-inhibiting peptide with each protease by using the decomposition of a peptide substrate as an indicator (part 4).
[Figure 68(E)] Figure 68(E) is a diagram showing the results of evaluating the cross-reactivity of a HTRA1-inhibiting peptide with each protease by using the decomposition of a peptide substrate as an indicator (part 5).
[Figure 69] Figure 69 is a diagram showing the results of evaluating the binding of three HTRA1-inhibiting peptides to HTRA1 (cat) by an immunoprecipitation method.
[Figure 70(A)] Figure 70(A) is a diagram showing that a HTRA1-inhibiting peptide H308_D1G_S16A administration group of rat models of retinal damage induced by light exposure suppressed a decrease in nucleus count in an outer nuclear layer on a cross-section of the retina. n = 6 for all groups. The dose of the HTRA1-inhibiting peptide H308_D1G_S16A was 0.2 and 1 µg/eye.
[Figure 70(B)] Figure 70(B) is a diagram showing that a HTRA1-inhibiting peptide H321AT_D1G_S16A administration group of rat models of retinal damage induced by light exposure suppressed a decrease in nucleus count in an outer nuclear layer on a cross-section of the retina. n = 6 for all groups. The dose of the HTRA1-inhibiting peptide H321AT_D1G_S16A was 0.2 and 1 µg/eye.
[Figure 70(C)] Figure 70(C) is a diagram showing that a HTRA1-inhibiting peptide H322AT_D1G_S16A administration group of rat models of retinal damage induced by light exposure suppressed a decrease in nucleus count in an outer nuclear layer on a cross-section of the retina. n = 6 for all groups. The dose of the HTRA1-inhibiting peptide H322AT_D1G_S16A was 0.2 and 1 µg/eye.
[Figure 71(A)] Figure 71(A) is a diagram showing the results of immunostaining RPE cells in a 12-week-old rabbit, a 3-year-old rabbit, and a HFD-HQ-loaded 3-year-old rabbit with a ZO-1 antibody (Thermo Fisher Scientific Inc.) .
[Figure 71(B)] Figure 71(B) shows the average areas of RPE cells in a 12-week-old rabbit, a 3-year-old rabbit, and a HFD-HQ-loaded 3-year-old rabbit.
[Figure 71(C)] Figure 71(C) is a diagram showing the increased expression of the mRNA of complement component 3 "C3", which is an AMD-related factor, in the retinal tissue of a HFD-HQ-loaded 3-year-old rabbit.
[Figure 71(D)] Figure 71(D) is a diagram showing the increased expression of the mRNA of complement component 3 "C3", which is an AMD-related factor, in the RPE/choroid tissue of a HFD-HQ-loaded 3-year-old rabbit.
[Figure 71(E)] Figure 71(E) is a diagram showing results of measuring a HTRA1 concentration in the vitreous humor of a 12-week-old rabbit, a 3-year-old rabbit, and a HFD-HQ-loaded 3-year-old rabbit by LC-MS/MS.
[Figure 72(A)] Figure 72(A) is a diagram showing that a HTRA1-inhibiting peptide H308 administration group of HFD-HQ-loaded 3-year-old rabbits exhibited a suppressive effect on the hypertrophy of RPE cells. n = 5 for all groups. An average area was used as an indicator.
[Figure 72(B)] Figure 72(B) is a diagram showing that a HTRA1-inhibiting peptide H308 administration group of HFD-HQ-loaded 3-year-old rabbits exhibited a suppressive effect on the hypertrophy of RPE cells. n = 5 for all groups. The number of RPE cells having a cell area of 1500 µm² or larger was used as an indicator.

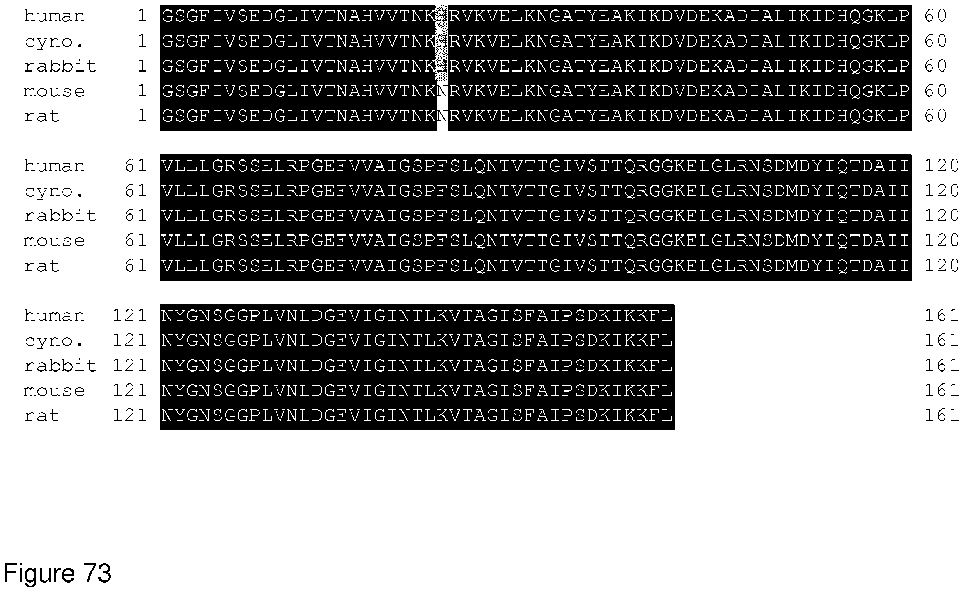

[Figure 73] Figure 73 is a diagram showing the results of comparing sequence similarity among human, monkey, rabbit, mouse, and rat HTRA1. The broken line depicts an enzymatically active domain (204Gly to 364Leu).
[Figure 74] Figure 74 is a diagram showing that a HTRA1-inhibiting peptide exhibited a suppressive effect on VEGF mRNA induced in a human retinal pigment epithelial cell line ARPE-19 by the addition of H₂O₂, normal human serum complement, and HTRA1.
[Figure 75] Figure 75 is a diagram showing that a HTRA1-inhibiting peptide exhibited a suppressive effect on the migration of human umbilical vein endothelial cells (HUVEC) induced by serum.
[Figure 76] Figure 76 shows the nucleotide sequence of primer 21.
[Figure 77] Figure 77 shows the nucleotide sequence of primer 22.

In the present invention, the phrase "SEQ ID NO: X (Figure Y)" or "Figure Y (SEQ ID NO: X)" means that the sequence is shown in SEQ ID NO: X or shown in Figure Y.

### Description of Embodiments

### 1. Definition

In the present invention, the term "gene" is used to mean a nucleic acid molecule comprising a nucleotide sequence encoding an amino acid sequence contained in a protein, or a complementary strand thereof. The gene consists of a single strand, a double strand or a triple or more strand. An association of a DNA strand and an RNA strand, ribonucleotides and deoxyribonucleotides coexisting on one strand, and a double-stranded or triple- or more stranded nucleic acid molecule including such a strand are also included in the meaning of the "gene".

In the present invention, the terms "gene", "polynucleotide" and "nucleic acid molecule" are synonymous with each other and are not limited by the number of their constituent units such as ribonucleotides, deoxyribonucleotides, nucleotides, and nucleosides by any means. For example, DNA, RNA, mRNA, cDNA, cRNA, a probe, an oligonucleotide, a primer, and the like are also included in the scope thereof. The term "nucleic acid molecule" is also abbreviated to a "nucleic acid".

In the present invention, the terms "polypeptide", "peptide" and "protein" are synonymous with each other. A peptide that inhibits or suppresses one or two or more activities or functions of target molecule X (hereinafter, these inhibitory or suppressive effects are collectively referred to as "X inhibitory activity") can be referred to as an "X-inhibiting peptide".

The term "SPINK2" is used to mean serine protease inhibitor Kazal-type 2. This protein of 7 kDa consists of a Kazal-like domain having three disulfide bonds. SPINK2 is preferably human-derived. In the present invention, human SPINK2 is simply referred to as "SPINK2", unless otherwise specified.

The term "HTRA1" is used to mean high temperature requirement A serine peptidase 1. This protein is constituted by an N-terminal domain consisting of an IGFBP-like module and a Kazal-like module, a protease domain and a C-terminal PDZ domain and belongs to the HTRA family. HTRA1 is preferably human-derived. In the present invention, human HTRA1 is also simply referred to as "HTRA1", unless otherwise specified.

The term "HTRA1-inhibiting peptide" is used to mean a peptide that inhibits or suppresses one or two or more activities or functions of HTRA1. A fragment of the peptide, an adduct of the peptide with an additional moiety, or a conjugate of the peptide that maintains HTRA1 inhibitory activity is included in the scope of the term "HTRA1-inhibiting peptide". Specifically, a fragment, an adduct and a modified form of the peptide that maintain HTRA1 inhibitory activity are also included within the term "HTRA1-inhibiting peptide".

In the present invention, the term "cell" is used to include various cells derived from animal individuals, subcultured cells, primary cultured cells, cell lines, recombinant cells, yeasts, microbes and the like.

In the present invention, the term "site" to which a peptide binds, i.e., the "site" that is recognized by a peptide is used to mean a consecutive or intermittent partial amino acid sequence or partial conformation on a target molecule to be bound or recognized by a peptide. In the present invention, such a site can be referred to as an epitope or a binding site on the target molecule.

The term "SPINK2 mutant" is used to mean a peptide comprising an amino acid sequence derived from the amino acid sequence of wild-type SPINK2 by the substitution of one or two or more amino acids by amino acids different from the wild-type ones, the deletion of one or two or more wild-type amino acids, the insertion of one or two or more amino acids absent in the wild type, and/or the addition of amino acid(s) absent in the wild type to the amino terminus (N terminus) and/or carboxyl terminus (C terminus) of the wild type (hereinafter, collectively referred to as a "mutation"). A "SPINK2 mutant" having HTRA1 inhibitory activity is included in the HTRA1-inhibiting peptide. In the present invention, the "insertion" can also be included in the "addition".

In the present invention, the term "several" in the phrase "one or several" refers to 3 to 10.

In the present invention, the phrase "to hybridize under stringent conditions" is used to mean that hybridization is carried out under conditions in which hybridization is carried out at 65°C in a solution containing 5 × SSC, and the resultant is then washed at 65°C for 20 minutes in an aqueous solution containing 2 × SSC and 0.1% SDS, at 65°C for 20 minutes in an aqueous solution containing 0.5 × SSC and 0.1% SDS, and at 65°C for 20 minutes in an aqueous solution containing 0.2 × SSC and 0.1% SDS, or conditions equivalent thereto. SSC means an aqueous solution of 150 mM NaCl and 15 mM sodium citrate, and n × SSC means an n-fold concentration of SSC.

In the present invention, the terms "specific" and "specificity" are synonymous and interchangeable with the terms "selective" and "selectivity", respectively. For example, a HTRA1-specific inhibiting peptide is synonymous with a HTRA1-selective inhibiting peptide.

### 2. Peptide

### 2-1. Amino acid

The term "amino acid" is used to mean an organic compound containing an amino group and a carboxyl group and to mean an α-amino acid contained as a constituent unit preferably in a protein, and more preferably in a natural protein. In the present invention, the amino acid is more preferably Ala, Arg, Asn, Asp, Cys, Gln, Glu, Gly, His, Ile, Leu, Lys, Met, Phe, Pro, Ser, Thr, Trp, Tyr and Val. The term "amino acid" is used to mean these 20 amino acids in total, unless otherwise specified. These 20 amino acids in total can be referred to as "natural amino acids". The HTRA1-inhibiting peptide of the present invention preferably contains natural amino acids.

In the present invention, the term "amino acid residue" is also referred to as an "amino acid".

In the present invention, the amino acid may be an L-amino acid, a D-amino acid, or a mixture thereof (DL-amino acid) and means an L-amino acid, unless otherwise specified.

Natural amino acids can be divided into, for example, the following groups, on the basis of the common properties of side chains:
(1) hydrophobic amino acid group: Met, Ala, Val, Leu, and Ile;
(2) neutral hydrophilic amino acid group: Cys, Ser, Thr, Asn, and Gln;
(3) acidic amino acid group: Asp and Glu;
(4) basic amino acid group: His, Lys, and Arg;
(5) group of amino acids influencing the direction of the main chain: Gly and Pro; and
(6) aromatic amino acid group: Trp, Tyr, and Phe.

However, the classification of natural amino acids is not limited thereto.

In the present invention, each natural amino acid may receive a conservative amino acid substitution.

The "conservative amino acid substitution" means a substitution by a functionally equivalent or similar amino acid. The conservative amino acid substitution in a peptide brings about static change in the amino acid sequence of the peptide. For example, one or two or more amino acids substitutions having similar polarity act functionally equivalently and bring about static change in the amino acid sequence of the peptide. In general, a substitution within a certain group can be regarded as being conservative in terms of structures and functions. However, as is obvious to a person skilled in the art, the role of a specific amino acid residue can be determined by its position in the three-dimensional structure of a molecule containing the amino acid. For example, a cysteine residue can take an oxidized (disulfide) form having lower polarity than that of a reduced (thiol) form. The long aliphatic moiety of an arginine side chain is capable of constituting structurally and functionally important features. Alternatively, a side chain containing an aromatic ring (tryptophan, tyrosine, and phenylalanine) is capable of contributing to ion-aromatic interaction or cation-pi interaction. In such a case, even the substitution of amino acids having these side chains with amino acids belonging to an acidic or nonpolar group can be structurally and functionally conservative. Residues such as proline, glycine, and cysteine (disulfide form) might have a direct effect on the three-dimensional structure of the main chain and cannot often be substituted without structural distortion.

The conservative amino acid substitution includes, as shown below, a specific substitution based on side chain similarity (L. Lehninger, Biochemistry, 2nd edition, pp. 73-75, Worth Publisher, New York (1975)) and a typical substitution.
(1) Nonpolar amino acid group: alanine (hereinafter, referred to as "Ala" or simply "A"), valine (hereinafter, referred to as "Val" or simply "V"), leucine (hereinafter, referred to as "Leu" or simply "L"), isoleucine (hereinafter, referred to as "Ile" or simply "I"), proline (hereinafter, referred to as "Pro" or simply "P"), phenylalanine (hereinafter, referred to as "Phe" or simply "F"), tryptophan (hereinafter, referred to as "Trp" or simply "W"), and methionine (hereinafter, referred to as "Met" or simply "M")
(2) Uncharged polar amino acid group: glycine (hereinafter, referred to as "Gly" or simply "G"), serine (hereinafter, referred to as "Ser" or simply "S"), threonine (hereinafter, referred to as "Thr" or simply "T"), cysteine (hereinafter, referred to as "Cys" or simply "C"), tyrosine (hereinafter, referred to as "Tyr" or simply "Y"), asparagine (hereinafter, referred to as "Asn" or simply "N"), and glutamine (hereinafter, referred to as "Gln" or simply "Q")
(3) Acidic amino acid group: aspartic acid (hereinafter, referred to as "Asp" or simply "D") and glutamic acid (hereinafter, referred to as "Glu" or simply "E")
(4) Basic amino acid group: lysine (hereinafter, referred to as "Lys" or simply "K"), arginine (hereinafter, referred to as "Arg" or simply "R"), and histidine (hereinafter, referred to as "His" or simply "H")

In the present invention, amino acids may be amino acids other than the natural amino acids. Examples thereof can include selenocysteine, N-formylmethionine, pyrrolysine, pyroglutamic acid, cystine, hydroxyproline, hydroxylysine, thyroxine, O-phosphoserine, desmosine, β-alanine, sarcosine, ornithine, creatine, γ-aminobutyric acid, opine, theanine, tricholomic acid, kainic acid, domoic acid, and acromelic acid, which are found in natural peptides or proteins, and can include: N-terminally protected amino acids such as norleucine, Ac-amino acid, Boc-amino acid, Fmoc-amino acid, Trt-amino acid, and Z-amino acid; C-terminally protected amino acids such as t-butyl ester, benzyl ester, cyclohexyl ester, and fluorenyl ester of amino acids; and other amino acids that are not found in the natural world, including diamine, ω amino acid, β amino acid, γ amino acid, Tic derivatives of amino acids, and aminophosphonic acid. The amino acids other than the 20 "natural amino acids" are not limited thereto and are collectively referred to as "non-natural amino acids" in the present invention for the sake of convenience.

### 2-2. HTRA1-inhibiting peptide

The HTRA1-inhibiting peptide of the present invention is a SPINK2 mutant that at least partially maintains the framework of SPINK2 (hereinafter, referred to as a "SPINK2 mutant") and inhibits or suppresses the protease activity of HTRA1 or a fragment that retains its enzyme activity (hereinafter, referred to as a "functional fragment") (hereinafter, such inhibition and suppression are collectively referred to as "HTRA1 inhibitory activity").

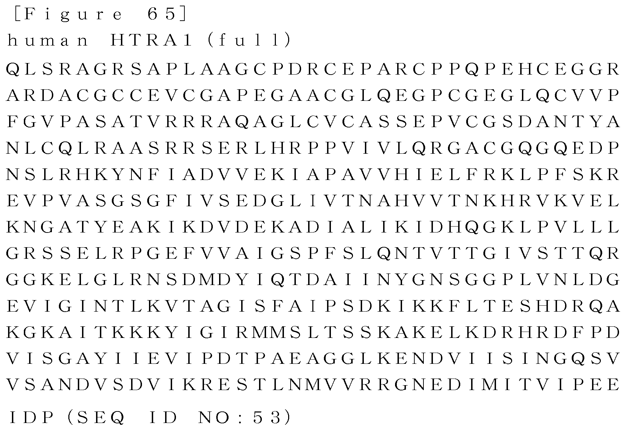

HTRA1, which is a target of the inhibiting peptide of the present invention, is preferably mammalian HTRA1, more preferably primate HTRA1, and still more preferably human HTRA1. The amino acid sequence of full-length mature human HTRA1 (hereinafter, referred to as "HTRA1 (full)") has the amino acid sequence shown in SEQ ID NO: 53 (Figure 65). This amino acid sequence consists of positions 23 to 480 and is free of the signal sequence consisting of positions 1 to 22. The amino acid sequence of the functional fragment of human HTRA1 (hereinafter, referred to as "HTRA1 (cat)") is not particularly limited as long as the functional fragment retains the protease activity. Examples thereof can include a functional fragment consisting of 158Gly to 373Lys of SEQ ID NO: 53 (Figure 65), and a functional fragment comprising 158Gly to 373Lys thereof. HTRA1 or the functional fragment thereof which is a target of the inhibiting peptide of the present invention is also referred to as HTRA1 protease. The HTRA1 protease is preferably vertebrate-derived, more preferably mammal-derived, still more preferably primate-derived, and most preferably human-derived, and can be prepared by purification from the tissues or cells of any of these animals, or by a method known to a person skilled in the art as a protein preparation method such as gene recombination, *in vitro* translation, or peptide synthesis. HTRA1 or the functional fragment thereof may be linked to a signal sequence, an immunoglobulin Fc region, a tag, a label, or the like.

HTRA1 inhibitory activity can be evaluated by using the protease activity of HTRA1 as an indicator. For example, HTRA1 or the functional fragment thereof, a substrate and the inhibiting peptide of the present invention or a candidate thereof are allowed to coexist with each other. In this case, when the protease activity of HTRA1 is 70% or less, 50% or less, 30% or less, 20% or less, 10% or less, 5% or less, 1% or less or 0% as compared with that in the presence of a control or in the absence of the inhibitor or the candidate thereof, the inhibition of HTRA1 occurs with inhibitory activity of 30% or more, 50% or more, 70% or more, 80% or more, 90% or more, 95% or more, 99% or more or 100%, respectively. The HTRA1 inhibitory activity can differ depending on reaction conditions, the type of substrate, concentration, etc. Examples of the reaction conditions can include, but are not limited to, those described in the Examples. The enzyme activity can be evaluated by adding a substrate peptide or a substrate protein to a given concentration of HTRA1, and reacting the mixture for a given time, followed by detection of the fluorescence of the substrate peptide, or detection of the substrate protein by SDS-PAGE, Western blot, liquid chromatography, or the like. For example, phosphate buffered saline (hereinafter, referred to as "PBS"), a borate buffer (50 mM borate, pH 7 to 9, for example, pH 8.5), or a Tris buffer (50 mM Tris, pH 6 to 9, for example, pH 8.0) can be used as a buffer solution. NaCl (50 to 300 mM, for example, 150 mM) or a surfactant such as CHAPS, or octyl β-D-glucopyranoside may be added to the reaction system, though the additive is not limited thereto.

Examples of the substrate of the HTRA1 protease include, but are not particularly limited to, endogenous substrates, exogenous substrates, and synthetic substrates. Examples of the human endogenous substrate can include vitronectin. Examples of the synthetic substrate can include, but are not particularly limited to, H2-Opt (Mca-IRRVSYSFK(Dnp)K), β-casein, and other HTRA1 substrates. The HTRA1 inhibitory activity (IC₅₀ or Kᵢ) of the peptide of the present invention is 1 µM or lower, more preferably 100 nM or lower.

It is preferred that the inhibiting peptide of the present invention should not inhibit or suppress the activity of a protease other than HTRA1, or should have a relatively weak degree of inhibition or suppression of such activity. In other words, the inhibiting peptide of the present invention preferably has high HTRA1 specificity. Preferably, the inhibiting peptide of the present invention does not inhibit or suppress the activities of proteases such as trypsin, α-chymotrypsin, tryptase, plasmin, thrombin, matriptase, protein C, tissue plasminogen activator (tPA), urokinase (uPA), plasmin, and plasma kallikrein, or has a relatively weak degree of inhibition or suppression of such activities. Such a preferred peptide of the present invention does not exhibit an adverse reaction caused by the inhibition or suppression of the activities of other proteases and can preferably be used as a therapeutic drug or a prophylactic drug for a HTRA1-related disease (mentioned later).

As mentioned above, HTRA1 which is a target of the peptide of the present invention is preferably vertebrate-derived, more preferably mammal-derived, still more preferably primate-derived, and most preferably human-derived. Alternatively, HTRA1 which is a target of the peptide of the present invention may be derived from a nonhuman animal, for example, a rodent such as a rat or a mouse, or a primate such as a cynomolgus monkey, a common marmoset, or a rhesus monkey. A peptide having inhibitory activity against nonhuman animal-derived HTRA1 can be used in the diagnosis, examination, treatment or prevention, etc. of a HTRA1-related disease in the nonhuman animal. When such a peptide also inhibits human HTRA1, the nonhuman animal can be used: in the nonclinical research and development of the peptide as a therapeutic drug or a prophylactic drug for a human HTRA1-related disease; or to conduct a pharmacological test or a pharmacokinetic test using the nonhuman animal as an animal model of the disease; or to conduct a safety test, a toxicity test, or the like using the nonhuman animal as a healthy animal.

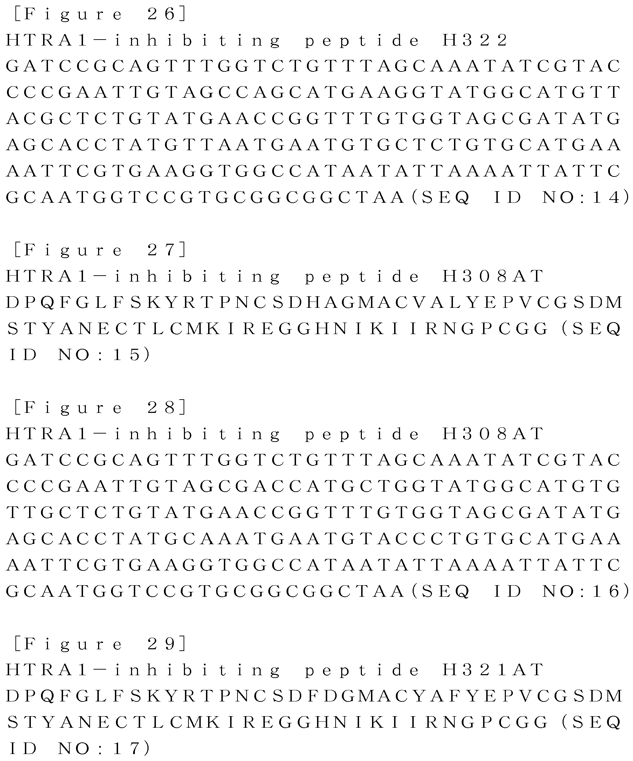

The HTRA1-inhibiting peptide of the present invention has advantages such as: a smaller molecular weight than that of other biomolecules such as antibodies for use as medicaments and diagnostic drugs in the art; relatively easy production (mentioned later) thereof; excellent physical properties in terms of tissue penetration, storage stability, thermal stability, and the like; and a wide range of choice for administration route, administration method, preparation, and the like for use as a pharmaceutical composition (mentioned later). The half-life in blood of the peptide of the present invention used as a pharmaceutical composition can be adjusted to be longer by applying a known method such as the addition of a biomolecule or a polymer and thereby increasing the molecular weight of the peptide. The molecular weight of such a HTRA1-inhibiting peptide of the present invention is smaller than 10,000, preferably smaller than 8,000, and more preferably about 7,000 to 7,200. A variable loop moiety consisting of 15Cys to 31Cys of SEQ ID NO: 23 (Figure 29) or a moiety consisting of 15Cys to 63Cys thereof (hereinafter, referred to as a "moiety containing six Cys residues") and having HTRA1 inhibitory activity is also included in the HTRA1-inhibiting peptide of the present invention. The molecular weight of the variable loop moiety is smaller than 2,500, and preferably about 1,800 to 2,000. The molecular weight of the moiety containing six Cys residues is smaller than 6,000, and preferably about 5,300 to 5,500.

The SPINK2 mutant that at least partially maintains the framework of SPINK2 (hereinafter, referred to as the "SPINK2 mutant"), included in the scope of the HTRA1-inhibiting peptide of the present invention, may bind to HTRA1 and is capable of binding to preferably mammalian HTRA1, more preferably primate HTRA1, and still more preferably human HTRA1. Such a peptide, which binds to HTRA1, recognizes or binds to a partial peptide, a partial conformation, or the like of HTRA1 (hereinafter, such recognizing and binding effects are collectively referred to as "target binding activity").

In an aspect, the inhibiting peptide of the present invention is capable of binding to an immunogenic fragment of HTRA1. The immunogenic fragment of HTRA1 has one or two or more epitopes, mimotopes or other antigenic determinants and is therefore capable of inducing an immune response or is capable of causing an antibody against the fragment to be produced.

The binding of the SPINK2 mutant according to the present invention to HTRA1 or the immunogenic fragment thereof can be evaluated, measured or determined by use of a method known to a person skilled in the art, such as the measurement of detectable binding affinity (ELISA, surface plasmon resonance (hereinafter, referred to as "SPR") analysis (also referred to as a "BIAcore" method), isothermal titration calorimetry (hereinafter, referred to as "ITC"), flow cytometry, an immunoprecipitation method, etc.).

Examples of the ELISA include a method which involves detecting a HTRA1-inhibiting peptide that has recognized and bound to HTRA1 immobilized on a plate. The immobilization of HTRA1 can employ biotin-streptavidin as well as, for example, an antibody for immobilization that recognizes a tag fused with HTRA1. The detection of the HTRA1-inhibiting peptide can employ labeled streptavidin as well as, for example, a labeled antibody for detection that recognizes a tag fused with the HTRA1-inhibiting peptide. The labeling can employ biotin as well as a method feasible in biochemical analysis, such as HRP, alkaline phosphatase, or FITC. The detection using enzymatic labeling can employ a chromogenic substrate such as TMB (3,3',5,5'-tetramethylbenzidine), BCIP (5-bromo-4-chloro-3-indolyl phosphate), p-NPP (p-nitrophenyl phosphate), OPD (o-phenylenediamine), ABTS (3-ethylbenzothiazoline-6-sulfonic acid), or SuperSignal ELISA Pico Chemiluminescent Substrate (Thermo Fisher Scientific Inc.), a fluorescent substrate such as QuantaBlu(TM) Fluorogenic Peroxidase Substrate (Thermo Fisher Scientific Inc.), and a chemiluminescent substrate. The measurement of a detection signal can employ an absorbance plate reader, a fluorescence plate reader, a luminescence plate reader, a RI liquid scintillation counter, or the like.

Examples of an instrument for use in the SPR analysis can include BIAcore(TM) (GE Healthcare), ProteOn(TM) (Bio-Rad Laboratories, Inc.), SPR-Navi(TM) (Oy BioNavis Ltd.), Spreeta(TM) (Texas Instruments Inc.), SPRi-PlexII(TM) (HORIBA, Ltd.), and Autolab SPR(TM) (Metrohm AG). Examples of an instrument for use in BLI can include Octet(TM) (Pall Corp.).

Examples of the immunoprecipitation method include a method which involves detecting HTRA1 that has recognized and bound to a HTRA1-inhibiting peptide immobilized on beads. Magnetic beads, agarose beads, or the like can be used as the beads. The immobilization of the HTRA1-inhibiting peptide can employ biotin-streptavidin as well as an antibody that recognizes the peptide or a tag fused with the peptide, protein A or protein G, etc. The beads are separated using a magnet, centrifugation, or the like, and HTRA1 precipitated with the beads is detected by SDS-PAGE or Western blot. The detection of HTRA1 can employ labeled streptavidin as well as, for example, a labeled antibody for detection that recognizes a tag fused with HTRA1. The labeling can employ biotin as well as a method feasible in biochemical analysis, such as HRP, alkaline phosphatase, or FITC. The detection using enzymatic labeling can employ the same substrate as in ELISA. The measurement of a detection signal can employ ChemiDoc(TM) (Bio-Rad Laboratories, Inc.), LuminoGraph (ATTO Corp.), or the like.

In the present invention, the term "specific recognition", i.e., "specific binding", is used to mean binding that is not nonspecific adsorption. Examples of a criterion for determining whether or not the binding is specific can include binding activity EC₅₀ in ELISA. Other examples of the criterion of determination can include the dissociation constant (hereinafter, referred to as "K_{D}"). The K_{D} value of the HTRA1-inhibiting peptide according to the present invention for HTRA1 is 1 × 10⁻⁴ M or lower, 1 × 10⁻⁵ M or lower, 5 × 10⁻⁶ M or lower, 2 × 10⁻⁶ M or lower or 1 × 10⁻⁶ M or lower, more preferably 5 × 10⁻⁷ M or lower, 2 × 10⁻⁷ M or lower or 1 × 10⁻⁷ M or lower, still more preferably 5 × 10⁻⁸ M or lower, 2 × 10⁻⁸ M or lower or 1 × 10⁻⁸ M or lower, further preferably 5 × 10⁻⁹ M or lower, 2 × 10⁻⁹ M or lower or 1 × 10⁻⁹ M or lower. Other examples of the criterion of determination can include results of analysis by an immunoprecipitation method. The preferred HTRA1-inhibiting peptide according to the present invention is immobilized on beads, and HTRA1 is added thereto. Then, the beads are separated, and HTRA1 precipitated with the beads is detected. In this case, the signal of HTRA1 is detected.

In spite of the description above, the ability to bind to HTRA1 or the immunogenic fragment thereof is not essential for the SPINK2 mutant serving as the inhibiting peptide of the present invention as long as the SPINK2 mutant has HTRA1 inhibitory activity.

The inhibiting peptide of the present invention may be competitive in the binding of a protease substrate to HTRA1.

In some preferred aspects, the inhibiting peptide of the present invention has a retinal protective effect. For example, the preferred inhibitor of the present invention can suppress a light exposure-induced decrease in nucleus count in an outer nuclear layer in a model of retinal damage induced by light exposure, which is described in detail in the Examples. A larger amount of the HTRA1 protein has been detected in the vitreous humor of the light exposure group of this model, as compared with a non-exposure group. Thus, the present invention discloses that: HTRA1 is involved in retinal damage; and HTRA1 inhibitory activity brings about a retinal protective effect.

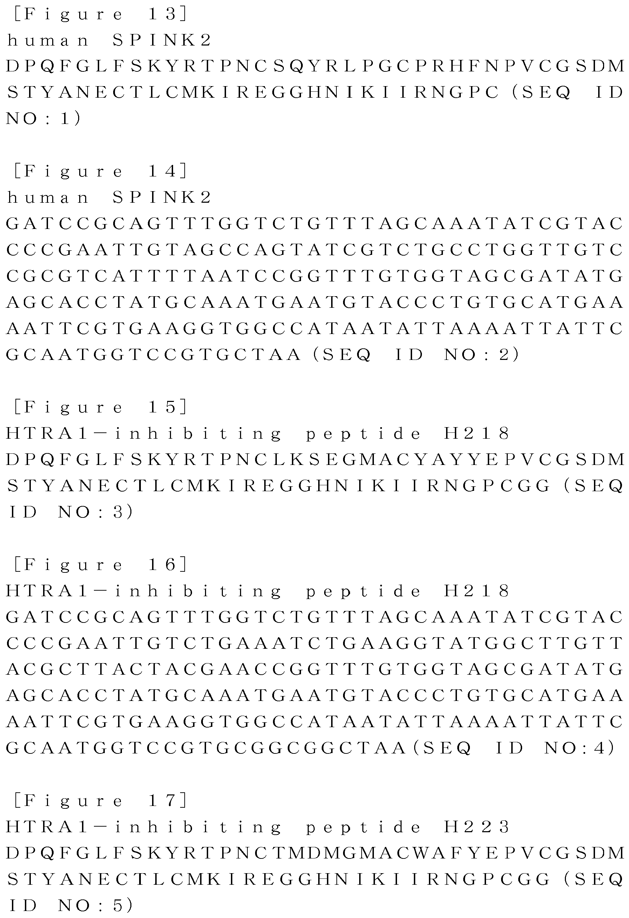

The SPINK2 mutant serving as the inhibiting peptide of the present invention can have the activities, the properties, the functions, the features, etc. as described above, whereas its full-length amino acid sequence has high sequence identity to the amino acid sequence of human wild-type SPINK2. The SPINK2 mutant of the present invention has 70% or higher, 75% or higher, 80% or higher, 85% or higher, 90% or higher, 95% or higher, 98% or higher or 99% or higher sequence identity to the amino acid sequence (SEQ ID NO: 1: Figure 13) of human SPINK2.

The term "identity" is used to mean a property that indicates the degree of similarity or relation between two sequences. The identity (%) of amino acid sequences is calculated by dividing the number of identical amino acids or amino acid residues by the total number of amino acids or amino acid residues and multiplying the resulting numeric value by 100.

The term "gap" is used to mean space in the alignment between two or more sequences as a result of deletion and/or addition in at least one of the two or more sequences.

The identity between two amino acid sequences having completely identical amino acid sequences is 100%. Provided that one of the amino acid sequences has the substitution, deletion or addition of one or two or more amino acids or amino acid residues as compared with the other amino acid sequence, the identity between these two amino acid sequences is lower than 100%. Examples of an algorithm or a program for determining the identity between two sequences in consideration of a gap can include those known to a person skilled in the art, such as BLAST (Altschul, et al., Nucleic Acids Res., Vol. 25, p. 3389-3402, 1997), BLAST2 (Altschul, et al., J. Mol. Biol., Vol. 215, p. 403-410, 1990), and Smith-Waterman (Smith, et al., J. Mol. Biol., Vol. 147, p. 195-197, 1981).

In the present invention, the term "mutated" is used to mean that one or two or more nucleotides or nucleotide residues or amino acids or amino acid residues are substituted, deleted or inserted in a nucleotide sequence or an amino acid sequence compared with a naturally occurring nucleic acid molecule or peptide. The amino acid sequence of the SPINK2 mutant of the present invention has one or two or more mutated amino acids or amino acid residues as compared with the amino acid sequence of human SPINK2.

In an aspect of the present invention, the amino acid sequence of the SPINK2 mutant may have any of:
the substitution of 1, 2, 3, 4, 5, 6 or 7 amino acids from 16Ser to 22Gly by other amino acids or amino acid residues;
the substitution of 1, 2, 3, 4 or 5 amino acids from 24Pro to 28Asn by other amino acids or amino acid residues;
the substitution of 1 or 2 amino acids from 39Ala and 43Thr by other amino acids or amino acid residues in the amino acid sequence (SEQ ID NO: 1: Figure 13) of human SPINK2,
or may have these amino acids as wild-type ones (the 2 amino acids or amino acid residues are included in an α helix) as long as the tertiary structure of the principal chain of a loop constituted by amino acid residues at positions 16 to 30, etc. is capable of at least partially exerting HTRA1 inhibitory activity; each of 15Cys, 23Cys, 31Cys, 42Cys, 45Cys and 63Cys is preferably Cys, as in the wild type, in order to maintain natural disulfide bonds. 1, 2, 3, 4, 5 or 6 thereof may be substituted with other amino acids in order to delete natural disulfide bonds or generate a non-natural disulfide bond. Some preferred HTRA1-inhibiting peptides among the SPINK2 mutants of the present invention maintain Cys at these 6 positions, as in natural SPINK2, and retain the disulfide bonds. In some more preferred forms of such an inhibiting peptide, 15Cys-45Cys, 23Cys-42Cys, and 31Cys-63Cys respectively form disulfide bonds.

When the amino acid sequence of such a SPINK2 mutant is contained in the HTRA1-inhibiting peptide, it is preferred that a three-dimensional structure constituted by, for example, a loop structure consisting of 16Ser to 30Val, β sheet constituted by β strand (1) consisting of 31Cys and 32Gly and β strand (2) consisting of 57Ile to 59Arg, α helix consisting of 41Glu to 51Gly, or a loop structure, β sheet, or α helix similar thereto or at least partially corresponding thereto (or to these positions), contained in the amino acid sequence of wild-type SPINK2 should be maintained to the extent that HTRA1 inhibitory activity can be exerted.

The amino acid sequences of some HTRA1-inhibiting peptides among the SPINK2 mutants of the present invention will be mentioned below. As mentioned above, in the present invention, the term "amino acid residue" is also simply referred to as an "amino acid".

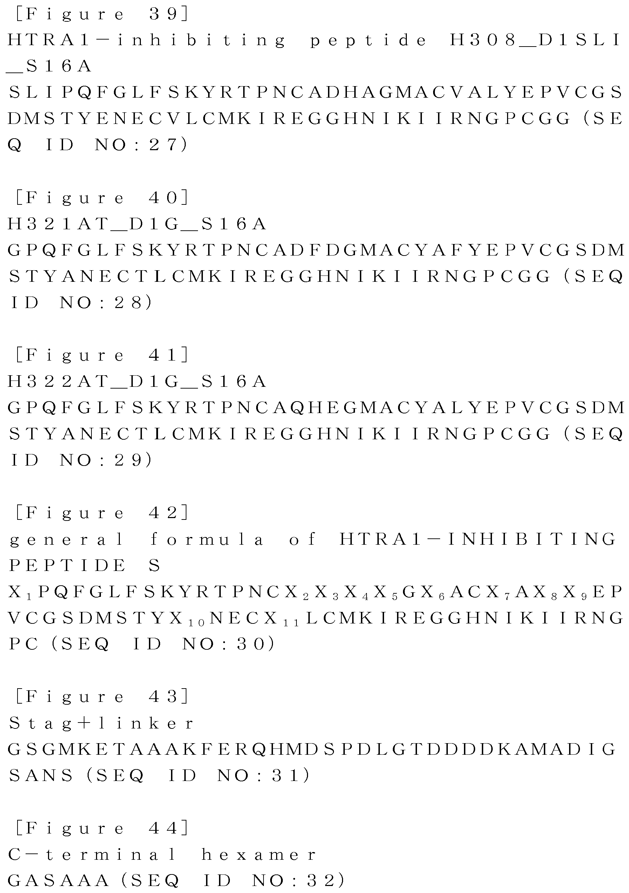

Each of the first to eleventh Xaa (which are the same as X1 to X11, respectively) in the amino acid sequence shown in SEQ ID NO: 30 (Figure 42) is any arbitrary amino acid without particular limitations as long as the resulting mutant binds to HTRA1 and inhibits HTRA1 activity. Hereinafter, preferred amino acids of X1 to X11 will be described. However, these amino acids may include natural amino acids, i.e., amino acids identical to those in the amino acid sequence of wild-type human SPINK2.
X1 at position 1 is preferably Asp, Glu, Gly, Ser or Ile, more preferably Asp or Gly, and still more preferably Gly;
X2 at position 16 is preferably Ala, Asp, Glu, Phe, Gly, His, Lys, Leu, Met, Gln, Arg, Ser, Thr or Tyr, more preferably Ala, Asp, Gly, His, Lys, Leu, Met, Gln, Arg, Ser or Thr, still more preferably Ala, Gly, Lys, Leu, Ser or Thr, further preferably Ala, Gly, Leu, Ser or Thr, and still further preferably Ala or Ser;
X3 at position 17 is preferably Ala, Asp, Glu, Gly, His, Lys, Leu, Met, Asn, Gln, Arg, Ser, Thr or Tyr, more preferably Asp, Gly, His, Lys, Leu, Met, Asn, Gln, Arg, Ser, Thr or Tyr, still more preferably Asp, His, Lys, Met or Gln, and further preferably Asp or Gln;
X4 at position 18 is preferably Ala, Asp, Glu, Phe, Gly, His, Ile, Leu, Lys, Met, Asn, Gln, Arg, Ser, Thr, Val, Trp or Tyr, more preferably Asp, Phe, His, Met, Asn, Gln, Ser or Tyr, still more preferably Asp, Phe, His, Ser or Tyr, and further preferably Phe or His;
X5 at position 19 is preferably Ala, Asp, Glu, Gly, His, Ile, Lys, Met, Asn, Gln, Arg, Ser, Thr, Val or Tyr, more preferably Ala, Asp, Glu, Gly, His, Lys, Met, Asn, Gln, Arg, Ser or Val, still more preferably Ala, Asp, Glu, Met or Asn, and further preferably Ala, Asp or Glu;
X6 at position 21 is preferably Ala, Glu, Phe, Gly, Ile, Leu, Met, Gln, Arg, Ser, Trp or Tyr, more preferably Glu, Phe, Ile, Leu, Met, Gln, Arg or Trp, still more preferably Met or Trp, further preferably Met;
X7 at position 24 is preferably Ala, Asp, Glu, Phe, Gly, His, Lys, Leu, Met, Pro, Gln, Arg, Ser, Thr, Val, Trp or Tyr, more preferably Asp, Glu, His, Pro, Gln, Ser, Thr, Val, Trp or Tyr, still more preferably Gln, Trp, Tyr or Val, and further preferably Tyr or Val;
X8 at position 26 is preferably Ala, Asp, Glu, Phe, His, Ile, Lys, Leu, Met, Asn, Gln, Arg, Ser, Val or Tyr, more preferably Ala, Phe, His, Ile, Leu, Met, Gln, Arg, Ser, Val or Tyr, still more preferably Phe, Leu or Tyr, and further preferably Phe or Leu;
X9 at position 27 is preferably Glu, Phe, Leu, Ser, Thr or Tyr, more preferably Phe, Leu, Ser, Thr or Tyr, still more preferably Phe or Tyr, further preferably Tyr;
X10 at position 39 is preferably Ala, Glu, Met or Val, and more preferably Ala or Glu; and
X11 at position 43 is preferably Ala, Thr or Val, more preferably Thr or Val.

Wild-type X1 to X11 are Asp, Ser, Gln, Tyr, Arg, Pro, Pro, His, Phe, Ala and Thr, respectively. Position 20 is Leu, position 22 is Gly, position 25 is Arg, and position 28 is Asn.

In the present invention, one or several or more amino acids may be further added to the N-terminal side of the first amino acid. Examples of such amino acids to be added can include Ser-Leu, and an amino acid sequence consisting of S tag and a linker (SEQ ID NO: 31: Figure 43) .

One or several amino acids may be further added to 63Cys positioned at the C terminus. Examples of such an amino acid sequence can include an amino acid sequence having 64Gly at the C terminus, and an amino acid sequence having 65Gly at the C terminus by the addition of Gly-Gly. Examples of such an amino acid to be added can include Gly-Gly, and a C-terminal hexamer (SEQ ID NO: 32: Figure 44).

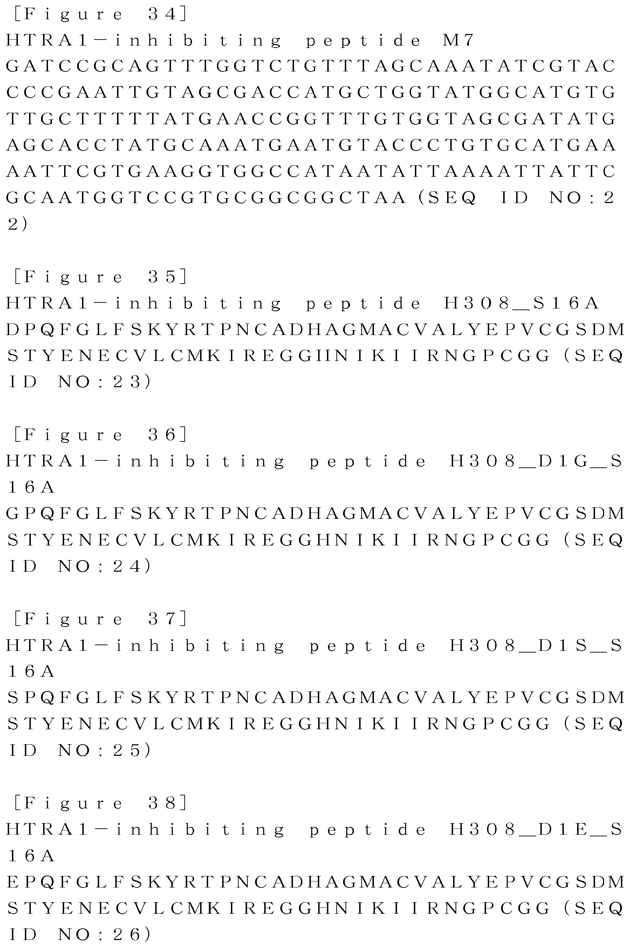

A more preferred form of an amino acid sequence prepared by the addition of other amino acids to the N terminus and/or C terminus of the amino acid sequence shown in SEQ ID NO: 30 (Figure 42) or an amino acid sequence derived from SEQ ID NO: 30 includes an amino acid sequence shown in any one of SEQ ID NOs: 3, 5, 7, 9, 11, 13, 15, 17, 19, 21 and 23 to 29 (Figures 15, 17, 19, 21, 23, 25, 27, 29, 31, 33 and 35 to 41) and an amino acid sequence encoded by a nucleotide sequence shown in any one of SEQ ID NOs: 4, 6, 8, 10, 12, 14, 16, 18, 20 and 22 (Figures 16, 18, 20, 22, 24, 26, 28, 30, 32 and 34). A still more preferred form thereof includes an amino acid sequence shown in any one of SEQ ID NOs: 24, 28 and 29 (Figures 36, 40 and 41).

In the present invention, a peptide prepared by the substitution, addition and/or deletion of one or two or more amino acids in, to or from the SPINK2 mutant peptide or the N-terminal and/or C-terminal adduct of the SPINK2 mutant peptide (hereinafter, referred to as a "parent peptide") is referred to as a "derivative of the parent peptide" or a "parent peptide derivative" (e.g., Example 6). Such a "derivative" is also included in the scope of the "peptide" of the present invention.

The amino acid sequence of the SPINK2 mutant included in the scope of the inhibiting peptide of the present invention can comprise a natural amino acid or a mutated amino acid or amino acid sequence at moieties other than X1 to X11, i.e., the positions of 2Pro to 15Cys, 20Pro, 22Gly, 23Cys, 25Arg, 28Asn to 38Tyr, 41Glu, 42Cys and 44Thr to 63Cys, in the amino acid sequence (SEQ ID NO: 1: Figure 13) of wild-type human SPINK2. For example, the SPINK2 mutant may have a mutation at one or two or more positions as long as HTRA1 inhibitory activity or folding is at least partially neither hindered nor interfered with. Such a mutation is attained by use of a standard method known to a person skilled in the art. Typical examples of the mutation in the amino acid sequence can include the substitution, deletion or addition of one or two or more amino acids. Examples of the substitution can include conservative substitution. The conservative substitution substitutes a certain amino acid residue with an amino acid residue similar thereto in chemical features in terms of not only bulkiness but polarity. Examples of the conservative substitution are described in other parts of the present specification. On the other hand, the moieties other than X1 to X11 may accept the non-conservative substitution of one or two or more amino acids as long as HTRA1 inhibitory activity or folding is at least partially neither hindered nor interfered with.

In the amino acid sequence of the SPINK2 mutant serving as the inhibiting peptide of the present invention, X1 to X11 are preferably amino acids X1 to X11, respectively, in any one of SEQ ID NOs: 3, 5, 7, 9, 11, 13, 15, 17, 19, 21 and 23 to 29 (Figures 15, 17, 19, 21, 23, 25, 27, 29, 31, 33 and 35 to 41), and more preferably SEQ ID NOs: 24, 28 and 29 (Figures 36, 40 and to 41), and the moieties other than X1 to X11 can have an amino acid or an amino acid sequence that at least partially neither hinders nor interferes with HTRA1 inhibitory activity or folding.

Examples of the amino acid sequence of the SPINK2 mutant serving as the HTRA1-inhibiting peptide of the present invention can include any one of the following amino acid sequences (a) to (e):
(a) an amino acid sequence shown in any one of SEQ ID NOs: 3, 5, 7, 9, 11, 13, 15, 17, 19, 21, and 23 to 29 (Figures 15, 17, 19, 21, 23, 25, 25, 29, 31, 33, and 35 to 41) ;
(b) an amino acid sequence encoded by a nucleotide sequence which hybridizes under stringent conditions to a nucleotide sequence complementary to a nucleotide sequence encoding amino acid sequence (a) and encodes an amino acid sequence comprised in a peptide having HTRA1 inhibitory activity;
(c) an amino acid sequence which is derived from amino acid sequence (a) by the substitution, deletion, addition and/or insertion of 1 to 15, 1 to 10, 1 to 8, 1 to 6, 1 to 5, 1 to 4, 1 to 3, 1 or 2, or 1 amino acid and comprised in a peptide having HTRA1 inhibitory activity;
(d) an amino acid sequence which has 70%, 80%, 85%, 90%, 92%, 94%, 96%, 97%, 98% or 99% or higher identity to amino acid sequence (a) and is comprised in a peptide having HTRA1 inhibitory activity; and
(e) an amino acid sequence encoded by a nucleotide sequence shown in any one of SEQ ID NOs: 4, 6, 8, 10, 12, 14, 16, 18, 20 and 22 (Figures 16, 18, 20, 22, 24, 26, 28, 30, 32 and 34).

However, the nucleic acid molecule encoding the HTRA1-inhibiting peptide is not limited to (a) to (e). Every nucleic acid molecule comprising a nucleotide sequence encoding an amino acid sequence contained in a SPINK2 mutant having HTRA1 inhibitory activity, and preferably the amino acid sequence shown in SEQ ID NO: 30 (Figure 42), is included in the scope of the nucleic acid molecule encoding the HTRA1-inhibiting peptide.

A mutation can be introduced to the inhibiting peptide of the present invention for the purpose of improving its folding stability, thermal stability, storage stability, half-life in blood, water solubility, biological activity, pharmacological activity, secondary effect, etc. For example, a new reactive group such as Cys can be introduced thereto by a mutation in order to conjugate the inhibiting peptide to an additional substance such as polyethylene glycol (PEG), hydroxyethyl starch (HES), biotin, a peptide or a protein. In the present invention, the HTRA1-inhibiting peptide may be added, linked, or bound to an additional moiety. Such conjugates are collectively referred to as a "conjugate of the HTRA1-inhibiting peptide". In the present invention, the term "conjugate" is used to mean a molecule of the peptide of the present invention or a fragment thereof added, linked or bound to an additional moiety. The term "conjugate" or "conjugation" includes a form in which a certain moiety is linked or bound, via an agent or the like suitable for linking the certain moiety to the side chain of an amino acid, including a chemical substance such as a cross-linking agent, to the N terminus and/or C terminus of the peptide of the present invention by a synthetic chemical approach, a genetic engineering approach, or the like. Examples of such a "moiety" for improving the half-life in blood can include polyalkylene glycol molecules such as polyethylene glycol (PEG), hydroxyethyl starch, fatty acid molecules such as palmitic acid, immunoglobulin Fc regions, immunoglobulin CH3 domains, immunoglobulin CH4 domains, albumin or fragments thereof, albumin-binding peptides, albumin-binding proteins such as streptococcal protein G, and transferrin. Alternatively, the "moiety" may be linked to the peptide of the present invention via a linker such as a peptide linker.

The HTRA1-inhibiting peptide of the present invention may be conjugated with an additional drug in order to exert or enhance pharmacological activity. In the field of antibodies, a technique or a form known to a person skilled in the art as antibody-drug conjugates (ADCs) constitutes some aspects of the present invention by the replacement of the antibody with the peptide of the present invention.

The HTRA1-inhibiting peptide of the present invention may further comprise one or two or more moieties that exert binding affinity, inhibitory activity, antagonistic activity, agonistic activity, or the like against a target molecule other than HTRA1, or may be conjugated with such moieties. Examples of the "moiety" can include antibodies or fragments thereof, and proteins having a non-antibody framework, such as SPINK2 mutants, or fragments thereof. In the field of antibodies, a technique or a form known to a person skilled in the art as multispecific antibodies and bispecific antibodies constitutes some aspects of the conjugate of the present invention by the replacement of at least one or two or more "antibodies" comprised therein with the peptide of the present invention.

The peptide of the present invention or a precursor thereof may comprise a signal sequence. A signal sequence present at or added to the N terminus of a certain polypeptide or a precursor thereof is useful for delivering the polypeptide to a specific compartment of a cell, for example, the periplasm of *E. coli* or the endoplasmic reticulum of a eukaryotic cell. Many signal sequences are known to a person skilled in the art, and the signal sequence can be selected according to host cells. Examples of the signal sequence for secreting the desired peptide into the periplasm of *E. coli* can include OmpA. The form comprising the signal sequence can also be included in some aspects of the conjugate of the present invention.

The peptide of the present invention can be tagged in advance and thereby purified by affinity chromatography. The peptide of the present invention can comprise, for example, biotin, Strep tag(TM), Strep tag II(TM), oligohistidine such as His6, polyhistidine, an immunoglobulin domain, a maltose-binding protein, glutathione-S-transferase (GST), a calmodulin-binding peptide (CBP), a hapten such as digoxigenin or dinitrophenol, an epitope tag such as FLAG(TM), myc tag, or HA tag (hereinafter, collectively referred to as an "affinity tag") at its C-terminus. The tagged form can also be included in some aspects of the conjugate of the present invention. The conjugate of the present invention may be a peptide (polypeptide) as a whole.

The peptide of the present invention can comprise a moiety for labeling. Specifically, the peptide of the present invention can be conjugated to a label moiety such as an enzymatic label, a radiolabel, a colored label, a fluorescent label, a coloring label, a luminescent label, a hapten, digoxigenin, biotin, a metal complex, a metal, or colloidal gold. The form comprising the moiety for labeling can also be included in some aspects of the conjugate of the present invention.

The inhibiting peptide of the present invention can comprise any of the natural amino acids and non-natural amino acids in its peptide moiety and can comprise an L-amino acid and a D-amino acid as a natural amino acid.

The amino acid sequence of the inhibiting peptide of the present invention can comprise any of the natural amino acids and non-natural amino acids and can comprise an L-amino acid and a D-amino acid as a natural amino acid.

The inhibiting peptide of the present invention can be present as a monomer, a dimer, or a trimer or higher oligomer or multimer. The dimer or the trimer or higher oligomer or multimer can be any of a homo form constituted by single monomers, and a hetero form constituted by two or more different monomers. The monomer may be rapidly diffused and be excellent in permeation into tissues, for example. The dimer, the oligomer and the multimer may have an excellent aspect, for example, high local affinity or binding activity for a target molecule, a slow dissociation rate, or high HTRA1 inhibitory activity. In addition to spontaneous dimerization, oligomerization and multimerization, intended dimerization, oligomerization and multimerization are attained by introducing a jun-fos domain, leucine zipper, or the like to the inhibiting peptide of the present invention.

The inhibiting peptide of the present invention can bind to one or two or more target molecules or inhibit the activity of the target molecules, in the form of a monomer, a dimer, or a trimer or higher oligomer or multimer.

Examples of the form that can be taken by the inhibiting peptide of the present invention can include, but are not limited to, isolated forms (freeze-dried preparations, solutions, etc.), the conjugates mentioned above, and forms bound to other molecules (immobilized forms, associates with a different molecule, forms bound to a target molecule, etc.). A form compatible with expression, purification, use, storage, or the like can be arbitrarily selected.

### 3. Identification of HTRA1-inhibiting peptide

The HTRA1-inhibiting peptide can be identified by a method well known to a person skilled in the art using a starting material such as the amino acid sequence of SPINK2 or the amino acid sequence (e.g., an amino acid sequence selected from the group consisting of SEQ ID NOs: 3, 5, 7, 9, 11, 13, 15, 17, 19, 21 and 23 to 29, or the group consisting of Figures 15, 17, 19, 21, 23, 25, 27, 29, 31, 33 and 35 to 41) of the HTRA1-inhibiting peptide of the present invention, a nucleotide sequence encoding the amino acid sequence, or a nucleic acid molecule comprising the nucleotide sequence. As a preferred example, the HTRA1-inhibiting peptide can be identified from a human SPINK2 mutant library by using HTRA1 inhibitory activity as an indicator which may be combined with HTRA1 binding activity as another indicator.

For example, the nucleic acid molecule serving as a starting material can be mutagenized and transferred into an appropriate bacterial host or eukaryotic host by use of a recombinant DNA technique. The SPINK2 mutant library is known as a technique for identifying a binder or an inhibitor of a target molecule. For example, the disclosure of WO2012/105616 After expression of the mutagenized nucleotide sequence in the appropriate host, a clone in which a SPINK2 mutant having the desired properties, activity, function, etc. is linked to its genotype can be enriched and/or selected from the library and identified. The enrichment and/or selection of the clone employs a method known to a person skilled in the art, such as a bacterial display method (Francisco, J.A., et al., (1993), Proc. Natl. Acad. Sci. U.S.A. Vol. 90, p. 10444-10448), a yeast display method (Boder, E.T., et al., (1997), Nat. Biotechnol., Vol. 15, p. 553-557), a mammalian cell display method (Ho M, et al., (2009), Methods Mol Biol., Vol. 525: p. 337-52), a phage display method (Smith, G.P. (1985), Science., Vol. 228, p. 1315-1317), a ribosome display method (Mattheakis LC, et al., (1994), Proc. Natl. Acad. Sci. U.S.A. Vol. 91, No. 19, p. 9022-9029), a nucleic acid display method such as mRNA display (Nemoto N, et al., (1997), FEES Lett., Vol. 414, No. 2, p. 405-408), or a colony screening method (Pini, A. et al., (2002), Comb. Chem. High Throughput Screen., Vol. 5, p. 503-510). The nucleotide sequence of a SPINK2 mutant contained in the clone thus selected and identified can be determined to determine an amino acid sequence encoded by the nucleotide sequence as the amino acid sequence of the SPINK2 mutant contained in the clone, i.e., the HTRA1-inhibiting peptide.

The SPINK2 mutant of the present invention can be obtained, for example, by mutagenizing natural SPINK2. The term "mutagenesis" is used to mean that one or two or more amino acids at their respective positions of a certain amino acid sequence can be substituted by other amino acids or deleted, or an amino acid absent from the amino acid sequence can be added or inserted thereinto. Such a deletion or such an addition or insertion can change the sequence length. In the SPINK2 mutant of the present invention, the mutagenesis can preferably occur at one or two or more positions of X1 to X11 in the amino acid sequence shown in SEQ ID NO: 30 (Figure 42).

However, a form that maintains, after such preferred mutagenesis, natural amino acids at one or two or more positions of X1 to X11, i.e., the same amino acid as that present at the specific position in the natural amino acid sequence, is also included in the scope of the mutant as long as at least one amino acid is mutated in the whole. Likewise, in an aspect of the present invention, a form that maintains, after mutagenesis at one or more positions of moieties other than X1 to X11, natural amino acids at these positions, i.e., the same amino acid as that present at the specific position in the natural amino acid sequence, is also included in the scope of the mutant as long as at least one amino acid is mutated in the whole.

The term "random mutagenesis" is used to mean that as to a specific position on a sequence, one or two or more different amino acids are introduced with a given probability to the position by mutagenesis. The probabilities of introduction of at least two different amino acids may not always be the same. The present invention does not exclude said at least two different amino acids from including the naturally-occurring amino acid (as one of the amino acids). Such a case is also included in the scope of the term "random mutagenesis".

A standard method known to a person skilled in the art can be used as a method for random mutagenesis at a specific position. The mutagenesis can be attained by, for example, PCR (polymerase chain reaction) using a mixture of synthetic oligonucleotides including a degenerate nucleotide composition at a specific position in a sequence. For example, use of codon NNK or NNS (N = adenine, guanine, cytosine or thymine; K = guanine or thymine; and S = guanine or cytosine) causes mutagenesis to introduce any of all 20 natural amino acids as well as a stop codon, whereas use of codon VVS (V = adenine, guanine or cytosine) eliminates the possibility of introducing Cys, Ile, Leu, Met, Phe, Trp, Tyr and Val and causes mutagenesis to introduce any of the remaining 12 natural amino acids. For example, use of codon NMS (M = adenine or cytosine) eliminates the possibility of introducing Arg, Cys, Gly, Ile, Leu, Met, Phe, Trp and Val and causes mutagenesis to introduce any of the remaining 11 natural amino acids. A specific codon, an artificial codon, or the like can be used for mutagenesis to introduce a non-natural amino acid.

The site-directed mutagenesis can also be performed through the use of structural information on a target containing a conformation and/or a peptide against the target or a wild-type peptide from which the peptide is derived. In the present invention, a site-directed mutation can be introduced through the use of structural information including higher-order information on target HTRA1 and/or a SPINK2 mutant against the target or wild-type SPINK2, or a complex therebetween. In one example, a SPINK2 mutant having HTRA1 inhibitory activity is identified. Subsequently, a crystalline complex of HTRA1 and the SPINK2 mutant is obtained and subjected to X-ray crystallography. On the basis of the analysis results, a site on the HTRA1 molecule to which the SPINK2 mutant binds, and an amino acid residue in the SPINK2 mutant involved in interaction with the site are identified. The correlation of structural information obtained through such procedures with HTRA1 inhibitory activity may be found. On the basis of such structure-activity correlation, the substitution of an amino acid at a specific position by a specific amino acid, the insertion or deletion of an amino acid to a specific position, or the like can be designed to actually confirm HTRA1 activity.

The mutagenesis can also be attained using, for example, a nucleotide constituent unit with modified base pair specificity, such as inosine.

Furthermore, mutagenesis at a random position is achieved by, for example, error-prone PCR using DNA polymerase, such as Taq DNA polymerase, which lacks an error correcting function and produces a high rate of errors, or chemical mutagenesis.

The HTRA1-inhibiting peptide can be enriched and/or selected from a library, such as a phage library or a colony library, which is suitable for the respective screening method and known to a person skilled in the art, through the use of bacterial display, yeast display, mammalian cell display, phage display, ribosome display, nucleic acid display, colony screening, or the like. These libraries can be constructed using a vector and a method, such as a phagemid for the phage library or a cosmid for the colony screening, which are suitable for each library and known to a person skilled in the art. Such a vector may be a virus or viral vector that infects prokaryotic cells or eukaryotic cells. These recombinant vectors can be prepared by a method known to a person skilled in the art, such as genetic manipulation.

Bacterial display is a technique of fusing a desired protein with, for example, a portion of *E. coli* outer membrane lipoprotein (Lpp) and outer membrane protein OmpA, and displaying the desired protein on the surface of *E. coli.* A DNA group obtained by the random mutagenesis of a nucleotide sequence encoding the amino acid sequence of a certain protein is inserted into vectors suitable for bacterial display, and bacterial cells can be transformed with the vectors to obtain a library displaying a randomly mutagenized protein group on transformed bacterial cell surface (Francisco, J.A., et al., (1993), Proc. Natl. Acad. Sci. U.S.A. Vol. 90, p. 10444-10448) .

Yeast display is a technique of fusing a desired protein with a coat protein such as α-agglutinin which is on the cell surface of yeast, and displaying the desired protein on the surface of yeast. The α-agglutinin comprises a C-terminal hydrophobic region with a putative glycosylphosphatidylinositol (GPI) anchor attachment signal, a signal sequence, an active domain, a cell wall domain, and the like. The desired protein can be displayed on the cell surface of yeast by manipulation thereof. A DNA group obtained by the random mutagenesis of a nucleotide sequence encoding the amino acid sequence of a certain protein is inserted into vectors suitable for yeast display, and yeast cells can be transformed with the vectors to obtain a library displaying a randomly mutagenized protein group on the cell surface of transformed yeast (Ueda, M.& Tanaka, A., Biotechnol. Adv., Vol. 18, p. 121-, 2000; Ueda, M.& Tanaka, A., J. Biosci. Bioeng., Vol. 90, p. 125-, 2000; etc.).

Animal cell display is a technique of fusing a desired protein with, for example, the transmembrane region of a membrane protein typified by platelet-derived growth factor receptor (PDGFR), and displaying the desired protein on the surface of mammalian cells (e.g., HEK293 and Chinese hamster ovary (CHO) cells). A DNA group obtained by the random mutagenesis of a nucleotide sequence encoding the amino acid sequence of a certain protein is inserted into vectors suitable for animal cell display, and animal cells can be transfected with the vectors to obtain a library displaying a randomly mutagenized protein group on the surface of transfected animal cells (Ho M, et al., (2009), Methods Mol Biol. Vol. 525: p. 337-52).

The desired library displayed on cells such as yeast cells, bacterial cells, or animal cells can be incubated in the presence of a target molecule or contacted with a target molecule. For example, the cells involving the library are incubated for a given time with HTRA1 modified with biotin or the like. Then, a support such as magnetic beads is added thereto, and the cells are separated from the support. Subsequently, the support can be washed to effect the removal of nonspecific adsorbed matter and bound matter in order to recover a cell group displaying a peptide, a peptide collection or an enriched peptide collection bound to the support (which has HTRA1 bound thereto). Likewise, the cell group displaying a peptide, a peptide collection or an enriched peptide collection bound to the support (which has HTRA1 bound thereto), or the cell group displaying a peptide, a peptide collection or an enriched peptide collection bound to HTRA1, can be recovered by magnetic-activated cell sorting (MACS) after addition of the magnetic beads, or by FACS after cell staining using an anti-HTRA1 antibody, respectively. A nonspecific adsorption site and/or binding site may be blocked (saturated), for example. The blocking step can be incorporated herein as long as the blocking is performed by an appropriate method. The vector of the expressed peptide, peptide collection or enriched peptide collection thus obtained is recovered, and the nucleotide sequence of the polynucleotide inserted into the vector can be determined to determine the amino acid sequence encoded by the nucleotide sequence. Moreover, the vector can be transferred again into host cells, and a cycle of the operation mentioned above can be repeated once to several times to highly enrich a peptide collection which binds to the target molecule.

In the case of phage display, for example, the phagemid is a bacterial plasmid containing a plasmid replication origin as well as the second replication origin derived from a single-stranded bacteriophage. A cell having the phagemid can replicate the phagemid via a single-strand replication mode by co-infection with M13 or a helper bacteriophage similar thereto. Specifically, single-stranded phagemid DNA is packaged into an infectious particle coated with a bacteriophage coat protein. In this way, the phagemid DNA can be formed as a clone double-stranded DNA plasmid in an infected bacterium, while the phagemid can be formed as a bacteriophage-like particle from a culture supernatant of co-infected cells. The bacteriophage-like particle is injected into a bacterium having F-pilus for the infection of the bacterium with the DNA so that the particle itself can be re-formed as a plasmid.

A fusion gene comprising a polynucleotide having a nucleotide sequence encoding the amino acid sequence of a test peptide and a bacteriophage coat protein gene is inserted into the phagemid, and a bacterium is infected with the resulting phagemid. The cells are cultured so that the peptide can be expressed or displayed on the bacterium or on a phage-like particle, or can be produced as a fusion protein with the coat protein into a phage particle or into a culture supernatant of the bacterium.

For example, a fusion gene comprising the polynucleotide and the bacteriophage coat protein gene gpIII is inserted into the phagemid, and *E. coli* is co-infected with the resulting phagemid and M13 or a helper phage similar thereto so that a fusion protein comprising the peptide and the coat protein can be produced and released into the culture supernatant of the *E. coli.*

In the case of using various circular or noncircular vectors, for example, a virus vector, instead of the phagemid, a peptide having an amino acid sequence encoded by the nucleotide sequence of the polynucleotide inserted in the vector can be expressed or displayed on cells or virus-like particles harboring the vector, or can be produced and released into the culture supernatant of the cells, according to a method known to a person skilled in the art.

The peptide-expressing library thus obtained can be incubated in the presence of a target molecule, or contacted with a target molecule. For example, a HTRA1-immobilized support is incubated for a given time with a mobile phase containing the library. Then, the mobile phase is separated from the support. Subsequently, the support is washed to effect the removal of nonspecific adsorbed matter and bound matter. A peptide, a peptide collection or an enriched peptide collection bound to the support (which has HTRA1 bound thereto) can be recovered by elution. The elution can be non-selectively performed, for example, in the presence of relatively high ionic strength, low pH, moderate denaturation conditions, or chaotropic salt, or can be selectively performed by adding a soluble target molecule such as HTRA1, an antibody which binds to the target molecule, a natural ligand, a substrate, or the like and competing with an immobilized target molecule. A non-specific adsorption site and/or binding site may be blocked, for example. The blocking step can be incorporated herein as long as the blocking is performed by an appropriate method.

The vector of the expressed peptide, peptide collection or enriched peptide collection thus obtained is recovered, and the nucleotide sequence of the polynucleotide inserted in the vector can be determined to determine an amino acid sequence encoded by the nucleotide sequence. Moreover, the vector can be transferred again into host cells, and a cycle of the operation mentioned above can be repeated once to several times to highly enrich a peptide collection which binds to the target molecule.

Ribosome display is a technique of using, for example, mRNA that encodes a desired protein and lacks a stop codon, and a cell-free protein synthesis system, and thereby synthesizing *in vitro* a molecule of the desired protein, its corresponding mRNA, and a ribosome linked to each other. A library displaying a randomly mutagenized protein group on ribosomes can be obtained through the use of a mRNA group obtained by the random mutagenesis of a nucleotide sequence encoding the amino acid sequence of a certain protein, and a cell-free protein synthesis system (Mattheakis LC, et al., (1994) Proc. Natl. Acad. Sci. U.S.A. Vol. 91, No. 19, p. 9022-9029).

Nucleic acid display, also called mRNA display, is a technique of using, for example, a linker such as puromycin structurally similar to the 3' end of tyrosyl tRNA, and thereby synthesizing a molecule of a desired protein, its encoding mRNA and a ribosome linked to each other. This technique employs a cell-free protein synthesis system, not live cells, and therefore permits *in vitro* synthesis. A library displaying a randomly mutagenized protein group on a ribosome can be obtained through the use of an mRNA group obtained by the random mutagenesis of a nucleotide sequence encoding the amino acid sequence of a certain protein, a linker such as puromycin, and a cell-free protein synthesis system (Nemoto N, et al., (1997), FEES Lett., Vol. 414, No. 2, p. 405-408) .

The peptide-displaying library obtained via a cell-free synthesis system, such as ribosome display or nucleic acid display, can be incubated in the presence of a target molecule, or contacted with a target molecule. For example, a HTRA1-immobilized support is incubated for a given time with a mobile phase containing the library. Then, the mobile phase is separated from the support. Subsequently, the support is washed to effect the removal of nonspecific adsorbed matter and bound matter. A peptide, a peptide collection or an enriched peptide collection bound to the support (which has HTRA1 bound thereto) can be recovered by elution. The elution can be non-selectively performed, for example, in the presence of relatively high ionic strength, low pH, moderate denaturation conditions, or chaotropic salt, or can be selectively performed by adding a soluble target molecule such as HTRA1, an antibody which binds to the target molecule, a natural ligand, a substrate, or the like and competing with an immobilized target molecule. A non-specific adsorption site and/or binding site may be blocked, for example. The blocking step can be incorporated herein as long as the blocking is performed by an appropriate method.

The nucleic acid of the expressed peptide, peptide collection or enriched peptide collection thus obtained is recovered. In the case of mRNA, the nucleotide sequence can be determined after a reverse transcription reaction into cDNA to determine an amino acid sequence encoded by the nucleotide sequence. Moreover, the recovered nucleic acid is transcribed into mRNA, and a cycle of the operation mentioned above can be repeated once to several times to highly enrich a peptide collection which binds to the target molecule.

Provided that the peptide, the peptide collection or the enriched peptide collection is conjugated in advance with an affinity tag, the peptide or the collection can be efficiently purified. For example, the peptide collection is conjugated in advance with a protease substrate as a tag so that the peptide can be eluted by cleavage through the protease activity.

On the basis of the obtained sequence information and the function of the peptide, etc., the obtained clone or library may be further mutagenized, and a peptide improved in its function (e.g., HTRA1 inhibitory activity), physical properties (thermal stability, storage stability, etc.), *in vivo* kinetics (distribution and half-life in blood), etc. can be obtained from the mutated library.

The HTRA1-inhibiting peptide can be identified by determining whether or not the obtained peptide has HTRA1 inhibitory activity.

The HTRA1-inhibiting peptide is preferably capable of maintaining a three-dimensional structure constituted by, for example, a loop structure consisting of 16Ser to 30Val, β sheet constituted by β strand (1) consisting of 31Cys and 32Gly and β strand (2) consisting of 57Ile to 59Arg, and α helix consisting of 41Glu to 51Gly, or a loop structure, β sheet, or α helix similar thereto or at least partially corresponding thereto (or to these positions), contained in the amino acid sequence of wild-type SPINK2, to the extent that HTRA1 inhibitory activity can be exerted. A more preferred HTRA1-inhibiting peptide may be identified by using such a three-dimensional structure (whole structure or partial structure) as a portion of an indicator.

### 4. Nucleic acid molecule encoding HTRA1-inhibiting peptide, vector comprising the nucleic acid molecule, cell comprising the nucleic acid molecule or the vector, and method for producing recombinant HTRA1-inhibiting peptide

The present invention also provides a polynucleotide comprising a nucleotide sequence encoding an amino acid sequence contained in the HTRA1-inhibiting peptide (hereinafter, referred to as a "nucleic acid molecule encoding the HTRA1-inhibiting peptide"), a recombinant vector having an insert of the gene, a cell harboring the gene or the vector (hereinafter, referred to as a "cell containing the nucleic acid molecule encoding the HTRA1-inhibiting peptide"), or a cell producing the HTRA1-inhibiting peptide (hereinafter, referred to as a "HTRA1-inhibiting peptide-producing cell").

Some preferred examples of the nucleic acid molecule encoding the HTRA1-inhibiting peptide of the present invention can include a nucleic acid molecule comprising any one of the following nucleotide sequences (a) to (e) (hereinafter, referred to as the "nucleotide sequence of the HTRA1-inhibiting peptide"), a nucleic acid molecule consisting of a nucleotide sequence comprising an antibody gene sequence, and a nucleic acid molecule consisting of an antibody gene sequence:
(a) a nucleotide sequence encoding an amino acid sequence shown in any one of SEQ ID NOs: 3, 5, 7, 9, 11, 13, 15, 17, 19, 21, and 23 to 29 (Figures 15, 17, 19, 21, 23, 25, 25, 29, 31, 33, and 35 to 41);
(b) a nucleotide sequence shown in any one of SEQ ID NOs: 4, 6, 8, 10, 12, 14, 16, 18, 20 and 22 (Figures 16, 18, 20, 22, 24, 26, 28, 30, 32 and 34);
(c) a nucleotide sequence which hybridizes under stringent conditions to a nucleotide sequence complementary to the nucleotide sequence (a) or (b) and encodes an amino acid sequence comprised in a peptide having HTRA1 inhibitory activity;
(d) a nucleotide sequence which is derived from the nucleotide sequence (a) or (b) by the substitution, deletion, addition and/or insertion of 1 to 15, 1 to 10, 1 to 8, 1 to 6, 1 to 5, 1 to 4, 1 to 3, 1 or 2, or 1 nucleotide or nucleotide residue and encodes an amino acid sequence comprised in a peptide having HTRA1 inhibitory activity; and
(e) a nucleotide sequence which has 70%, 80%, 85%, 90%, 92%, 94%, 96%, 97%, 98% or 99% or higher identity to the nucleotide sequence (a) or (b) and encodes an amino acid sequence comprised in a peptide having HTRA1 inhibitory activity.

However, the nucleic acid molecule encoding the HTRA1-inhibiting peptide is not limited to the nucleotide sequences (a) to (e). Every nucleic acid molecule comprising a nucleotide sequence encoding an amino acid sequence comprised in a SPINK2 mutant having HTRA1 inhibitory activity, and preferably the amino acid sequence shown in SEQ ID NO: 30 (Figure 42), is included in the scope of the nucleic acid molecule encoding the HTRA1-inhibiting peptide.

One or two or more codons corresponding to each amino acid can be used for designing the nucleotide sequence encoding the amino acid sequence. Hence, a nucleotide sequence encoding the single amino acid sequence of a certain peptide can have a plurality of variations. For the selection of such codons, the codons can be appropriately selected according to the codon usage of host cells for expression to harbor a polynucleotide comprising the nucleotide sequence or a vector comprising the polynucleotide, or the frequency or rate of a plurality of codons used can be appropriately adjusted. For example, in the case of using *Escherichia coli* cells as host cells, the nucleotide sequence may be designed using codons with high frequency of use in *Escherichia coli.*

The nucleic acid molecule encoding the HTRA1-inhibiting peptide may be functionally linked to one or two or more regulatory sequences. The phrase "functionally linked" is used to mean that the linked nucleic acid molecule can be expressed, or a nucleotide sequence contained in the molecule is expressible. The regulatory sequence includes a sequence element involving information on transcriptional regulation and/or translational regulation. Although the regulatory sequence varies depending on species, the regulatory sequence generally includes a promoter and includes 5' non-coding sequences involved in the initiation of transcription and translation, for example, a prokaryotic -35/-10 box and Shine-Dalgarno sequence or a eukaryotic TATA box, CAAT sequence, and 5' capping sequence. This sequence may comprise an enhancer element and/or a repressor element, and a translatable signal sequence, leader sequence, or the like for delivering a natural or mature peptide to a specific compartment inside or outside a host cell. The regulatory sequence may further include a 3' noncoding sequence, and this sequence may comprise an element involved in transcription termination or polyadenylation, etc. However, the sequence related to transcription termination, when functioning insufficiently in specific host cells, can be substituted with a sequence suitable for the cells.

Examples of the promoter sequence can include a prokaryotic tet promoter, a lacUV5 promoter, and a T7 promoter, and an SV40 promoter and a CMV promoter for eukaryotic cells.

The nucleic acid molecule encoding the HTRA1-inhibiting peptide may be in an isolated form or in a form contained in a vector or another cloning vehicle (hereinafter, simply referred to as a "vector"; plasmid, phagemid, phage, baculovirus, cosmid, etc.) or in a chromosome, though the form is not limited thereto. The vector may comprise, in addition to the nucleotide sequence of the HTRA1-inhibiting peptide and the regulatory sequence, a replicating sequence and a control sequence suitable for host cells for use in expression, and a selective marker which confers a phenotype that permits selection of cells harboring the nucleic acid molecule by transformation or the like.

The nucleic acid molecule encoding the HTRA1-inhibiting peptide or the vector comprising the nucleotide sequence of the HTRA1-inhibiting peptide can be transferred to host cells capable of expressing the peptide or the nucleotide sequence by a method known to a person skilled in the art, such as transformation. The host cells harboring the nucleic acid molecule or the vector can be cultured under conditions suitable for the expression of the peptide or the nucleotide sequence. The host cells can be any of prokaryotic and eukaryotic cells. Examples of the prokaryote can include *E. coli* and *Bacillus subtilis.* Examples of the eukaryotic cells can include cells of yeasts such as *Saccharomyces cerevisiae* and *Pichia pastoris,* insect cells such as SF9 and High 5, and animal cells such as HeLa cells, CHO cells, COS cells, and NS0. The peptide of the present invention expressed by using host cells such as eukaryotic cells can undergo a desired posttranslational modification. Examples of the posttranslational modification can include the addition of a functional group such as a sugar chain, the addition of a peptide or a protein, and the conversion of the chemical properties of an amino acid. Alternatively, the peptide of the present invention may be artificially modified as desired. Such a modified form of the peptide is also included within the scope of the "peptide" of the present invention.

The present invention also includes a method for producing the HTRA1-inhibiting peptide. The method comprises: step 1 of culturing a host cell harboring the nucleic acid molecule encoding the HTRA1-inhibiting peptide or the vector comprising the nucleotide sequence of the HTRA1-inhibiting peptide, or a cell expressing the HTRA1-inhibiting peptide; and/or step 2 of recovering the HTRA1-inhibiting peptide from the cultures obtained in step 1. An operation, such as fractionation, chromatography, or purification, known to a person skilled in the art can be applied to step 2. For example, affinity purification using the antibody of the present invention mentioned later is applicable thereto.

In some aspects of the present invention, the HTRA1-inhibiting peptide has an intramolecular disulfide bond. It may be preferred that the peptide having an intramolecular disulfide bond should be delivered to a cell compartment having an oxidizing redox environment, by using a signal sequence or the like. The oxidizing environment can be provided by the periplasm of a gram-negative bacterium such as *E. coli,* the extracellular environment of a gram-positive bacterium, the endoplasmic reticulum lumen of a eukaryotic cell, or the like. Such an environment is capable of promoting the formation of a structural disulfide bond. Alternatively, the peptide having an intramolecular disulfide bond may be prepared in the cytoplasm of a host cell such as an *E. coli* cell. In this case, the peptide can be directly acquired in a soluble folded state or recovered in the form of an inclusion body and subsequently reconstructed *in vitro.* Furthermore, a host cell having an oxidizing intracellular environment is selected, and the peptide having an intramolecular disulfide bond can also be prepared in the cytoplasm thereof. On the other hand, when the HTRA1-inhibiting peptide has no intramolecular disulfide bond, the peptide can be prepared in a cell compartment having a reducing redox environment, for example, the cytoplasm of a gram-negative bacterium.

The HTRA1-inhibiting peptide of the present invention can also be produced by other methods known to a person skilled in the art, such as chemical synthesis, for example, the solid-phase peptide synthesis method of Merrifield, et al. and an organic synthetic chemical peptide synthesis method using t-butoxycarbonyl (Boc) or 9-fluorenylmethoxycarbonyl (Fmoc), and *in vitro* translation.

In some aspects, the present invention provides an antibody which binds to a SPINK2 mutant peptide having HTRA1 inhibitory activity, and a functional fragment thereof. The antibody can be any of a polyclonal antibody and a monoclonal antibody. The monoclonal antibody is not particularly limited as long as the monoclonal antibody is an immunoglobulin or is derived therefrom. The functional fragment of the antibody is not limited as long as the functional fragment has antigen binding activity, i.e., binding activity against the SPINK2 mutant peptide. Examples thereof include both or one of heavy and light chains or a fragment thereof, an antibody fragment lacking a constant region or a Fc region, and a conjugate with an additional protein or a substance for labeling. Such an antibody and a functional fragment thereof can be prepared by a method known to a person skilled in the art and are useful in, for example, the purification of the SPINK2 mutant peptide by affinity chromatography, clinical examination related to a pharmaceutical composition comprising the peptide or use thereof, the detection of the peptide in diagnosis or the like, and immunoassay. The antibody of the present invention can be purified by affinity chromatography using the peptide of the present invention to which the antibody binds.

### 5. Pharmaceutical composition

The present invention also provides a pharmaceutical composition comprising the HTRA1-inhibiting peptide or a conjugate thereof.

The pharmaceutical composition comprising the HTRA1-inhibiting peptide of the present invention or the conjugate thereof is useful in the treatment and/or prevention of various diseases that are induced or exacerbated by HTRA1 and permit suppression of the induction or the exacerbation, cure, maintenance or amelioration of a symptom, avoidance of a secondary disease, etc. by inhibiting or suppressing the expression or function of HTRA1 (hereinafter, these diseases are referred to as "HTRA1-related diseases"). The HTRA1-related diseases also include various diseases that permit suppression of induction or exacerbation, cure, maintenance or amelioration of a symptom, avoidance of a secondary disease, etc. through a retinal protective effect. The HTRA1-inhibiting peptide has a retinal protective effect and is useful in the treatment and/or prevention of these HTRA1-related diseases.

Examples of the HTRA1-related disease can include, but are not limited to, age-related macular degeneration, retinopathy of prematurity, polypoidal choroidal vasculopathy, rheumatoid arthritis, and osteoarthritis. Examples of the age-related macular degeneration can include, but are not limited to, wet age-related macular degeneration, dry age-related macular degeneration, and geographic atrophy. Also, the pharmaceutical composition of the present invention can be used as a photoreceptor cell protection agent, a retinal protection agent, and the like (collectively referred to as a "retinal protection agent").

The age-related macular degeneration is divided into wet type and dry type. The wet type is further classified into typical age-related macular degeneration (typical AMD), polypoidal choroidal vasculopathy (PCV), and retinal angiomatous proliferation (RAP). For the wet type, there exist treatment methods such as anti-VEGF drugs and photodynamic therapy (PDT), which are however not always sufficient. For the dry type, only diet modifications or supplement intake based on Age-Related Eye Disease Study (AREDS) are practiced.

The pharmaceutical composition of the present invention can be used in the treatment or prevention of age-related macular degeneration, for example, as seen from results showing that: the nucleus count in an outer nuclear layer was decreased in a control group of rat models of retinal damage induced by light exposure, whereas the administration of the HTRA1-inhibiting peptide of the present invention before or after light exposure suppressed the decrease in nucleus count in an outer nuclear layer in an administration group of rat models (Examples 5, 10 and 14); and the area of retinal pigment epithelial cells (RPE cells) or the number of RPE cells having a cell area equal to or larger than a given value was increased in a control group of rabbit retinal damage models (mentioned later) prepared by feeding with a high-fat diet and hydroquinone, whereas the administration of the HTRA1-inhibiting peptide of the present invention suppressed this increase in an administration group of the rabbit models (Example 11). The rabbit retinal damage model takes risk factors for human dry age-related macular degeneration into consideration and is therefore excellent as a model of dry age-related macular degeneration in particular.

In a VEGF mRNA induction test using retinal pigment epithelial (RPE) cells, the administration of the HTRA1-inhibiting peptide was confirmed to suppress the expression of VEGF mRNA (Example 12). VEGF induction from retinal pigment epithelial cells is involved in the pathogenesis of wet age-related macular degeneration (Klettner A. et al., (2009), Graefes Arch Clin Exp Ophthalmol., Vol. 247: p. 1487-1492). In addition, it is considered that such morbid VEGF induction is also involved in the maintenance of the pathological condition, indicating that the HTRA1-inhibiting peptide of the present invention is useful in the treatment and prevention of dry age-related macular degeneration and wet age-related macular degeneration.

In a migration test of human umbilical vein endothelial cells (HUVEC), the administration of the HTRA1-inhibiting peptide was confirmed to suppress migration (Example 13), indicating that the HTRA1-inhibiting peptide of the present invention is useful in the treatment and prevention of wet age-related macular degeneration characterized by angiogenesis.

Wet age-related macular degeneration is progressive. Although the administration of an anti-VEGF drug can temporarily suppress the disease state, repeated recurrence with a high frequency is problematic (Yand S, et al., Drug Des Devel Ther., Vol. 2, No. 10: p. 1857-67, 2016). Problems of the drugs newly found in recent years are the development of atrophy and a decline in visual acuity in wet age-related macular degeneration patients (Berg K, et al., Acta Ophthalmologica, Vol. 95, No. 8: p. 796-802, 2017). The administration of the HTRA1-inhibiting peptide of the present invention alone or by combined use with an anti-VEGF drug can exert a therapeutic effect on wet age-related macular degeneration (including polypoidal choroidal vasculopathy and retinal angiomatous proliferation). In addition, the prophylactic administration of the peptide can prevent or delay the recurrence of the disease state and delay the start or restart of treatment with an anti-VEGF drug, for example. Furthermore, the peptide works suppressively on the development of atrophy in wet age-related macular degeneration patients and is therefore capable of bringing about long-term benefits in terms of visual acuity.

The HTRA1-inhibiting peptide of the present invention is excellent in terms of tissue penetration (Example 11) and also excellent in terms of its physical properties, stability, safety, kinetics after administration, productivity, etc. and can thus preferably be contained as an active ingredient in a pharmaceutical composition.

The pharmaceutical composition of the present invention can contain a therapeutically or prophylactically effective amount of the HTRA1-inhibiting peptide or the conjugate thereof and a pharmaceutically acceptable diluent, carrier, solubilizer, emulsifier, preservative and/or adjuvant.

The term "therapeutically or prophylactically effective amount" is used to mean an amount that exerts a therapeutic or prophylactic effect on a specific disease through a dosage form and an administration route, and is synonymous with a "pharmacologically effective amount".

The pharmaceutical composition of the present invention can contain a substance for altering, maintaining, or retaining pH, osmotic pressure, viscosity, transparency, color, isotonicity, sterility, or the stability, solubility, sustained release rate, absorptivity, penetration, dosage form, strength, properties, shape, etc. of the composition or the peptide of the present invention or the conjugate thereof contained therein (hereinafter, referred to as a "pharmaceutical substance"). The pharmaceutical substance is not particularly limited as long as the substance is pharmacologically acceptable. For example, no or low toxicity is a property preferably possessed by the pharmaceutical substance.

Examples of the pharmaceutical substance can include the following substances, but are not limited thereto: amino acids such as glycine, alanine, glutamine, asparagine, histidine, arginine, and lysine; antimicrobial agents; antioxidants such as ascorbic acid, sodium sulfate, and sodium bisulfite; buffers such as phosphate, citrate, or borate buffers, sodium bicarbonate, and Tris-HCl solutions; fillers such as mannitol and glycine; chelating agents such as ethylenediaminetetraacetic acid (EDTA); complexing agents such as caffeine, polyvinylpyrrolidine, β-cyclodextrin, and hydroxypropyl-β-cyclodextrin; bulking agents such as glucose, mannose, and dextrin; other hydrocarbons such as monosaccharides, disaccharides, glucose, mannose, and dextrin; coloring agents; flavor agents; diluents; emulsifiers; hydrophilic polymers such as polyvinylpyrrolidine; low-molecular-weight polypeptides; salt-forming counterions; antiseptics such as benzalkonium chloride, benzoic acid, salicylic acid, thimerosal, phenethyl alcohol, methylparaben, propylparaben, chlorhexidine, sorbic acid, and hydrogen peroxide; solvents such as glycerin, propylene glycol, and polyethylene glycol; sugar alcohols such as mannitol and sorbitol; suspending agents; surfactants such as PEG, sorbitan ester, polysorbates such as polysorbate 20 and polysorbate 80, triton, tromethamine, lecithin, and cholesterol; stability enhancers such as sucrose and sorbitol; elasticity enhancers such as sodium chloride, potassium chloride, mannitol, and sorbitol; transporting agents; diluents: excipients; and/or pharmaceutical adjuvants.

Such a pharmaceutical substance is added to the HTRA1-inhibiting peptide in an amount of 0.001 to 1000 times, preferably 0.01 to 100 times, and more preferably 0.1 to 10 times higher than the weight of the HTRA1-inhibiting peptide.

A liposome containing the HTRA1-inhibiting peptide or the conjugate thereof, or a pharmaceutical composition containing a modified form comprising the HTRA1-inhibiting peptide or the conjugate thereof conjugated with a liposome is also included in the pharmaceutical composition of the present invention.

An excipient or a carrier is not particularly limited as long as the excipient or the carrier is usually a liquid or a solid and is water for injection, normal saline, an artificial cerebrospinal fluid, or other substances for use in preparations for oral administration or parenteral administration. Examples of the normal saline can include neutral normal saline or normal saline containing serum albumin.

Examples of the buffer can include a Tris buffer prepared to bring the final pH of the pharmaceutical composition to 7.0 to 8.5, an acetate buffer prepared to bring the final pH thereof to 4.0 to 5.5, a citrate buffer prepared to bring the final pH thereof to 5.0 to 8.0, and a histidine buffer prepared to bring the final pH thereof to 5.0 to 8.0.

The pharmaceutical composition of the present invention is a solid, a liquid, a suspension, or the like. Another example of the pharmaceutical composition of the present invention can include freeze-dried preparations. The freeze-dried preparations can be formed using an excipient such as sucrose.

The administration route of the pharmaceutical composition of the present invention may be any of ocular instillation, enteral administration, local administration, and parenteral administration. Examples thereof can include conjunctival instillation, intravitreal administration, intravenous administration, intraarterial administration, intramuscular administration, intradermal administration, subcutaneous administration, intraperitoneal administration, transdermal administration, intraosseous administration, and intraarticular administration.

The recipe of the pharmaceutical composition can be determined according to an administration method, the HTRA1 binding affinity of the HTRA1-inhibiting peptide, etc. The HTRA1-inhibiting peptide of the present invention having stronger inhibitory activity (smaller IC₅₀ value) against the target HTRA1 or having higher affinity (lower K_{D} value) for the HTRA1 protein is capable of exerting its medicinal effects at a lower dose.

The dose of the HTRA1-inhibiting peptide of the present invention or the conjugate thereof is not limited as long as the dose is a pharmacologically effective amount. The dose can be appropriately determined according to the species of an individual, the type of disease, symptoms, sex, age, pre-existing conditions, the binding affinity of the peptide for the HTRA1 protein or its biological activity, and other factors. The dose is usually 0.01 to 1000 mg/kg, and preferably 0.1 to 100 mg/kg, which can be administered once every day to every 180 days or twice or three or more times a day.

Examples of the form of the pharmaceutical composition can include injections (including freeze-dried preparations and drip infusions), suppositories, transnasal absorption preparations, transdermal absorption preparations, sublingual agents, capsules, tablets, ointments, granules, aerosols, pills, powders, suspensions, emulsions, eye drops, and biological implant formulations.

The pharmaceutical composition comprising the HTRA1-inhibiting peptide or the conjugate thereof as an active ingredient can be administered concurrently with or separately from an additional medicament. For example, the pharmaceutical composition comprising the HTRA1-inhibiting peptide or the conjugate thereof as an active ingredient may be administered after administration of the additional medicament, or the additional medicament may be administered after administration of the pharmaceutical composition. Alternatively, the pharmaceutical composition and the additional medicament may be administered concurrently. For concurrent administration, the HTRA1-inhibiting peptide or the conjugate thereof and the additional medicament may be contained in a single preparation or may be contained in separate preparations (a plurality of preparations).

Examples of the additional medicament used in combination with the pharmaceutical composition of the present invention can include anti-VEGF agents, anti-inflammatory agents, inflammatory cytokine-neutralizing agents, and complement activation pathway inhibitors. The anti-VEGF agents are classified into anti-VEGF antibodies, VEGF inhibitors, VEGF receptor antagonists and soluble VEGF receptors, etc. and include bevacizumab, ranibizumab, aflibercept, pegaptanib, brolucizumab, and the like. The anti-inflammatory agent is not particularly limited as long as the anti-inflammatory agent can be locally administered in order to suppress intraocular or intraarticular inflammation. Examples of the inflammatory cytokine-neutralizing agent include anti-TNFα antibodies, anti-interleukin-6 (hereinafter, referred to as "IL-6") antibodies, anti-IL-6 receptor antibodies, and soluble TNF receptors and can specifically include infliximab, adalimumab, golimumab, certolizumab, tocilizumab, and etanercept. Examples of the complement activation pathway inhibitor can include lampalizumab. These medicaments are suitable for the treatment or prevention of HTRA1-related diseases and can also be combined with the pharmaceutical composition of the present invention in the treatment or prevention of diseases other than HTRA1-related diseases.

One of these additional medicaments may be used, or two or three or more thereof may be administered or received. These approaches are collectively referred to as "combined use with the additional medicament" of or "combination with the additional medicament" and the pharmaceutical composition of the present invention. The pharmaceutical composition of the present invention comprising the additional medicament in addition to the antibody of the present invention, a binding fragment thereof, or a modified form of the antibody or the fragment, or used in combination with additional therapy is also included in the present invention as an aspect of the "combined use with the additional medicament" or the "combination with the additional medicament".

The present invention also provides a SPINK2 mutant peptide or conjugate thereof, as defined herein, for use in a method for treating or preventing a HTRA1-related disease such as age-related macular degeneration, use of the HTRA1-inhibiting peptide of the present invention or the conjugate thereof for preparing a pharmaceutical composition for the treatment or prevention of the disease, and use of the HTRA1-inhibiting peptide or the conjugate thereof for the treatment or prevention of the disease. The present invention also includes a kit for treatment or prevention comprising the HTRA1-inhibiting peptide of the present invention or the conjugate thereof.

The present invention further provides a polynucleotide comprising a nucleotide sequence encoding the amino acid sequence of the HTRA1-inhibiting peptide or the conjugate thereof, a vector comprising the polynucleotide, and a pharmaceutical composition comprising the polynucleotide or the vector or comprising a cell expressing the HTRA1-inhibiting peptide of the present invention or the conjugate thereof. For example, the polynucleotide and the vector can be applied to the gene therapy of HTRA1-related diseases by use of a known approach. The cell can be applied to the cell therapy of HTRA1-related diseases by use of a known approach. Also, the polynucleotide or the vector can be transferred to, for example, autologous cells or allogeneic cells (homologous cells) to prepare cells for cell therapy. Such a polynucleotide and vector are also included as compositions for cell therapy drug preparation in the present invention. However, the form of the pharmaceutical composition of the present invention comprising the polynucleotide, the vector, the cell, or the like is not limited to those described above.

### 6. Composition for diagnosis and methods for detecting and separating HTRA1

The HTRA1-inhibiting peptide of the present invention or the conjugate thereof may have HTRA1 binding activity in addition to HTRA1 protease inhibitory activity and can be used in various studies such as use as a positive control in studies to search for HTRA1 inhibitors, the detection of HTRA1, examination and diagnosis using the detection, the separation of HTRA1, reagents and other purposes. For the detection or separation of HTRA1, at least one of the peptide of the present invention and HTRA1 can be immobilized.

The present invention provides a composition for detection or for diagnosis (hereinafter, collectively referred to as a "composition for diagnosis") comprising the peptide of the present invention which binds to HTRA1, or the conjugate thereof.

The composition for diagnosis of the present invention is useful in the examination or diagnosis of HTRA1-related diseases, HTRA1 expression, etc. In the present invention, examples of the examination or the diagnosis include, but are not limited to, the determination or measurement of the risk of acquiring a disease, the determination of the presence or absence of a disease, the measurement of the degree of progression or exacerbation, the measurement or determination of the effect of medication with a pharmaceutical composition comprising the HTRA1-inhibiting peptide or the conjugate thereof, the measurement or determination of the effect of treatment other than medication, the measurement of the risk of recurrence, and the determination of the presence or absence of recurrence.

The composition for diagnosis of the present invention is useful in the identification of a recipient individual for the peptide of the present invention or the conjugate thereof, a composition comprising the peptide or the conjugate thereof, or a pharmaceutical composition comprising the peptide or the conjugate thereof.

The composition for diagnosis can contain a pH buffer, an osmotic pressure adjuster, salts, a stabilizer, an antiseptic, a developer, a sensitizer, an aggregation prevention agent, and the like.

The present invention also provides an in vitro method for examining or diagnosing a HTRA1-related disease, use of the peptide of the present invention for preparing a composition for the diagnosis of the disease, and use of the peptide of the present invention which binds to HTRA1, or the conjugate thereof for the examination or diagnosis of the disease. The present invention also includes a kit for examination or diagnosis comprising the peptide of the present invention or the conjugate thereof.

The examination or diagnosis method comprising the peptide of the present invention which binds to HTRA1 is desirably sandwich ELISA. Alternatively, a usual detection method such as ELISA, RIA, ELISPOT, dot blot, an Ouchterlony method, CIE, CLIA, or flow cytometry may be used. The examination or the diagnosis is also achieved by a method based on an immunoprecipitation method.

The present invention also provides a method for detecting or measuring HTRA1 in a test sample. Such a detection or measurement method can employ the composition for diagnosis of the present invention. HTRA1 in a test sample can be detected by: contacting the HTRA1-inhibiting peptide or the conjugate thereof with the test sample (step 1); and subsequently measuring the amount of HTRA1 bound to the peptide (step 2). Step 1 can involve, for example, immobilizing a conjugate of the HTRA1-inhibiting peptide with an immunoglobulin Fc region onto magnetic beads via protein G, and adding the test sample thereto. Step 2 can involve, for example, separating the magnetic beads, and analyzing a soluble protein precipitated with the beads by SDS-PAGE or Western blot to detect HTRA1. In addition to a human- or nonhuman animal-derived sample, even an artificially treated sample such as a recombinant protein can be subjected to this measurement. Examples of the organism individual-derived test sample can include, but are not limited to, blood, synovial fluid, ascitic fluid, lymph, cerebrospinal fluid, bronchoalveolar lavage, saliva, sputum, tissue homogenate supernatants, and tissue sections.

The HTRA1 detection can be carried out not only *in vitro* but *in vivo.* In the case of diagnostic imaging, the HTRA1-inhibiting peptide or the conjugate thereof labeled with a pharmaceutically acceptable radionuclide or light emitter can be used. Step 1 can involve, for example, administering the labeled peptide or conjugate thereof to a test subject. Step 2 can involve, for example, obtaining an image by use of a diagnostic imaging technique such as PET/CT, and determining or examining the presence of HTRA1.

The peptide or the conjugate thereof contained in the composition for diagnosis of the present invention binds to HTRA1, and preferably has HTRA1-specific binding activity.

The present invention also includes a method for identifying a recipient individual for the pharmaceutical composition of the present invention. In such an identification method, HTRA1 in a sample derived from an individual is measured using the HTRA1-binding peptide of the present invention, and the individual can be determined to be positive when HTRA1 is detected in the sample or when a larger amount of HTRA1 is detected therein as compared with the amount of HTRA1 detected in a sample derived from a healthy individual. This method can employ the composition for diagnosis of the present invention.

In a preferred aspect of the identification method, the individual has a HTRA1-related disease or has a risk of acquiring the disease.

The pharmaceutical composition of the present invention can be administered to the individual determined to be positive in the identification method.

HTRA1 can be specifically separated from a sample in which the HTRA1 coexists with other components, using the peptide of the present invention having HTRA1-specific binding activity, or the conjugate thereof. The release of the HTRA1 from the peptide can be nonselectively performed, for example, in the presence of relatively high ionic strength, low pH, moderate denaturation conditions, or chaotropic salt, and is preferably performed without attenuating the protease activity of the HTRA1.

### 7. Method for identifying therapeutic drug or prophylactic drug for HTRA1-related disease

In an aspect, the present invention provides a method for identifying a therapeutic drug or a prophylactic drug for a HTRA1-related disease, and preferably age-related macular degeneration, or a candidate thereof by using HTRA1 inhibitory activity as an indicator. The method can comprise: step 1 of incubating HTRA1 protease and a substrate in the presence or absence of a test substance (or in the presence of a vehicle); step 2 of determining HTRA1 protease activity in the presence and absence of the test substance; and/or step 3 of determining the test substance as a therapeutic drug or a prophylactic drug for age-related macular degeneration, or a candidate thereof when the HTRA1 protease activity in the presence of the test substance is smaller than the HTRA1 protease activity in the absence of the test substance. The test substance may be peptidic or nonpeptidic. The peptidic test substance is not limited to SPINK2 mutants. Examples thereof can include, but are not limited to, antibodies, peptides other than SPINK2 mutants having a non-immunoglobulin protein framework, and HTRA1 substrate analogs. Examples of the nonpeptidic test substance can include, but are not limited to, synthetic low-molecular compounds and nucleic acids. One or two or more of the steps described above can also be preferably included in a method for identifying a substance having a retinal protective effect, or a candidate thereof. The present invention also relates to a method for identifying a substance having a retinal protective effect, or a candidate thereof.

### 8. Rabbit retinal damage model

The present invention also provides a rabbit model of retinal damage caused by loading with a high-fat diet (hereinafter, referred to as "HFD") containing hydroquinone (hereinafter, referred to as "HQ"), a method for preparing the model, a testing method using the model, etc.

This model can employ an arbitrary white rabbit, such as NZW or JW, at age 2 or over, regardless of sex.

HFD can contain an arbitrary quantity of an arbitrary fat or oil, for example, 0.1 to 2% (w/v) cholesterol, 1 to 10% (w/v) coconut oil, 1 to 10% (w/v) peanut oil, 1 to 10% (w/v) soybean oil, 10 to 20% (w/v) beef tallow, 10 to 50% (w/v) lard, or 1 to 10% (w/v) corn oil, though the component is not limited thereto as long as the component is suitable for the preparation of the model.

The amount of HQ contained in HQ-HFD is 0 to 4% (w/v), preferably 0.5 to 3.5% (w/v), more preferably 1.0 to 3.0% (w/v), still more preferably 2.2 to 2.8% (w/v), and most preferably 2.4% (w/v).

The feeding period of HQ-HFD is 3 to 6 months, preferably 3.5 to 5 months, and more preferably 4 months. The continuation of feeding for 8 months or longer should be avoided.

The model of the present invention is preferred because the model is more similar to humans in the size of the eyeball and functions, as compared with mouse models (Non Patent Literature 18 and 19). Conventional rabbit models (Non Patent Literature 20) require 8 months for their preparation. By contrast, the model of the present invention can be prepared in a shorter period and furthermore, is more preferred as a model of age-related macular degeneration because the model can induce hypertrophy of RPE cells, which is unclear in conventional models.

The retinal pigment epithelial cells (RPE cells) of the model of the present invention are hypertrophied as compared with the RPE cells of a normal rabbit, manifesting the early pathological condition of age-related macular degeneration. On the other hand, RPE cell hypertrophy was unable to be visually confirmed when 10-week-old rabbits similar to conventional rabbit models (Non Patent Literature 20) were fed with HQ-HFD for 4 months.

A therapeutic drug and/or a prophylactic drug for age-related macular degeneration, or a retinal protection agent can be identified by using this model manifesting the pathological condition. The identification method can comprise the steps of: (i) measuring hypertrophy of retinal pigment epithelial cells in the rabbit of this model with or without administration of a test substance; and (ii) determining the test substance to be positive when the hypertrophy of retinal pigment epithelial cells with administration of the test substance is suppressed as compared with the hypertrophy of retinal pigment epithelial cells without administration thereof. The measurement in step (i) is preferably the measurement of an average area of the retinal pigment epithelial cells and/or the number of hypertrophied retinal pigment epithelial cells.

### Examples

Hereinafter, some aspects of the present invention will be described in more detail with reference to Examples. However, the present invention is not limited to these examples.

In the following examples, unless otherwise specified, individual operations regarding genetic manipulation have been carried out according to the method described in "Molecular Cloning" (Sambrook, J., Fritsch, E. F. and Maniatis, T., published by Cold Spring Harbor Laboratory Press in 1982 or 1989) or other methods described in experimental manuals used by persons skilled in the art, or, when commercially available reagents or kits have been used, the examples have been carried out in accordance with the instructions included in the commercially available products.

### Example 1. Preparation of HTRA1-inhibiting peptide

### (1-1) Construction of HTRA1-inhibiting peptide expression vector

### (1-1-1) Construction of pET 32a (modified)_HTRA1-inhibiting peptide

First, a HTRA1-inhibiting peptide expression vector with SPINK2 scaffold as a backbone was constructed. An inhibitor fragment was amplified by PCR ((94°C for 15 sec, 60°C for 30 sec, and 68°C for 30 sec) × 30 cycles) with the nucleotide sequence (SEQ ID NOs: 4, 6, 8, 10, 12, 14, 16, 18, 20 and 22) of each inhibiting peptide and the nucleotide sequence (SEQ ID NO: 2) of SPINK2 as templates using the following primers and KOD-plus- (Toyobo Co., Ltd.).
Primer 1: 5'-AAAAGAATTCTGATCCGCAGTTTGGTCTGTTTAG-3'
Primer 2: 5'-AAAACTCGAGTTATGCGGCCGCAGACGCGCCGCACGGACC-3'

Each amplified fragment was subjected to agarose gel electrophoresis. Then, the desired DNA fragment was excised from the gel, and DNA was prepared using a QIAquick Gel Extraction Kit (Qiagen N.V.). The prepared DNA fragment and pET 32a (modified) were each treated with restriction enzymes EcoRI (New England BioLabs Inc.) and XhoI (New England BioLabs Inc.) at 37°C for 1 hour or longer. After agarose gel electrophoresis, the desired DNA fragments were excised from the gel and purified using a QIAquick PCR Purification Kit (Qiagen N.V.). The purified fragments were reacted overnight at 16°C for a ligation reaction using T4 DNA Ligase (New England BioLabs Inc.). The ligation solution was added to *E*. *coli* JM109 (Toyobo Co., Ltd.), and the mixture was left standing on ice for 30 minutes, then heat-treated at 42°C for 45 seconds, further left standing on ice for 5 minutes, and inoculated to a 2YT plate containing 0.1 mg/ml ampicillin, followed by static culture overnight at 37°C to transform the *E. coli.* On the next day, the transformed *E. coli* was inoculated to a Terrific Broth medium (Invitrogen Corp.) containing 0.1 mg/ml ampicillin and cultured overnight at 37°C. Then, plasmid DNA was recovered using a QIAprep 96 Turbo Miniprep Kit (Qiagen N.V.) (hereinafter, this treatment is referred to as "miniprep treatment") and subjected to sequence analysis to construct "pET 32a (modified)_HTRA1-inhibiting peptide".

### (1-1-2) Construction of pET 32a_HTRA1-inhibiting peptide_Kex2

Likewise, an inhibitor fragment was amplified by PCR ((94°C for 15 sec, 60°C for 30 sec, and 68°C for 30 sec) × 30 cycles) with the sequence (Sequence Listing) of each inhibitor and the nucleotide sequence of SPINK2 as templates using the following primers and KOD-plus-(Toyobo Co., Ltd.).
Primer 3: 5'-AAAAGGATCCCTGGACAAACGTGATCCGCAGTTTGGTCTGTTTAG-3'
Primer 4: 5'-AAAACTCGAGTTAGCCGCCGCACGGACCATTGCGAATAATTTTA-3'

Each amplified fragment was subjected to agarose gel electrophoresis. Then, the desired DNA fragment was excised from the gel, and DNA was prepared using a QIAquick Gel Extraction Kit (Qiagen N.V.). The prepared DNA fragment and pET 32a (Novagen) were each treated with restriction enzymes BamHI (New England BioLabs Inc.) and XhoI (New England BioLabs Inc.) at 37°C for 1 hour or longer. After agarose gel electrophoresis, the desired DNA fragments were excised from the gel and purified using a QIAquick PCR Purification Kit (Qiagen N.V.). The purified fragments were reacted overnight at 16°C for a ligation reaction using T4 DNA Ligase (New England BioLabs Inc.). The ligation solution was added to *E*. *coli* JM109 (Toyobo Co., Ltd.), and the mixture was left standing on ice for 30 minutes, then heat-treated at 42°C for 45 seconds, further left standing on ice for 5 minutes, and inoculated onto a 2YT plate containing 0.1 mg/ml ampicillin, followed by static culture overnight at 37°C to transform the *E. coli.* The transformed *E. coli* was cultured, and miniprep and sequence analysis were then carried out to construct "pET 32a_HTRA1-inhibiting peptide_Kex2". The operation was performed in accordance with the method described in (1-1-1).

### (1-2) Expression and purification of HTRA1-inhibiting peptide

*E. coli* Origami B (DE3) (Novagen) was transformed with the vector pET 32a (modified)_HTRA1-inhibiting peptide constructed in (1-1-1), and cultured at 37°C using a 2YT medium containing 0.1 mg/ml ampicillin. Then, IPTG (final concentration: 1 mM) was added thereto, and the *E. coli* was cultured overnight at 16°C. On the next day, after harvest by centrifugation (3,000 g, 20 min, 4°C), a lysate was prepared using BugBuster Master Mix (Novagen), and a His tag fusion protein of interest was purified using a TALON Metal Affinity Resin (Clontech Laboratories, Inc.). Next, a thioredoxin tag and the desired protein were cleaved using a Thrombin Cleavage Capture Kit (Novagen) and purified using TALON. The resultant was subjected to gel filtration chromatography (Superdex 75 10/300 GL) or reverse-phase chromatography (YMC-Pack ODS-AM) to prepare a HTRA1-inhibiting peptide. The obtained peptide was conjugated at its N terminus with a moiety consisting of S tag and a linker (SEQ ID NO: 31: Figure 43) and at its C terminus with a C-terminal hexamer (SEQ ID NO: 32: Figure 44) instead of Gly-Gly.

Likewise, *E. coli* Origami B (DE3) (Novagen) was transformed with the vector pET 32a_HTRA1-inhibiting peptide_Kex2 constructed in (1-1-2), and cultured at 37°C using a 2YT medium containing 0.1 mg/ml ampicillin. Then, IPTG (final concentration: 1 mM) was added thereto, and the *E. coli* was cultured overnight at 16°C. On the next day, after harvest by centrifugation (3,000 g, 20 min, 4°C), a lysate was prepared using BugBuster Master Mix (Novagen), and a His tag fusion protein of interest was purified using a TALON Metal Affinity Resin (Clontech Laboratories, Inc.). Next, a thioredoxin tag and the desired protein were cleaved using Kex2 (*Saccharomyces cerevisiae:* Accession CAA96143) and purified using TALON. The resultant was subjected to gel filtration chromatography (Superdex 75 10/300 GL) or reverse-phase chromatography (YMC-Pack ODS-AM) to prepare a HTRA1-inhibiting peptide (with neither the N terminus nor the C terminus conjugated with a tag, a linker or the like).

### Example 2. Evaluation of HTRA1-inhibiting peptide for HTRA1 inhibitory activity

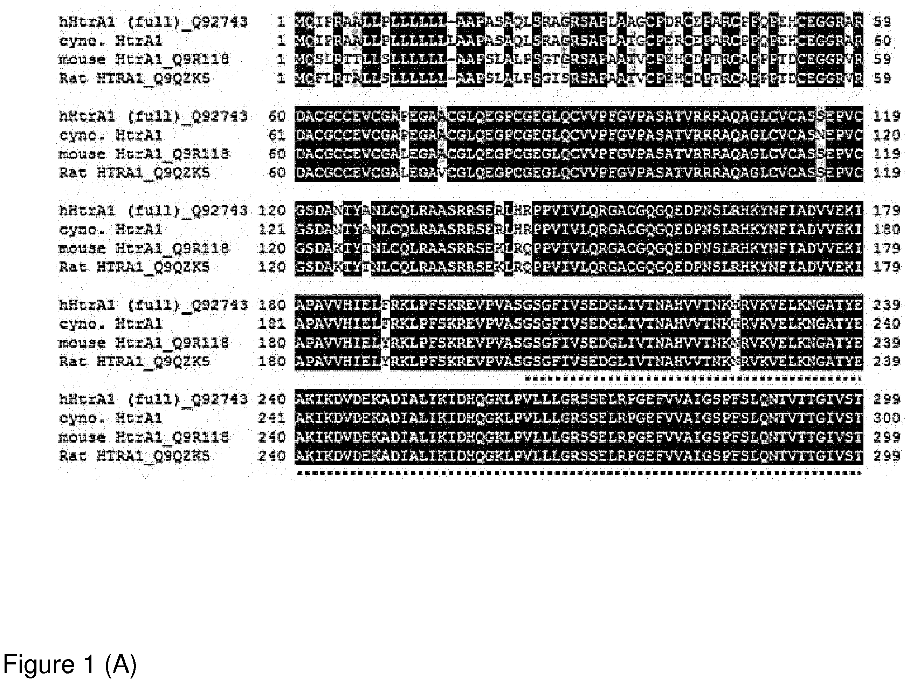

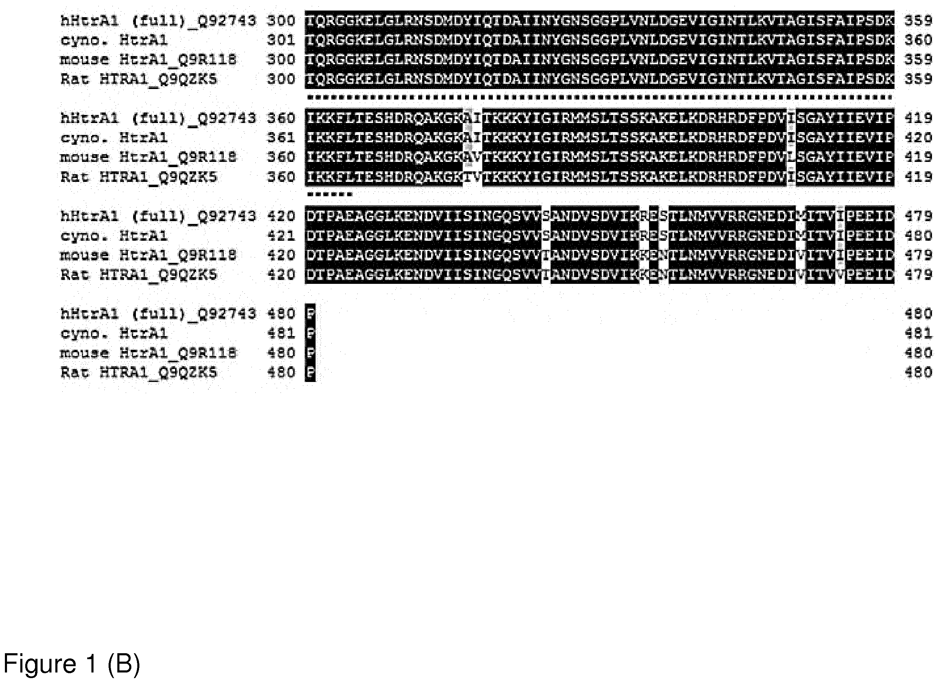

Sequence similarity among human, mouse, rat, and monkey HTRA1 is shown in Figure 1. A primary sequence constituting a HTRA1 protease domain (204Gly to 364Leu), which is an enzymatically active domain, is completely identical between the human and the monkey. The human and mouse or rat HTRA1 protease domain sequences differ by 1 residue. However, this residue is structurally positioned on a side opposite to the active center of the enzyme and was therefore presumed to have no influence on the active center of the enzyme (Figure 1). Accordingly, the HTRA1 protease domain has effectively the same sequence, regardless of the species (human/mouse/rat/monkey). Thus, no particular mention was made about the species.

### (2-1) Preparation of HTRA1 protease domain HTRA1 (cat)

### (2-1-1) Construction of pET 21b_HTRA1 (cat)

The protease domain (158Gly to 373Lys), except for the N-terminal domain and the PDZ domain, of human HTRA1 (Q92743) was used as HTRA1 (cat) to construct a HTRA1 (cat) expression vector. The desired DNA fragment was amplified by PCR ((94°C for 15 sec, 60°C for 30 sec, and 68°C for 45 sec) × 30 cycles) with a human HTRA1-inserted plasmid (GeneCopoeia, Inc.; GC-M0558) as a template using the following primers and KOD-plus- (Toyobo Co., Ltd.).
Primer 5: 5'-AAACATATGGGGCAGGAAGATCCCAACAGTTTGC-3'
Primer 6: 5'-AAACTCGAGTTTGGCCTGTCGGTCATGGGACTC-3'

The amplified fragment was subjected to agarose gel electrophoresis. Then, the desired DNA fragment was excised from the gel, and DNA was prepared using a QIAquick Gel Extraction Kit (Qiagen N.V.). The prepared DNA fragment and pET 32a (Novagen) were each treated with restriction enzymes NdeI (New England BioLabs Inc.) and XhoI (New England BioLabs Inc.) at 37°C for 1 hour or longer. After agarose gel electrophoresis, the desired DNA fragments were excised from the gel and purified using a QIAquick PCR Purification Kit (Qiagen N.V.). The purified fragments were reacted overnight at 16°C for a ligation reaction using T4 DNA Ligase (New England BioLabs Inc.). The ligation solution was added to *E*. *coli* JM109 (Toyobo Co., Ltd.), and the mixture was left standing on ice for 30 minutes, then heat-treated at 42°C for 45 seconds, further left standing on ice for 5 minutes, and inoculated onto a 2YT plate containing 0.1 mg/ml ampicillin, followed by static culture overnight at 37°C to transform the *E. coli.* The transformed *E. coli* was cultured, and miniprep and sequence analysis were then carried out to construct "pET 21b_HTRA1 (cat)". The operation was performed in accordance with the method described in (1-1-1).

### (2-1-2) Preparation of HTRA1 (cat)

*E. coli* BL21 (DE3) (Novagen) was transformed with the constructed pET 21b_HTRA1 (cat) and cultured at 37°C using a 2YT medium containing 0.1 mg/ml ampicillin. Then, IPTG (final concentration: 1 mM) was added thereto, and the *E. coli* was cultured overnight at 28°C. After harvest, a lysate was prepared by suspending in a phosphate buffer (50 mM sodium phosphate and 300 mM NaCl) containing 1 mg/ml lysozyme and ultrasonication, and the desired His tag fusion protein was recovered using TALON (Clontech Laboratories, Inc.). The resultant was subjected to gel filtration chromatography (Superdex 200 10/300 GL) to purify HTRA1 (cat).

### (2-2) Preparation of full-length HTRA1 (HTRA1 (full))

### (2-2-1) Construction of pcDNA3.1_HTRA1 (full)_His

The desired DNA fragment was amplified by PCR ((94°C for 15 sec, 60°C for 30 sec, and 68°C for 90 sec) × 30 cycles) with synthesized human HTRA1 (Q92743) DNA (GeneArt) as a template using the following primers and KOD-plus- (Toyobo Co., Ltd.).
Primer 7: 5'-AAAAGAATTCGCCACCATGCAGATTCCTAGAGCCG-3'
Primer 8: 5'-AAAACTCGAGTCAGTGGTGATGGTGGTGGTGGCCGG-3'

The amplified fragment was subjected to agarose gel electrophoresis. Then, the desired DNA fragment was excised from the gel, and DNA was prepared using a QIAquick Gel Extraction Kit (Qiagen N.V.). The prepared DNA fragment and pcDNA3.1 (Thermo Fisher Scientific Inc.) were each treated with restriction enzymes EcoRI (New England BioLabs Inc.) and XhoI (New England BioLabs Inc.) at 37°C for 1 hour or longer. After agarose gel electrophoresis, the desired DNA fragments were excised from the gel and purified using a QIAquick PCR Purification Kit (Qiagen N.V.). The purified fragments were reacted overnight at 16°C for a ligation reaction using T4 DNA Ligase (New England BioLabs Inc.). The ligation solution was added to *E. coli* JM109 (Toyobo Co., Ltd.), and the mixture was left standing on ice for 30 minutes, then heat-treated at 42°C for 45 seconds, further left standing on ice for 5 minutes, and inoculated onto a 2YT plate containing 0.1 mg/ml ampicillin, followed by static culture overnight at 37°C to transform the *E. coli.* The transformed *E. coli* was cultured, and miniprep and sequence analysis were then carried out to construct "pcDNA3.1_HTRA1 (full)_His". The operation was performed in accordance with the method described in (1-1-1).

### (2-2-2) Construction of pcDNA3.3_HTRA1 (full)_FLAG_His

Fragment A was amplified by PCR ((94°C for 15 sec, 60°C for 30 sec, and 68°C for 90 sec) × 30 cycles) with pcDNA3.1_HTRA1 (full)_His constructed in (2-2-1) as a template using the following primers and KOD-plus-(Toyobo Co., Ltd.).
Primer 7
Primer 9: 5'-CTTGTCGTCATCGTCCTTGTAGTCGCCGGGGTCGATTTCCTC-3'

Next, fragment B was amplified by PCR ((94°C for 15 sec, 60°C for 30 sec, and 68°C for 10 sec) × 30 cycles) using the following primers and KOD-plus- (Toyobo Co., Ltd.) .
Primer 10: 5'-GCGACTACAAGGACGATGACGACAAGCACCACCACCATCATCAC-3'
Primer 11: 5'-AAAAACTCGAGCTAGTGATGATGGTGGTGGTGCTTGTCGTC-3'

The desired DNA fragment was amplified by PCR ((94°C for 15 sec, 60°C for 30 sec, and 68°C for 90 sec) × 30 cycles) with fragment A and fragment B as templates using primers 7 and 11 and KOD-plus- (Toyobo Co., Ltd.). The amplified fragment was subjected to agarose gel electrophoresis. Then, the desired DNA fragment was excised from the gel, and DNA was prepared using a QIAquick Gel Extraction Kit (Qiagen N.V.). The prepared DNA fragment and pcDNA3.3 (Thermo Fisher Scientific Inc.) were each treated with restriction enzymes EcoRI (New England BioLabs Inc.) and XhoI (New England BioLabs Inc.) at 37°C for 1 hour or longer. After agarose gel electrophoresis, the desired DNA fragments were excised from the gel and purified using a QIAquick PCR Purification Kit (Qiagen N.V.). The purified fragments were reacted overnight at 16°C for a ligation reaction using T4 DNA Ligase (New England BioLabs Inc.). The ligation solution was added to *E. coli* JM109 (Toyobo Co., Ltd.), and the mixture was left standing on ice for 30 minutes, then heat-treated at 42°C for 45 seconds, further left standing on ice for 5 minutes, and inoculated onto a 2YT plate containing 0.1 mg/ml ampicillin, followed by static culture overnight at 37°C to transform the *E. coli.* The transformed *E. coli* was cultured, and miniprep and sequence analysis were then carried out to construct "pcDNA3.3_HTRA1 (full)_FLAG_His". The operation was performed in accordance with the method described in (1-1-1).

### (2-2-3) Preparation of HTRA1 (full)

FreeStyle 293F (Thermo Fisher Scientific Inc.) was transfected with pcDNA3.3_HTRA1 (full)_FLAG_His constructed in (2-2-2) using Polyethylenimine Max (Polysciences, Inc.). Six days later, a culture supernatant was recovered. A His tag fusion protein was recovered using HisTrap excel (GE Healthcare), and HTRA1 (full) was further purified using an ANTI-FLAG M2 Affinity Agarose Gel (Sigma-Aldrich Co. LLC).

### (2-3) Preparation of inactive HTRA1 mutant HTRA1 (S328A)

### (2-3-1) Construction of pcDNA3.3_HTRA1(S328A)_FLAG_His

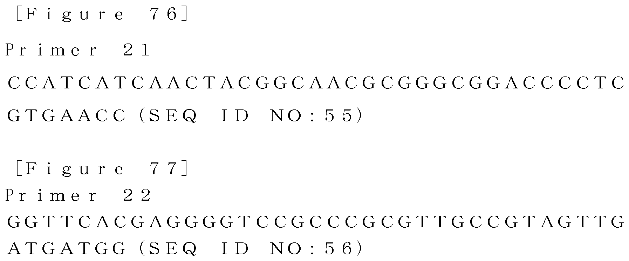

In order to construct an inactive HTRA1 mutant HTRA1 (S328A) expression vector, PCR ((95°C for 30 sec, 55°C for 1 min, and 68°C for 7 min) × 18 cycles) was carried out with the vector "pcDNA3.3_HTRA1 (full)_FLAG_His" constructed in Example (2-2-2) as a template using the following primers and QuikChange II Site-Directed Mutagenesis Kits (Agilent Technologies Japan, Ltd.).
Primer 21: 5'-CCATCATCAACTACGGCAACGCGGGCGGACCCCTCGTGAACC-3' (SEQ ID NO: 55: Figure 76)
Primer 22: 5'-GGTTCACGAGGGGTCCGCCCGCGTTGCCGTAGTTGATGATGG-3' (SEQ ID NO: 56: Figure 77)

After the PCR reaction, *E. coli* JM109 (Toyobo Co., Ltd.) was transformed with the PCR reaction solution treated with DpnI according to the protocol attached to the kit. The transformed *E. coli* was cultured, and miniprep and sequence analysis were then carried out to construct "pcDNA3.3_HTRA1(S328A)_FLAG_His". The operation was performed in accordance with the method described in (1-1-1).

### (2-3-2) Preparation of HTRA1 (S328A)

HTRA1 (S328A) was expressed using FreeStyle 293F according to the method described in (2-2-3), and HTRA1 (S328A) was prepared by affinity purification.

### Example 3. Evaluation of HTRA1-inhibiting peptide for HTRA1 inhibitory activity

### (3-1) Evaluation of HTRA1-inhibiting peptide for HTRA1 inhibitory activity using peptide substrate

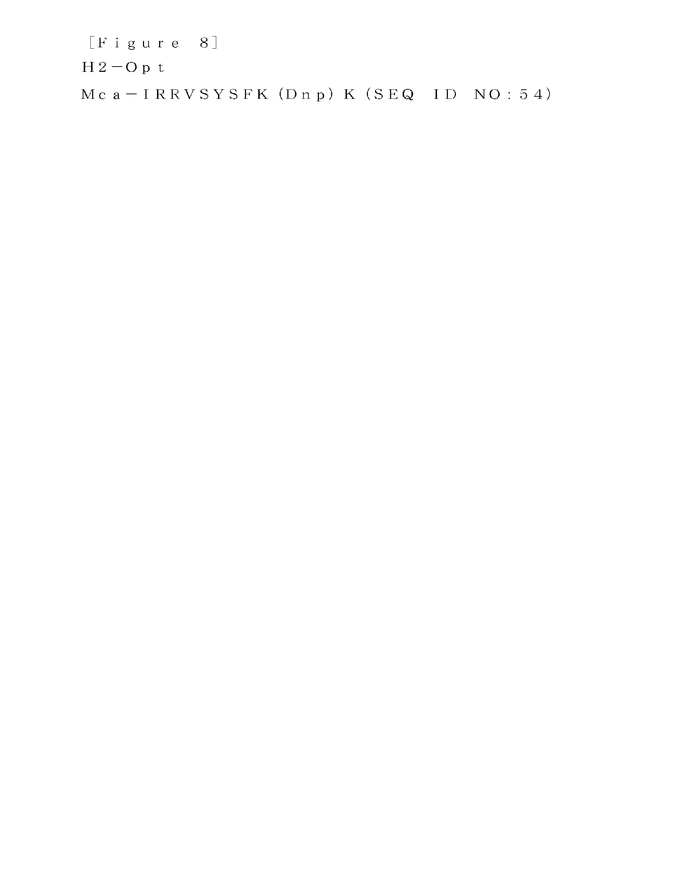

A substrate peptide H2-Opt (Mca-IRRVSYSFK(Dnp)K) (Peptide Institute, Inc.: SEQ ID NO: 54, Figure 8) was dissolved at 10 mM in DMSO, diluted with an assay buffer (50 mM borate and 150 mM NaCl, pH 8.5), and used at a final concentration of 10 µM. HTRA1 (HTRA1 (cat) or HTRA1 (full)) and each HTRA1-inhibiting peptide diluted with an assay buffer were mixed at 25 µL each and reacted at 37°C for 20 minutes. Then, 50 µL of the substrate diluted with an assay buffer was added thereto. A fluorescent signal (excitation at 328 nm/emission at 393 nm) was measured using Enspire (PerkinElmer, Inc.). The final concentration of HTRA1 was 100 nM, and the final concentration of the HTRA1-inhibiting peptide was 1.875 to 1,000 nM. PROTEOSAVE(R) SS96F black plate (Sumitomo Bakelite Co., Ltd.) was used in the reaction and the measurement.

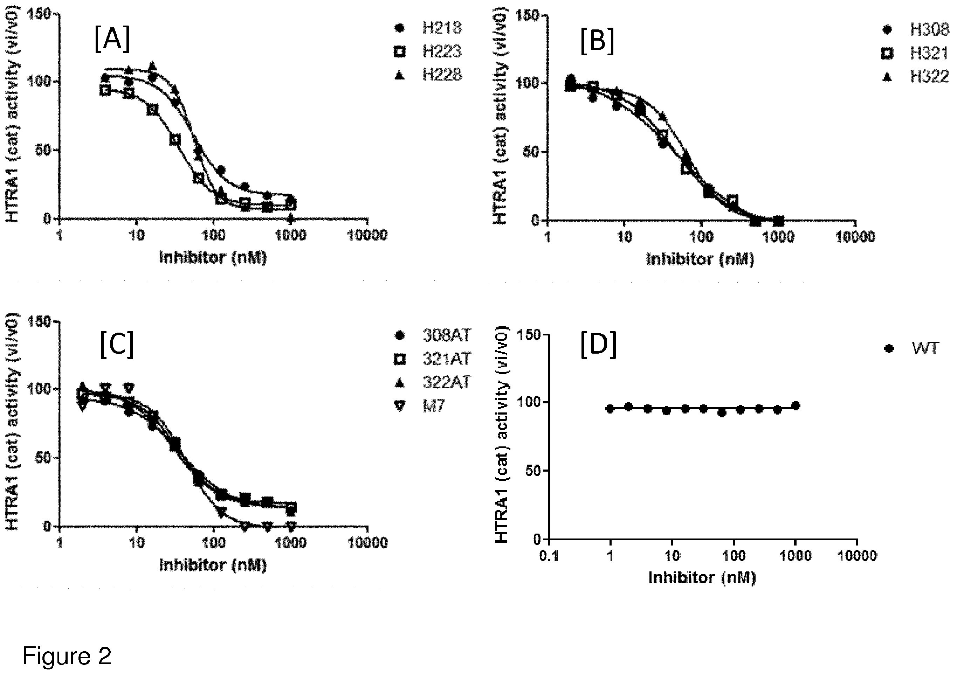

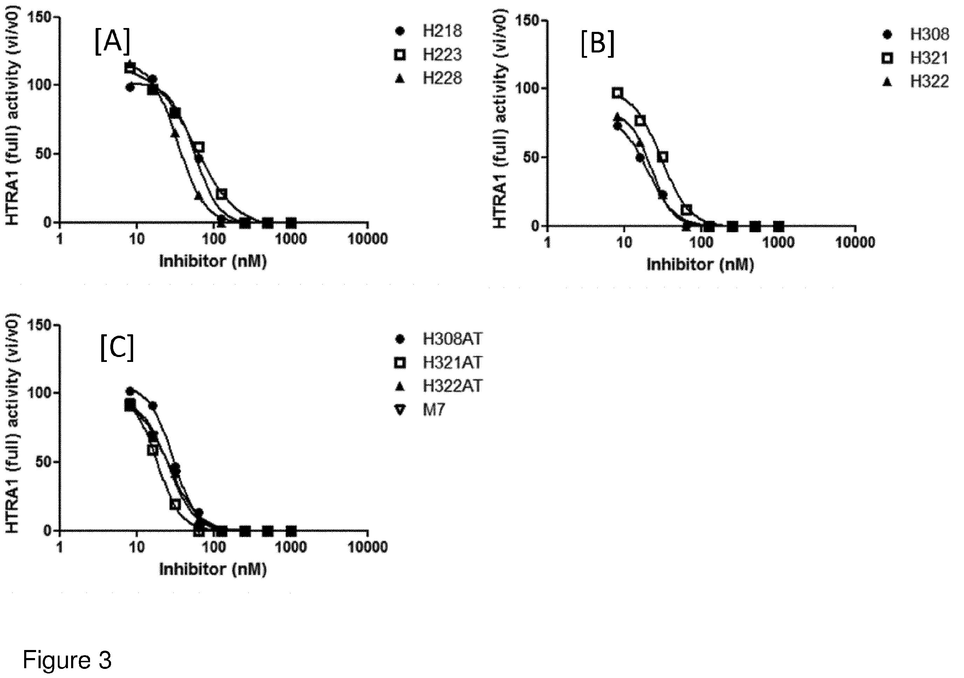

The substrate peptide decomposition rate of the HTRA1-inhibiting peptide at each concentration was calculated. When the decomposition rate at an inhibitor concentration of 0 nM was defined as 100%, the HTRA1 (cat) and HTRA1 (full) inhibitory activity of each HTRA1-inhibiting peptide was evaluated (Figures 2 and 3). As a result of calculating a 50% inhibitory concentration (IC50) using GraphPad Prism (version 5.0; GraphPad Software Inc.), all the HTRA1-inhibiting peptides were found to inhibit HTRA1 (cat) and HTRA1 (full) enzyme activity at a low concentration (Figures 2A to 2C and Figure 3). By control, wild-type SPINK2 (wt) exhibited no HTRA1 inhibitory activity (Figure 2D).

### HTRA1 inhibitory activity of HTRA1-inhibiting peptide

**[Table 1]**

| **ID** | **IC50 (nM) for HTRA1 (cat)** | **IC50 (nM) for HTRA1 (full)** |
|---|---|---|
| H218 | 72 | 55 |
| H223 | 41 | 66 |
| H228 | 71 | 38 |
| H308 | 37 | 15 |
| H321 | 48 | 29 |
| H322 | 50 | 17 |
| H308AT | 49 | 31 |
| H321AT | 46 | 18 |
| H322AT | 45 | 25 |
| M7 | 43 | 24 |

### (3-2) Evaluation of HTRA1-inhibiting peptide for HTRA1 inhibitory activity using protein substrate

The HTRA1 inhibitory activity of a HTRA1-inhibiting peptide was evaluated with human vitronectin as a protein substrate. HTRA1 (cat) and each HTRA1-inhibiting peptide diluted with an assay buffer (50 mM Tris and 150 mM NaCl, pH 8.0) were mixed and reacted at 37°C for 1 hour. Next, human vitronectin (BD Biosciences; 354238) diluted with an assay buffer was added thereto and reacted at 37°C for 2 hours. A SDS sample buffer was added thereto, and the enzyme reaction was terminated by treatment at 99°C for 5 minutes. Then, the decomposition of the human vitronectin was evaluated by SDS-PAGE and Western blot analysis. The final concentration of the HTRA1-inhibiting peptide was 0 to 25 µM, the final concentration of HTRA1 (cat) was 1 µM, and the final concentration of the human vitronectin was 1 µM. For the Western blot analysis, Human Vitronectin Antibody (R&D Systems, Inc.; MAB2349) was used as a primary antibody, and Anti-Mouse IgG, HRP-Linked Whole Ab Sheep (GE Healthcare; NA931) was used as a secondary antibody.

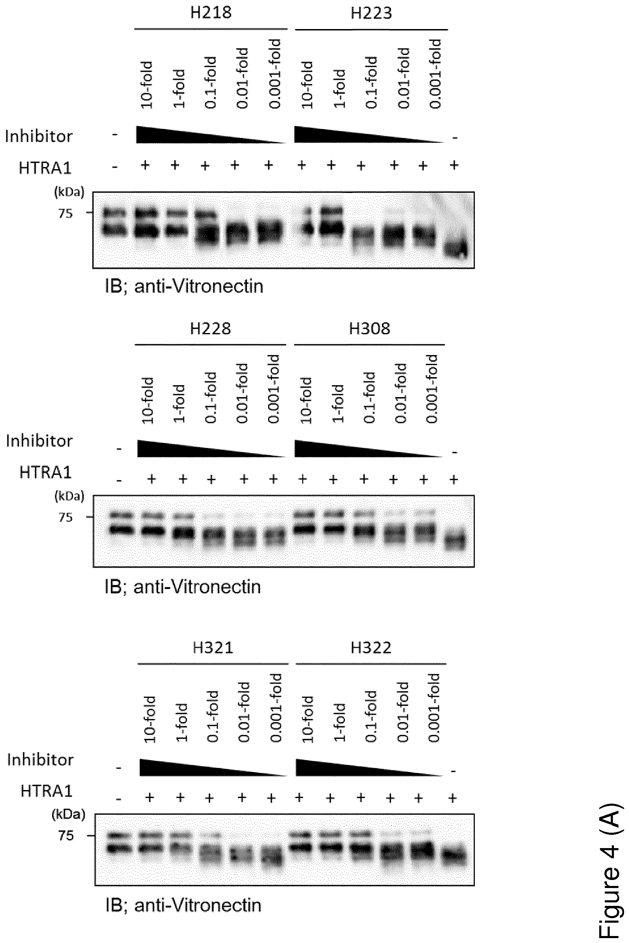

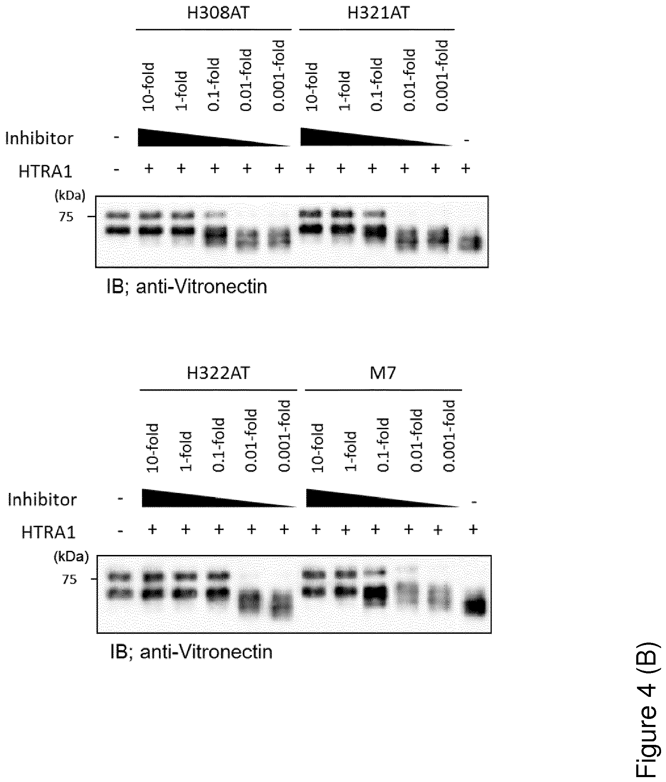

As in (3-1), the HTRA1-inhibiting peptides also strongly exhibited the inhibition of HTRA1 (cat) when human vitronectin was used as a substrate (Figure 4).

### (3-3) Evaluation of HTRA1-inhibiting peptide for specificity

Specificity for other proteases was evaluated by using the cleavage of a substrate peptide as an indicator. In the same way as the method described in (3-1), each protease and each sample (final concentration: 1 µM) diluted with an assay buffer were mixed at 25 µL each and reacted at 37°C for 20 minutes. Then, 50 µL of each substrate diluted with an assay buffer was added thereto. A fluorescent signal (excitation at 380 nm/emission at 460 nm) was measured using Enspire (PerkinElmer, Inc.). The same assay buffer (50 mM borate and 150 mM NaCl, pH 8.5) as in Example 2 was used in the evaluation of HTRA2 activity. An assay buffer (50 mM Tris and 150 mM NaCl, pH 8.0) was used in the evaluation of protease activity other than HTRA2 activity. PROTEOSAVE(R) SS96F black plate (Sumitomo Bakelite Co., Ltd.) was used in the reaction and the measurement. The combinations of the protease and the substrate used in the specificity evaluation were as follows.
Bovine trypsin inhibitory activity evaluation; 5 nM (final concentration) trypsin (Pierce; 20233) and 100 µM (final concentration) substrate peptide Boc-VPR-AMC Fluorogenic Peptide Substrate (R&D Systems, Inc.; ES011) Bovine α-chymotrypsin inhibitory activity evaluation; 10 nM (final concentration) chymotrypsin (Worthington Biochemical Corporation; LS001434) and 100 µM (final concentration) substrate peptide Suc-Leu-Leu-Val-Tyr-MCA (Peptide Institute, Inc.; 3120-v)
Human tryptase inhibitory activity evaluation; 1 nM (final concentration) tryptase (Sigma-Aldrich Co. LLC; T7063) and 100 µM (final concentration) substrate peptide Boc-Phe-Ser-Arg-MCA (Peptide Institute, Inc.; 3107-v) Human chymase inhibitory activity evaluation; 100 nM (final concentration) chymase (Sigma-Aldrich Co. LLC; C8118) and 100 µM (final concentration) substrate peptide Suc-Leu-Leu-Val-Tyr-MCA (Peptide Institute, Inc.; 3120-v) Human plasmin inhibitory activity evaluation; 50 nM (final concentration) plasmin (Sigma-Aldrich Co. LLC; P1867) and 100 µM (final concentration) substrate peptide Boc-Val-Leu-Lys-MCA (Peptide Institute, Inc.; 3104-v) Human thrombin inhibitory activity evaluation; 1 nM (final concentration) thrombin (Sigma-Aldrich Co. LLC; T6884) and 100 µM (final concentration) substrate peptide Boc-VPR-AMC Fluorogenic Peptide Substrate (R&D Systems, Inc.; ES011)
Human matriptase inhibitory activity evaluation; 1 nM (final concentration) matriptase (R&D Systems, Inc.; E3946-SE) and 100 µM (final concentration) substrate peptide Boc-QAR-AMC Fluorogenic Peptide Substrate (R&D Systems, Inc.; ES014)
Human protein C inhibitory activity evaluation; 100 nM (final concentration) protein C (Sigma-Aldrich Co. LLC; P2200) and 100 µM (final concentration) substrate peptide Boc-Leu-Ser-Thr-Arg-MCA (Peptide Institute, Inc.; 3112-v) Human tPA inhibitory activity evaluation; 10 nM (final concentration) tPA (Sigma-Aldrich Co. LLC; T0831) and 100 µM (final concentration) substrate peptide Pyr-Gly-Arg-MCA (Peptide Institute, Inc.; 3145-v)
Human uPA inhibitory activity evaluation; 10 nM (final concentration) uPA (Sigma-Aldrich Co. LLC; T0831) and 100 µM (final concentration) substrate peptide Pyr-Gly-Arg-MCA (Peptide Institute, Inc.; 3145-v)
Human plasma kallikrein inhibitory activity evaluation; 0.125 µg/ml (final concentration) plasma kallikrein (Sigma-Aldrich Co. LLC; T0831) and 100 µM (final concentration) substrate peptide Z-Phe-Arg-MCA (Peptide Institute, Inc.; 3095-v)
Human HTRA2 inhibitory activity evaluation; 200 nM (final concentration) HTRA2 (R&D Systems, Inc.; 1458-HT) and 50 µM (final concentration) substrate peptide H2-Opt (Peptide Institute, Inc.)

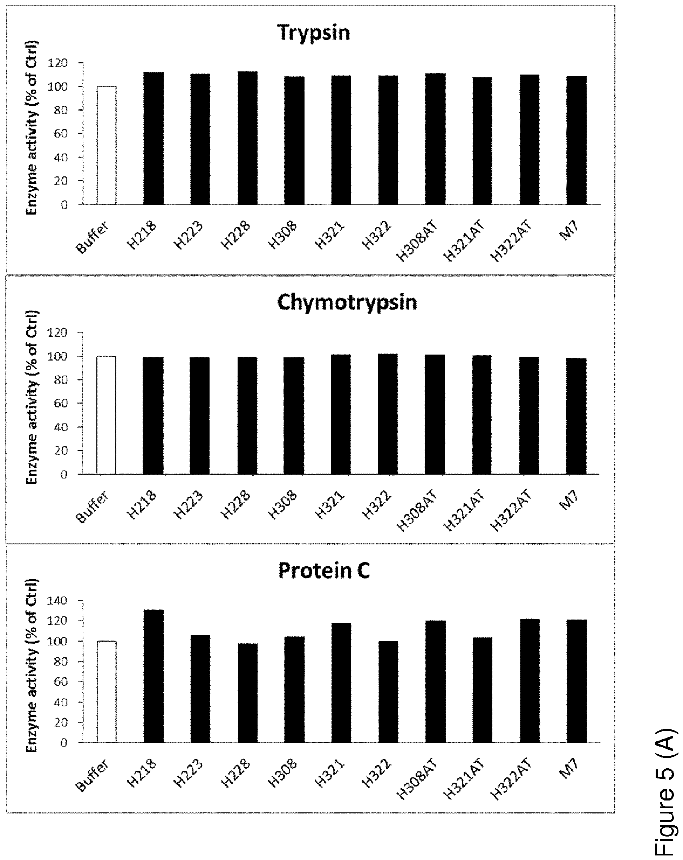

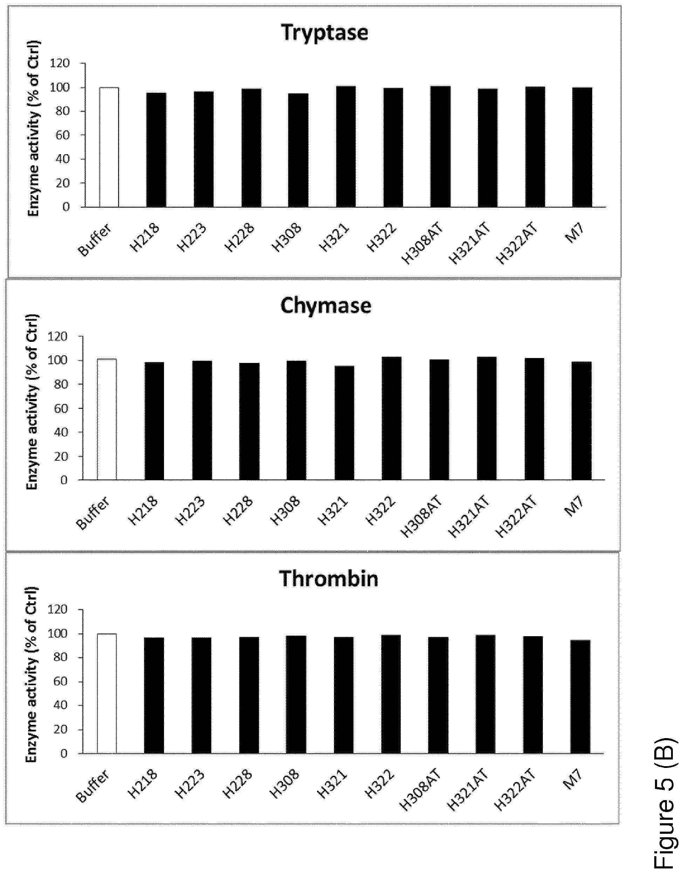

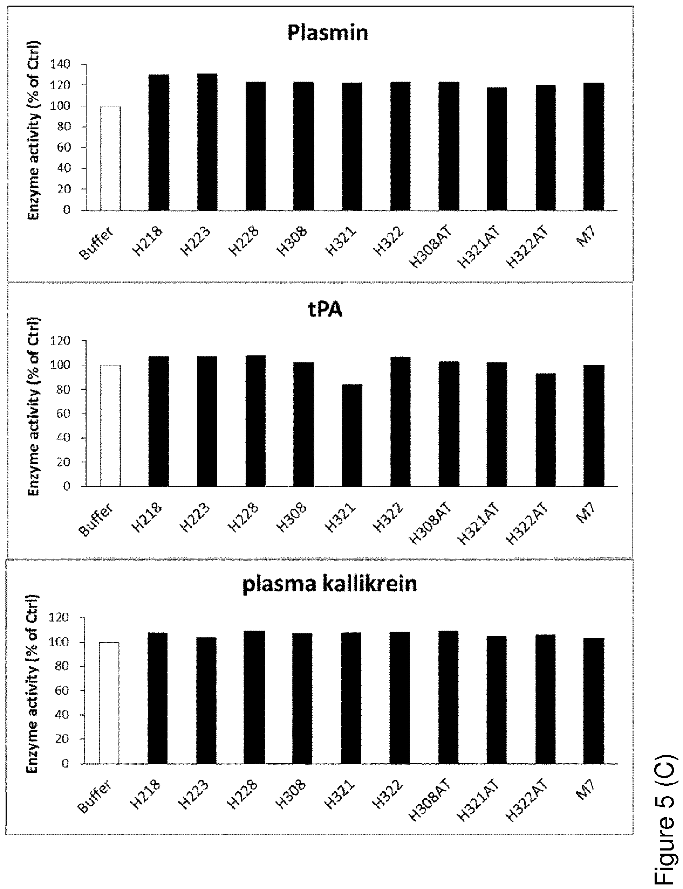

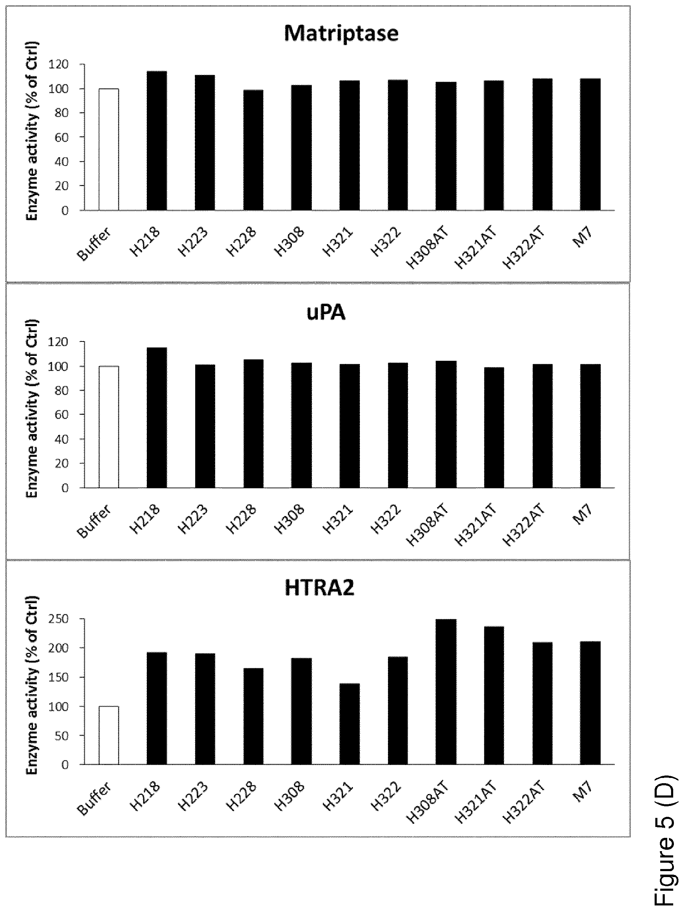

Cross-reactivity with proteases other than HTRA1 was evaluated by using the decomposition of the peptide substrate as an indicator in the same way as in (3-2). Each HTRA1-inhibiting peptide did not suppress the protease activity of any of the proteases at a final inhibitor concentration of 1 micro M, indicating that the HTRA1-inhibiting peptide has a HTRA1-specific inhibitory effect (Figure 5).

### Example 4. Analysis of HTRA1-inhibiting peptide using X-ray crystal structure

### (4-1) Preparation of HTRA1 (cat)/HTRA1-inhibiting peptide complex

HTRA1 (cat) and a HTRA1-inhibiting peptide having the amino acid sequence shown in SEQ ID NO: 3 were each prepared according to the methods described in (1-2) and (2-1). These were mixed under conditions of 20 mM Tris-HCl and 150 mM NaCl, pH 7.6. Then, a complex was isolated and purified by gel filtration chromatography (Superdex 200 10/300 GL).

### (4-2) X-ray crystallography

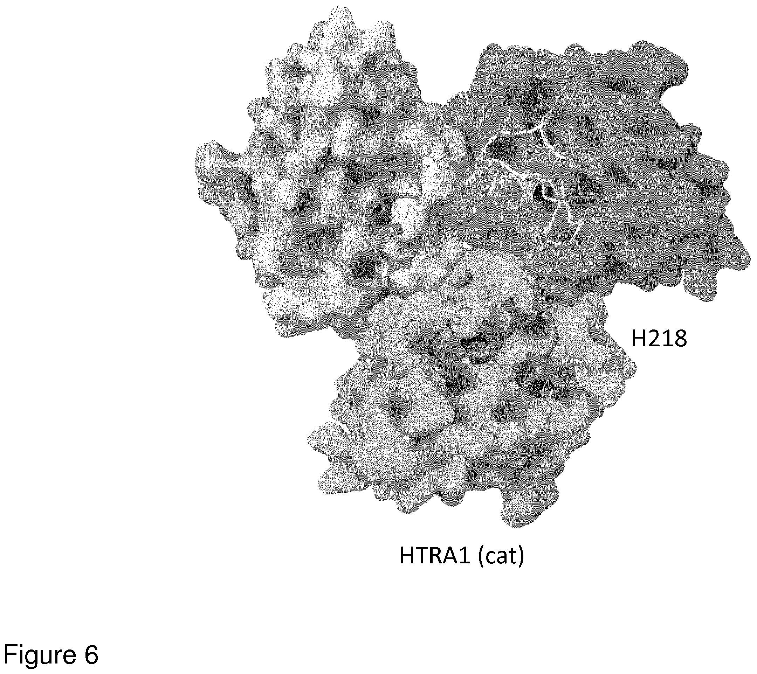

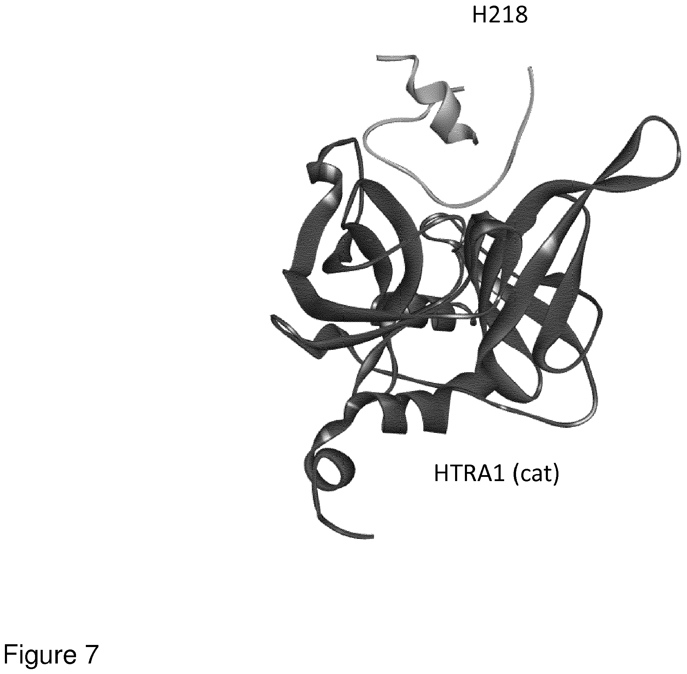

The complex solution prepared in (4-1) was concentrated into 18 mg/ml and then mixed with a reservoir solution (1.0 M LiCl, 7.5% PEG6000, and 0.1 M Tris/HCl (pH 8.5)) at a ratio of 1:1, and the mixture was crystallized by the vapor diffusion method. The obtained cubic monocrystals were dipped in a reservoir solution containing 20% ethylene glycol and then frozen in liquid nitrogen. The frozen crystals were exposed to X-rays under cryogenic air flow to obtain a diffraction image (photon factory BL5A: High Energy Accelerator Research Organization). Scaling data with a maximum resolution of 2.6 Angstrom was obtained by analysis using HKL2000. The phase was determined by the molecular replacement method using serine protease HTRA1 (PDB ID: 3NZI) as a template. After structure refinement, a crystalline complex of HTRA1 (cat) and the peptide was determined at a resolution of 2.6 Angstrom. One each of HTRA1 and SPINK2 molecules was contained in the unit cell. As for the SPINK2 molecule, a partial molecular model containing an interaction site with HTRA1 (cat) was constructed on the basis of the sequence information and the observed electron density. The HTRA1-inhibiting peptide was confirmed to bind to a region containing the active center of the HTRA1 enzyme (Figures 6 and 7).

Example 5. Retinal protective effect brought about by inhibition of HTRA1 in rat model of retinal damage induced by light exposure

### (5-1) Preparation of rat model of retinal damage induced by light exposure

Rat models of retinal damage induced by light exposure are models that induce the cell death of retinal photoreceptor cells by light exposure and are universally used as model animals of retinal degeneration (Daniel T. Organisciak et al., (1996) Invest Ophthalmol Vis Sci. Vol. 37 (No. 11): p. 2243-2257). A 0.5% (W/V) tropicamide-0.5% phenylephrine hydrochloride ophthalmic solution was ocularly instilled under adaptation to darkness to rats adapted to darkness for 72 hours. Then, the rats were exposed to white light of 5500 Lux for 3 hours. The rats thus exposed were adapted again to darkness for about 24 hours and then raised for 2 days under light-dark conditions of ordinary raising. After euthanasia, the eyeballs were excised and fixed by dipping in a 3.7% (W/V) formaldehyde-0.5 to 1% (W/V) methanol-0.2% (W/V) picric acid fixative for 24 hours or longer. After paraffin embedding, thin sliced sections were prepared. The sections were stained with hematoxylin-eosin, and a nucleus count in an outer nuclear layer of a cross-section of the retina was determined to evaluate retinal damage. The rat models of retinal damage induced by light exposure were found to have a marked decrease in nucleus count in an outer nuclear layer due to light exposure.

### (5-2) Confirmation of expression of extracellular HTRA1 at time of retinal damage

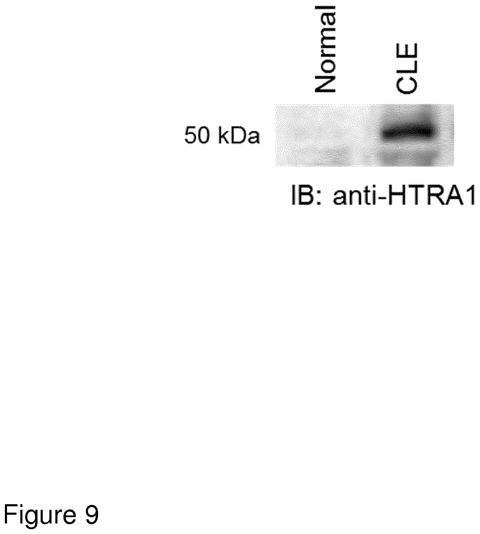

In order to examine the involvement of HTRA1 in rat models of retinal damage induced by light exposure, vitreous humor was collected from the model rats prepared in (5-1) and evaluated for a HTRA1 expression level by Western blot analysis. The vitreous humor was subjected to SDS-PAGE under reductive conditions. Rat HTRA1 was detected using as a primary antibody Human HTRA1/PRSS11 Antibody (R&D Systems, Inc.; AF2916) and as a secondary antibody Sheep IgG Horseradish Peroxidase-conjugated Antibody (R&D Systems, Inc.; HAF016). The increased amount of HTRA1 in the vitreous humor was confirmed in the light exposure group as compared with a non-exposure group, suggesting that in this model, HTRA1 is involved in the process of retinal damage caused by light exposure (Figure 9).

### (5-3) Retinal protective effect of HTRA1-inhibiting peptide in rat model of retinal damage induced by light exposure

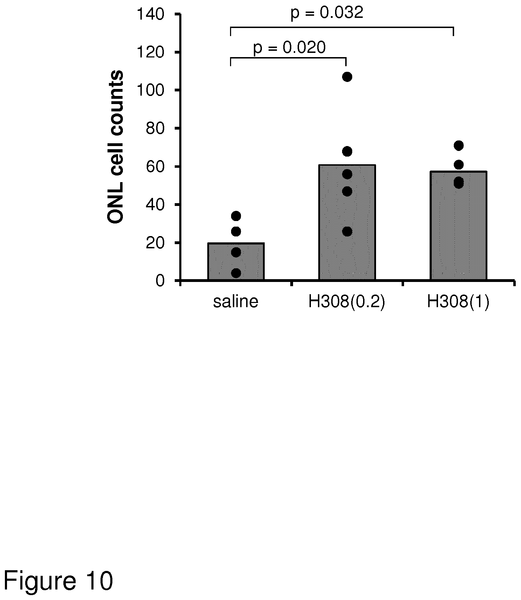

Immediately before light exposure of rats, 5 µL of HTRA1-inhibiting peptide H308 having a concentration of 0.04 mg/mL or 0.2 mg/mL was intravitreally administered under anesthesia. n = 4 for a normal saline administration group, and n = 5 for the other groups. Light exposure decreased the nucleus count in an outer nuclear layer on a cross-section of the retina in the normal saline administration group, whereas the effect of suppressing the decrease in nucleus count in an outer nuclear layer was confirmed in the HTRA1-inhibiting peptide administration group (Figure 10). These results demonstrated that the HTRA1-inhibiting peptide exhibits medicinal effects on tissue damage caused by HTRA1.

### Example 6. Evaluation of HTRA1-inhibiting peptide derivative

### (6-1) Construction of pET 32a_HTRA1-inhibiting peptide H308_S16A_Kex2

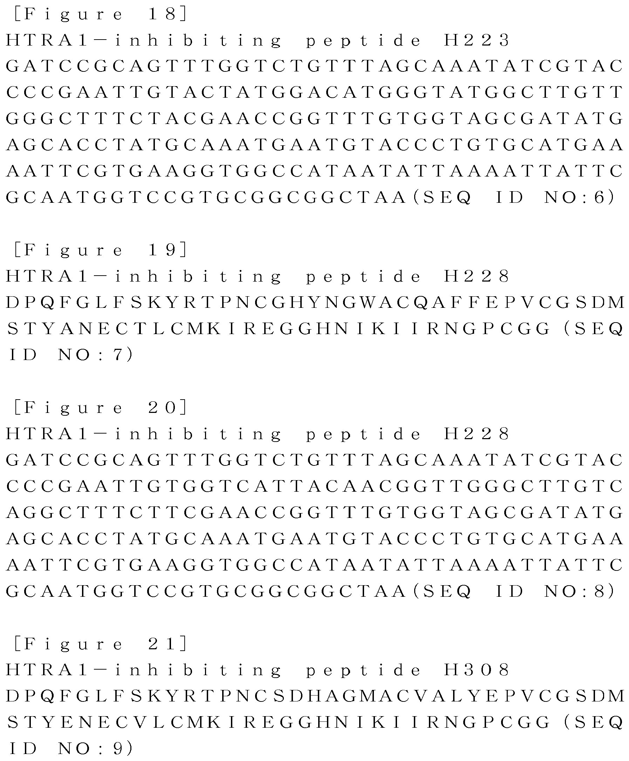

Derivative S16A having an amino acid sequence in which 16Ser in the amino acid sequence shown in SEQ ID NO: 9 (Figure 21) was substituted with Ala was prepared with HTRA1-inhibiting peptide H308 as a template. Fragment C was amplified by PCR ((94°C for 15 sec, 60°C for 30 sec, and 68°C for 15 sec) × 30 cycles) using the following primers and KOD-plus- (Toyobo Co., Ltd.).
Primer 12: 5'-CCGCAGTTTGGTCTGTTTAGCAAATATCGTACCCCGAATTGT-3'
Primer 13: 5'-GCCATACCAGCATGGTCCGCACAATTCGGGGTACGATATTTGC-3'

Next, fragment D was amplified by PCR ((94°C for 15 sec, 60°C for 30 sec, and 68°C for 20 sec) × 30 cycles) with HTRA1-inhibiting peptide H308 as a template using the following primers and KOD-plus- (Toyobo Co., Ltd.). Primer 14: 5'-GCGGACCATGCTGGTATGGCATGTGTTGCTCTGTATGAAC-3' Primer 15: 5'-AAAACTCGAGTTAGCCGCCGCACGGACCATTGCGAATAA-3'

The desired DNA fragment was amplified by PCR ((94°C for 15 sec, 60°C for 30 sec, and 68°C for 20 sec) × 30 cycles) using fragments C and D, the following primers, and KOD-plus- (Toyobo Co., Ltd.).
Primer 16: 5'-AAAAGGATCCCTGGACAAACGTGATCCGCAGTTTGGTCTGTTTAG-3'
Primer 15

The amplified fragment was subjected to agarose gel electrophoresis. Then, the desired DNA fragment was excised from the gel, and DNA was prepared using a QIAquick Gel Extraction Kit (Qiagen N.V.). The prepared DNA fragment and pET 32a (Novagen) were each treated with restriction enzymes BamHI (New England BioLabs Inc.) and XhoI (New England BioLabs Inc.) at 37°C for 1 hour or longer. After agarose gel electrophoresis, the desired DNA fragments were excised from the gel and purified using a QIAquick PCR Purification Kit (Qiagen N.V.). The purified fragments were reacted overnight at 16°C for a ligation reaction using T4 DNA Ligase (New England BioLabs Inc.). The ligation solution was added to *E*. *coli* JM109 (Toyobo Co., Ltd.), and the mixture was left standing on ice for 30 minutes, then heat-treated at 42°C for 45 seconds, further left standing on ice for 5 minutes, and inoculated onto a 2YT plate containing 0.1 mg/ml ampicillin, followed by static culture overnight at 37°C to transform the *E. coli.* The transformed *E. coli* was cultured, and miniprep and sequence analysis were then carried out to construct "pET 32a_HTRA1-inhibiting peptide H308_S16A_Kex2". The operation was performed in accordance with the method described in (1-1-1).

### (6-2) Preparation of HTRA1-inhibiting peptide_N-terminal derivative expression vector

In order to prepare four N-terminal sequence derivatives (designated as D1G, D1S, D1E, and D1SLI, respectively) of the HTRA1-inhibiting peptide having an amino acid sequence in which 1Asp in the amino acid sequence shown in SEQ ID NO: 9 (Figure 21) was substituted with Gly, Ser, Glu or Ser-Leu-Ile, expression vectors were constructed by the same approach as in (6-1). Four fragments of interest were each amplified by PCR ((94°C for 15 sec, 60°C for 30 sec, and 68°C for 20 sec) × 30 cycles) using fragments C and D, the following primers, and KOD-plus- (Toyobo Co., Ltd.).
D1G preparation primers
   Primer 17: 5'-AAAAGGATCCCTGGACAAACGTGGCCCGCAGTTTGGTCTGTTTAG-3'
   Primer 15
D1S preparation primers
   Primer 18: 5'-AAAAGGATCCCTGGACAAACGTAGCCCGCAGTTTGGTCTGTTTAG-3'
   Primer 15
D1E preparation primers
   Primer 19: 5'-AAAAGGATCCCTGGACAAACGTGAACCGCAGTTTGGTCTGTTTAG-3'
   Primer 15
D1SLI preparation primers
   Primer 20: 5'-AAAAGGATCCCTGGACAAACGTAGCCTGATTCCGCAGTTTGGTCTGTTTAG-3'
   Primer 15

Each of the four amplified fragments was subjected to agarose gel electrophoresis. Then, the desired DNA fragment was excised from the gel, and DNA was prepared using a QIAquick Gel Extraction Kit (Qiagen N.V.). The prepared DNA fragment and pET 32a (Novagen) were each treated with restriction enzymes BamHI (New England BioLabs Inc.) and XhoI (New England BioLabs Inc.) at 37°C for 1 hour or longer. After agarose gel electrophoresis, the desired DNA fragments were excised from the gel and purified using a QIAquick PCR Purification Kit (Qiagen N.V.). The purified fragments were reacted overnight at 16°C for a ligation reaction using T4 DNA Ligase (New England BioLabs Inc.). The ligation solution was added to *E. coli* JM109 (Toyobo Co., Ltd.), and the mixture was left standing on ice for 30 minutes, then heat-treated at 42°C for 45 seconds, further left standing on ice for 5 minutes, and inoculated onto a 2YT plate containing 0.1 mg/ml ampicillin, followed by static culture overnight at 37°C to transform the *E. coli.* The transformed *E. coli* was cultured, and miniprep and sequence analysis were then carried out to construct "pET 32a_HTRA1-inhibiting peptide H308_D1G_S16A_Kex2", "pET 32a_HTRA1-inhibiting peptide H308_D1S_S16A_Kex2", "pET 32a_HTRA1-inhibiting peptide H308_D1E_S16A_Kex2", and "pET 32a_HTRA1-inhibiting peptide H308_D1SLI_S16A_Kex2". The operation was performed in accordance with the method described in (1-1-1).

### (6-3) Preparation of HTRA1-inhibiting peptide derivative

*E. coli* Origami B (DE3) (Novagen) was transformed with each of the five vectors constructed in (6-1) and (6-2), and cultured at 37°C using a 2YT medium containing 0.1 mg/ml ampicillin. Then, IPTG (final concentration: 1 mM) was added thereto, and the *E. coli* was cultured overnight at 16°C. On the next day, after harvest by centrifugation (3,000 g, 20 min, 4°C), a lysate was prepared using BugBuster Master Mix (Novagen), and a His tag fusion protein of interest was purified using TALON Metal Affinity Resin (Clontech Laboratories, Inc.). Next, a thioredoxin tag and the desired protein were cleaved using Kex2 (mentioned above) and purified using TALON. The resultant was subjected to gel filtration chromatography (Superdex 75 10/300 GL) or reverse-phase chromatography (YMC-Pack ODS-AM) to prepare five HTRA1-inhibiting peptide derivatives. The amino acid sequences of the derivatives are shown in SEQ ID NOs: 23 to 27 (Figures 35 to 39).

### (6-4) Evaluation of HTRA1-inhibiting peptide derivative

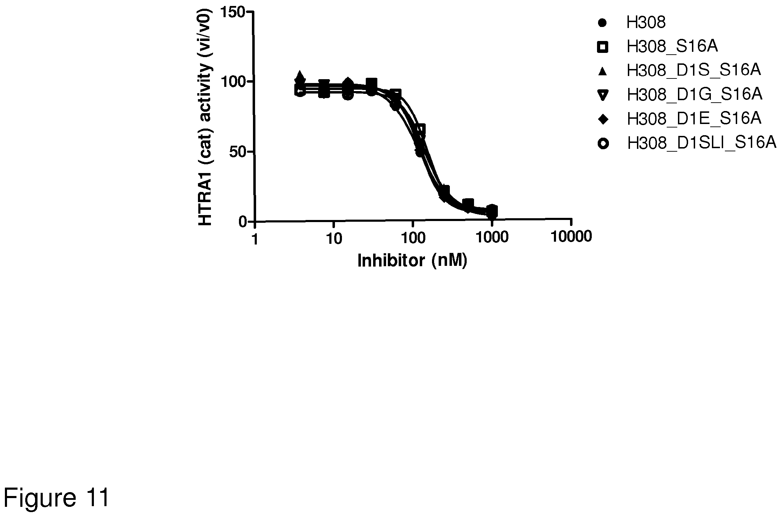

As a result of measuring HTRA1 (cat) inhibitory activity according to the method described in (3-1), all the derivatives had inhibitory activity equivalent to that of H308 (Figure 11).

### Example 7. Evaluation of HTRA1-inhibiting peptide for binding activity against HTRA1 (cat)

Binding activity was evaluated by the immunoprecipitation method using three HTRA1-inhibiting peptides (H308, H321AT, and H322AT) prepared in Example (1-2) and HTRA1 (cat) prepared in (2-1). 2.5 µg of each HTRA1-inhibiting peptide and 10 µg of HTRA1 (cat) were reacted at room temperature for 30 minutes. Then, 10 µL of TALON Metal Affinity Resin (Clontech Laboratories, Inc.) was added thereto. After further reaction for 30 minutes, the resin was recovered as an immunoprecipitation (IP) fraction and subjected to SDS-PAGE to evaluate binding activity. PBS was used as a buffer in the reaction.

When each of the three HTRA1-inhibiting peptides or HTRA1 (cat) was reacted with TALON, the band of only His tag-fused HTRA1 (cat) was detected in an input lane. On the other hand, the band of each inhibiting peptide and the enzyme was detected only in an IP lane where the inhibiting peptide was reacted with HTRA1 (cat). Accordingly, each of the three HTRA1-inhibiting peptides was confirmed to bind to HTRA1 (cat).

### Example 8. Evaluation of HTRA1-inhibiting peptide for HTRA1 inhibitory activity

### (8-1) Evaluation of HTRA1-inhibiting peptide for HTRA1 inhibitory activity using peptide substrate

Three HTRA1-inhibiting peptides (H308_D1G_S16A, H321AT_D1G_S16A, and H322AT_D1G_S16A) constructed in Example 6 were evaluated for their HTRA1 (cat) or HTRA1 (full) inhibitory activity using a substrate peptide H2-Opt (n = 3). A substrate peptide H2-Opt (Mca-IRRVSYSFK(Dnp)K) (Peptide Institute, Inc.: SEQ ID NO: 54, Figure 8) was dissolved at 10 mM in DMSO, diluted with an assay buffer (50 mM Tris, 150 mM NaCl, and 0.25% CHAPS, pH 8.0), and used at a final concentration of 10 µM. HTRA1 (HTRA1 (cat) or HTRA1 (full); Example 2) and each HTRA1-inhibiting peptide diluted with an assay buffer were mixed at 25 µL each and reacted at 37°C for 20 minutes. Then, 50 µL of the substrate diluted with an assay buffer was added thereto. A fluorescent signal (excitation at 328 nm/emission at 393 nm) was measured using Enspire (PerkinElmer, Inc.). The final concentration of HTRA1 was 100 nM, and the final concentration of the HTRA1-inhibiting peptide was 1.875 to 1,000 nM. PROTEOSAVE(R) SS96F black plate (Sumitomo Bakelite Co., Ltd.) was used in the reaction and the measurement.

The substrate peptide decomposition rate of the HTRA1-inhibiting peptide at each concentration was calculated. When the decomposition rate at an inhibitor concentration of 0 nM was defined as 100%, the HTRA1 (cat) and HTRA1 (full) inhibitory activity of each HTRA1-inhibiting peptide was evaluated.

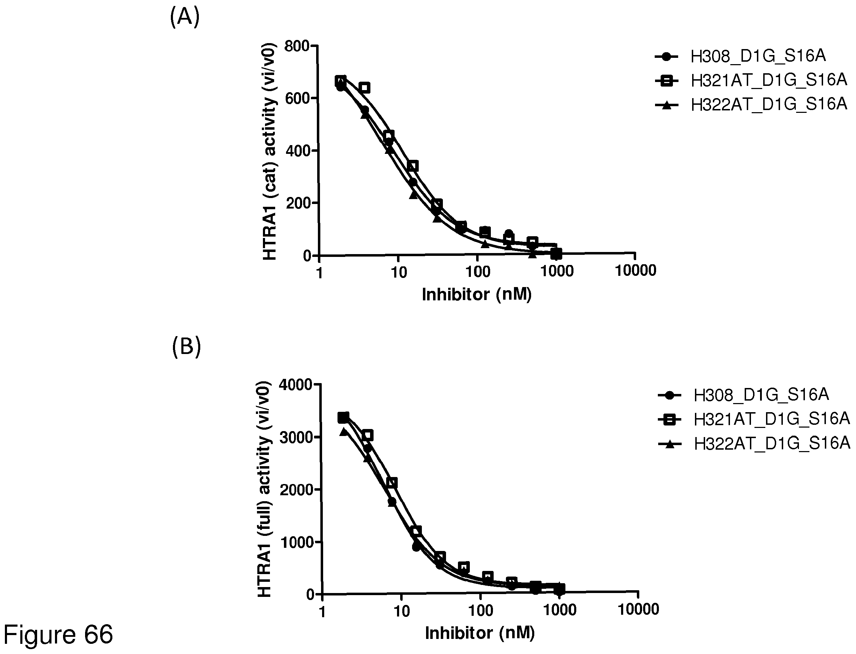

As a result of calculating a 50% inhibitory concentration (IC50) using GraphPad Prism (version 5.0; GraphPad Software Inc.), all the HTRA1-inhibiting peptides were found to inhibit HTRA1 (cat) and HTRA1 (full) enzyme activity at a low concentration (Figure 66).

### HTRA1 inhibitory activity of HTRA1-inhibiting peptide

**[Table 2]**

| | IC50 (nM) for HTRA1 (cat) | IC50 (nM) for HTRA1 (full) |
|---|---|---|
| H308_D1G_S16A | 7.9+1.3 | 9.1+1.4 |
| H321AT_D1G_S16A | 9.0+0.6 | 12.0±1.9 |
| H322AT_D1G_S16A | 12.9+0.2 | 12.2+2.2 |

### (8-2) Evaluation of HTRA1-inhibiting peptide for HTRA1 inhibitory activity using protein substrate

The HTRA1 inhibitory activity of a HTRA1-inhibiting peptide was evaluated with human vitronectin as a protein substrate. The operation followed Example (3-2).

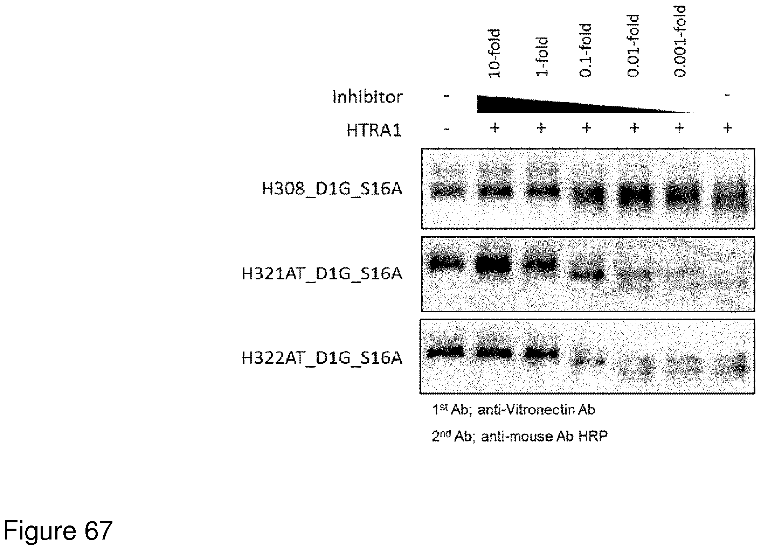

As in (8-1), the HTRA1-inhibiting peptides also strongly exhibited the inhibition of HTRA1 (cat) when human vitronectin was used as a substrate (Figure 67).

### (8-3) Evaluation of HTRA1-inhibiting peptide for specificity

Specificity for other proteases was evaluated by using the cleavage of a substrate peptide as an indicator. The operation for bovine trypsin, bovine α-chymotrypsin, protein C, tryptase, chymase, thrombin, plasmin, tPA, plasma kallikrein, matriptase, uPA, and HTRA2 followed the method described in Example (3-3) (n = 3). Procedures of measuring inhibitory activity against other proteases and combinations of the protease and the substrate were as follows.

PROTEOSAVE(R) SS96F black plate (Sumitomo Bakelite Co., Ltd.) was used in reaction and measurement. Each protease and each sample (final concentration: 1 µM) diluted with an assay buffer were mixed at 25 µL each and reacted at 37°C for 20 minutes. Then, 50 µL of each substrate diluted with an assay buffer was added thereto. A fluorescent signal was measured using Enspire (PerkinElmer, Inc.).

Human trypsin inhibitory activity evaluation; 1 nM (final concentration) trypsin (Sigma-Aldrich Co. LLC; T6424) and 100 µM (final concentration) substrate peptide Boc-VPR-AMC Fluorogenic Peptide Substrate (R&D Systems, Inc.; ES011), fluorescent signal: excitation at 380 nm/emission at 460 nm.

Human chymotrypsin inhibitory activity evaluation; 10 nM (final concentration) chymotrypsin (Sigma-Aldrich Co. LLC; C8946) and 10 µM (final concentration) substrate peptide Suc-Leu-Leu-Val-Tyr-MCA (Peptide Institute, Inc.; 3120-v), fluorescent signal: excitation at 380 nm/emission at 460 nm.

Human factor XIIa inhibitory activity evaluation; 100 nM (final concentration) Factor Alpha-XIIa (Enzyme Research Laboratories Inc.) and 100 µM (final concentration) substrate peptide Pyr-Gly-Arg-MCA (Peptide Institute, Inc.; 3145-v), fluorescent signal: excitation at 380 nm/emission at 460 nm.

Human MMP-2 inhibitory activity evaluation; 1 nM (final concentration) tryptase (Calbiochem; PF023) and 100 µM (final concentration) substrate peptide MOCAx-KPLGL-A2pr(Dnp)-AR (Peptide Institute, Inc.; 3226-v), fluorescent signal: excitation at 328 nm/emission at 393 nm.

Human TPP1 inhibitory activity evaluation; 0.5 µg/mL (final concentration) TPP1 (Calbiochem; 2237-SE) and 200 µM (final concentration) substrate peptide AAF-MCA (Peptide Institute, Inc.; 3201-v), fluorescent signal: excitation at 380 nm/emission at 460 nm.

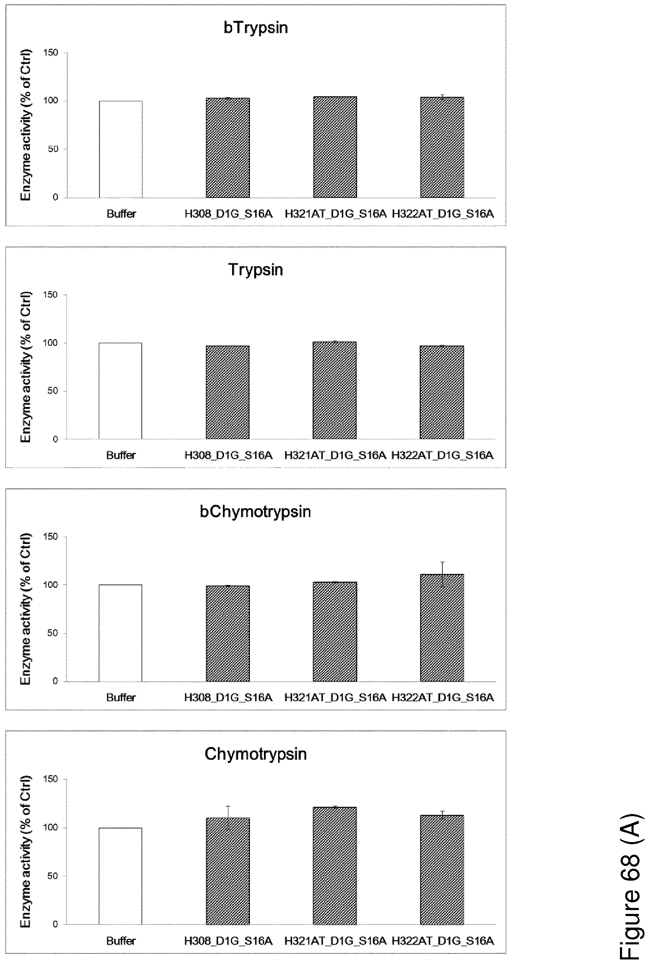

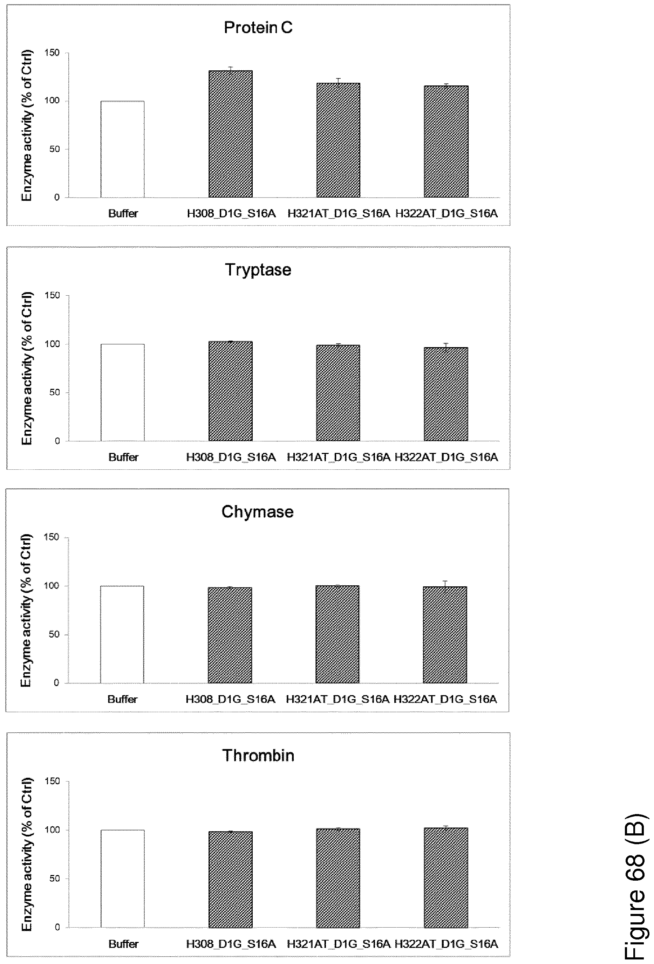

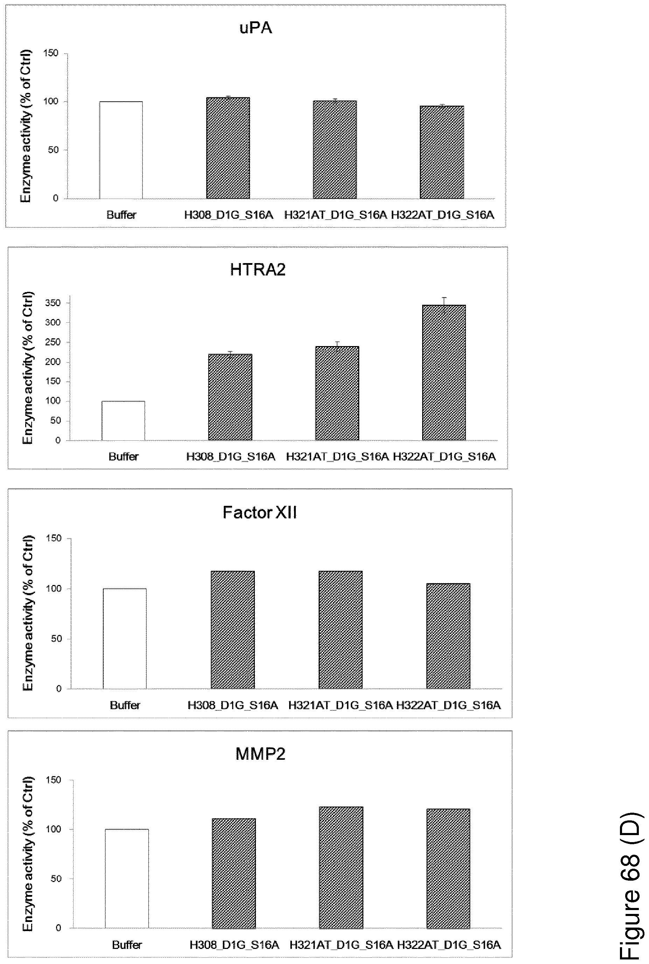

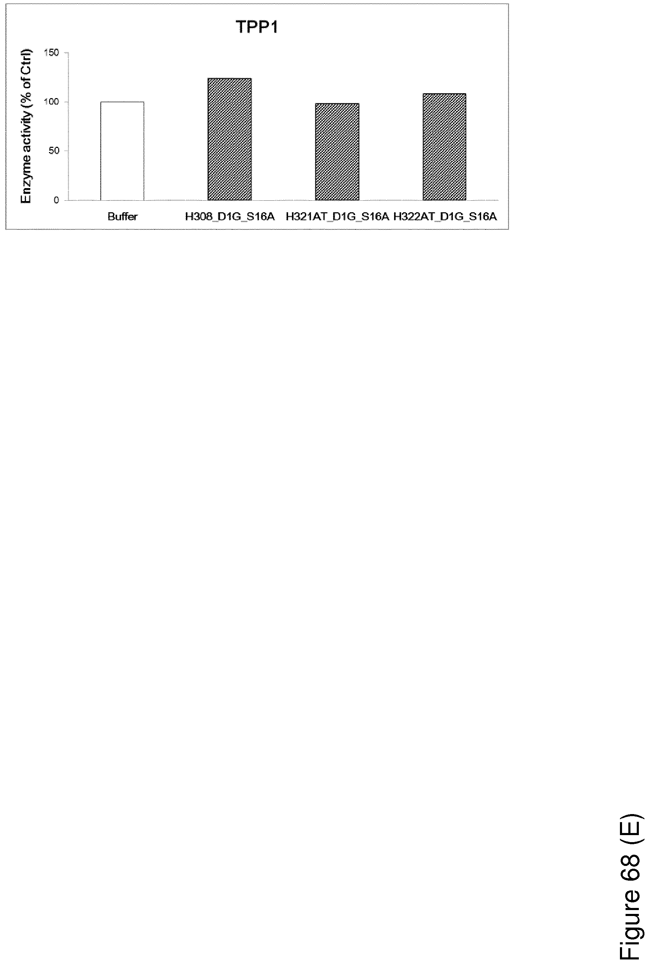

Cross-reactivity with proteases other than HTRA1 was evaluated by using the decomposition of the peptide substrate as an indicator. Each HTRA1-inhibiting peptide did not suppress the protease activity of any of the proteases at a final concentration of 1 µM, indicating that the HTRA1-inhibiting peptide has a HTRA1-specific inhibitory effect (Figure 68).

### Example 9. Evaluation of HTRA1-inhibiting peptide for binding activity against HTRA1 (cat)

Binding activity was evaluated by the immunoprecipitation method according to the operation of Example 7 using three HTRA1-inhibiting peptides prepared in Example 6 and HTRA1 (cat) prepared in (2-1).

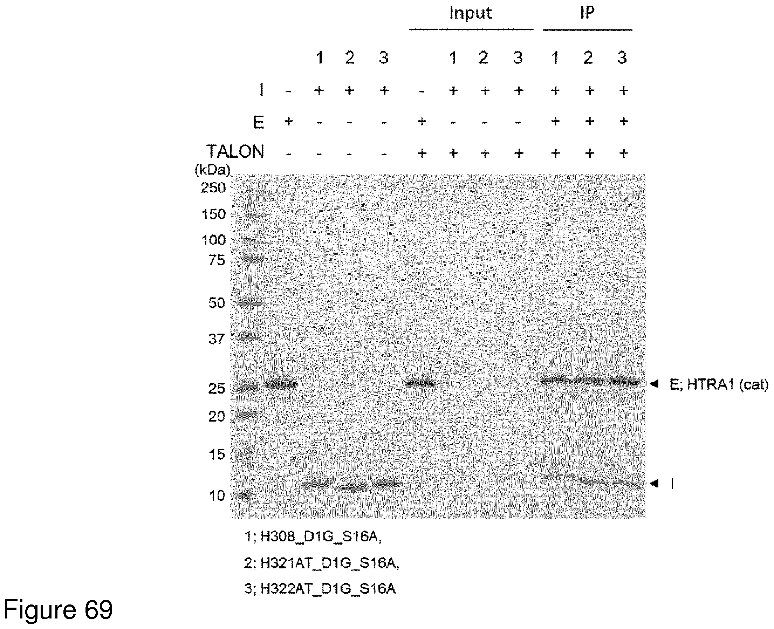

When each of the three HTRA1-inhibiting peptides or HTRA1 (cat) was reacted with TALON, the band of only His tag-fused HTRA1 (cat) was detected in an input lane. On the other hand, the band of each inhibiting peptide and the enzyme was detected only in an IP lane where the inhibiting peptide was reacted with HTRA1 (cat). Accordingly, each of the three HTRA1-inhibiting peptides was confirmed to bind to HTRA1 (cat) (Figure 69).

Example 10. Retinal protective effect brought about by inhibition of HTRA1 in rat model of retinal damage induced by light exposure (part 2)

The retinal protective effects of three HTRA1-inhibiting peptides prepared in Example 6 were evaluated using the rat models of retinal damage induced by light exposure, constructed in Example (5-1). The operation followed Example 5. n = 6 for all groups.

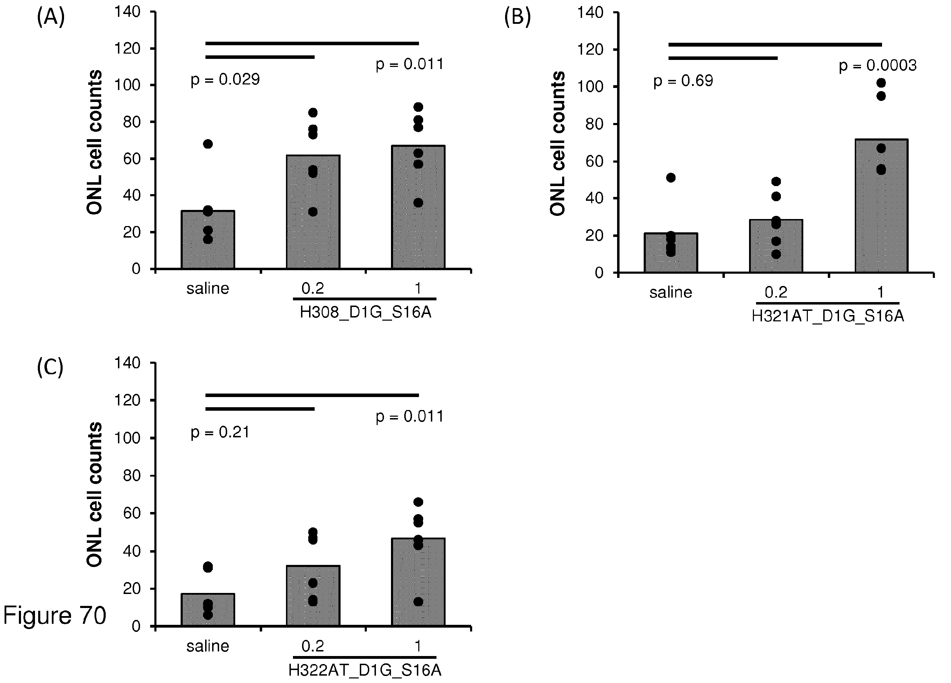

Results of pathologically evaluating the retina are shown in Figure 70. The three HTRA1-inhibiting peptides exhibited a marked suppressive effect on the decrease in nucleus count in an outer nuclear layer caused by light exposure.

Example 11. Protective effect on retinal pigment epithelial cells by inhibition of HTRA1 in rabbit model of retinal damage caused by loading with high-fat diet containing hydroquinone

### (11-1) Preparation of rabbit retinal damage model by loading with high-fat diet containing hydroquinone

Retinal damage models prepared using high-fat diet (HFD) and hydroquinone (HQ) are models in which oxidative stress is induced by a pro-oxidant to cause retinal damage. These models have been reported only for mice (Diego G. Espinosa-Heidmann et al., (2006) Invest Ophthalmol Vis Sci., Vol. 47 (No. 2): p. 729-737). Accordingly, 3-year-old JW rabbits were fed for 4 months with RC4 (Oriental Yeast Co., Ltd.) diet containing 1.5% (W/V) coconut oil-0.25% (W/V) cholesterol-1.5% (W/V) peanut oil-2.4% (W/V) hydroquinone (HFD-HQ) to construct rabbit retinal damage models. After euthanasia, the eyeballs were excised, and the anterior segment of the eye was removed by an external incision of about 5 mm from the corneal limbus. The vitreous body was further separated. Then, retina-choroid-sclera was fixed by dipping in 4% (W/V) paraformaldehyde fixative for 24 hours or longer. After the fixation, the choroid was separated and immunostained using as a primary antibody ZO-1 Monoclonal Antibody (ZO1-1A12) (Thermo Fisher Scientific Inc.; 33-9100) and as a secondary antibody Chicken anti-Mouse IgG (H+L) Cross-Adsorbed Secondary Antibody, Alexa Fluor 594 (Thermo Fisher Scientific Inc.; A-21201). The stained choroid was observed under a fluorescence microscope (BZ-9000; Keyence Corp.). The area of stained retinal pigment epithelial (RPE) cells was determined to evaluate RPE cell damage.

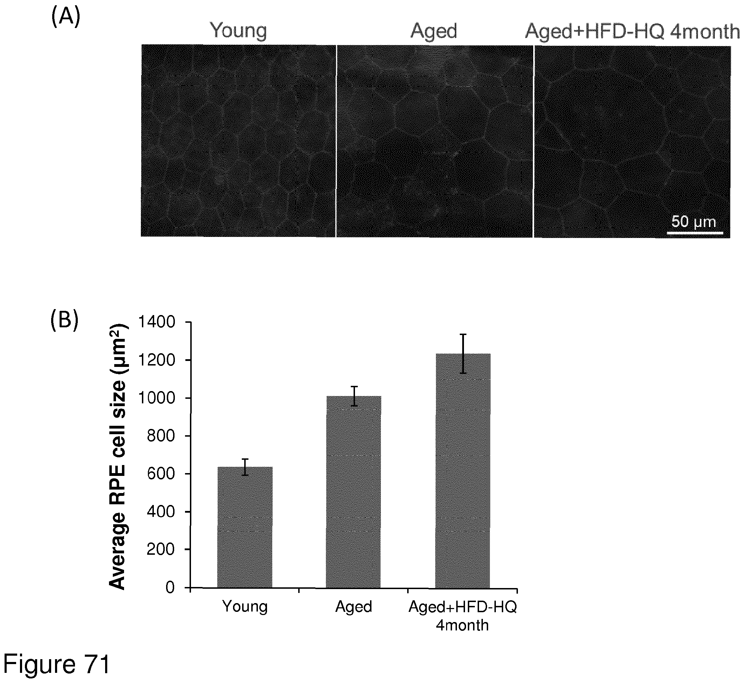

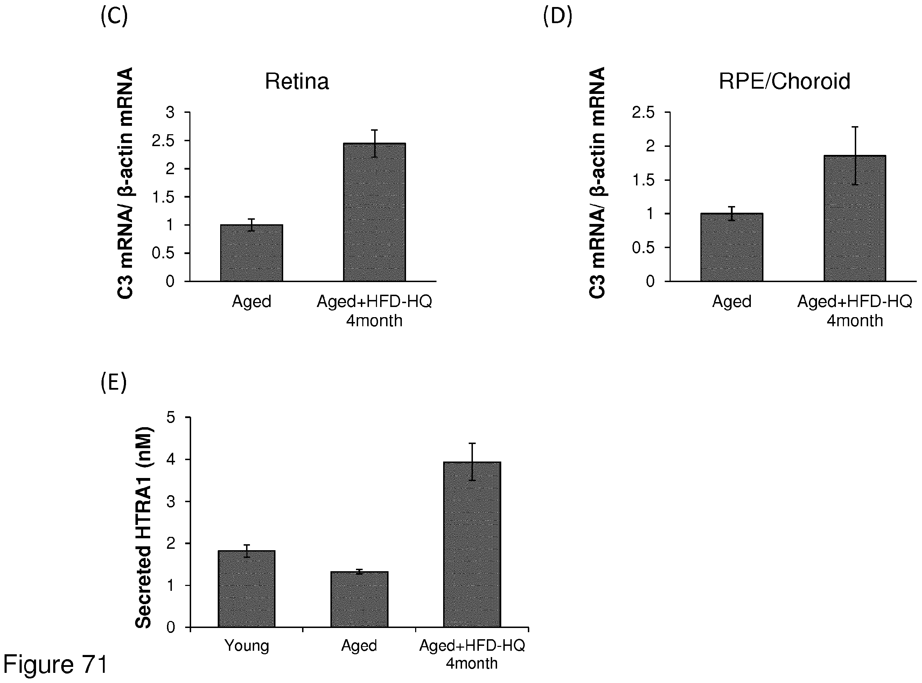

Figure 71 shows stain images of the RPE cells of a 12-week-old rabbit, a 3-year-old rabbit and a HFD-HQ-loaded 3-year-old rabbit (Figure 71(A)) and a graph of the average areas of the RPE cells (Figure 71(B)). The RPE cells were confirmed to be more hypertrophied in the 3-year-old rabbit than in the 12-week-old rabbit and to be further hypertrophied by HFD-HQ loading. Damage was confirmed to appear in the RPE cells. Similar change has been observed in the eyeballs of age-related macular degeneration patients (Ding JD et al., (2011) Proc Natl Acad Sci U S A., Vol. 108 (No. 28): p. 279-87).

### (11-2) Increase in AMD-related factor C3 expression level at time of retinal damage

In order to evaluate the expression of an AMD-related factor, tissues were respectively collected from the retina and the RPE/choroid of the rabbit retinal damage models. mRNA was extracted using an RNeasy mini kit (Qiagen N.V.) and then subjected to a reverse transcription reaction using a TaqMan Gene Expression Master Mix (Thermo Fisher Scientific Inc.). The mRNA levels of complement component 3 (C3) and an internal standard β-actin were quantitatively analyzed by a TaqMan Gene Expression Assay (Oc03397832_g1 and Oc03824857_g1; Thermo Fisher Scientific Inc.) using 7900HT Fast Real-Time PCR System (Applied Biosystems, Inc.). The analysis was carried out at n = 4 for 3-year-old rabbits and n = 10 for HFD-HQ-loaded 3-year-old rabbits.

The C3 expression levels in the retina and in the RPE cells and the choroid are shown in Figure 71 (C) and (D). For both the tissues, the expression level of C3 was confirmed to be increased in the rabbit group given HFD-HQ.

### (11-3) Increase in HTRA1 protein level at time of retinal damage

In order to examine the involvement of HTRA1 in rabbit retinal damage models, vitreous humor was collected from the model rabbits prepared in (11-1), and enzymatically digested with Trypsin/Lys-C Mix (Promega Corp.). Then, a peptide fragment of HTRA1 was quantified using LC (EASY-nLC 1000; Thermo Fisher Scientific Inc.)-MS (TripleTOF 6600; AB Sciex Pte. Ltd). The HTRA1 protein level was found to be increased in the vitreous humor of the rabbits given HFD-HQ (Figure 71(E)). From these results, the hypertrophy of RPE cells and the increased expression of the AMD-related factor C3 and HTRA1 were confirmed, indicating that the rabbit retinal damage models are useful in research on age-related retinal disease.

### (11-4) Retinal protective effect of HTRA1 inhibitor in rabbit retinal damage model

The retinal protective effect of the HTRA1-inhibiting peptide H308 prepared in Example 1 was evaluated using the rabbit models. After 2 months from the start of feeding with HFD-HQ, 50 µL of a 40 mg/mL H308 solution was intravitreally administered to one eye under anesthesia. Normal saline was intravitreally administered to the fellow eye. n = 5 for all groups.

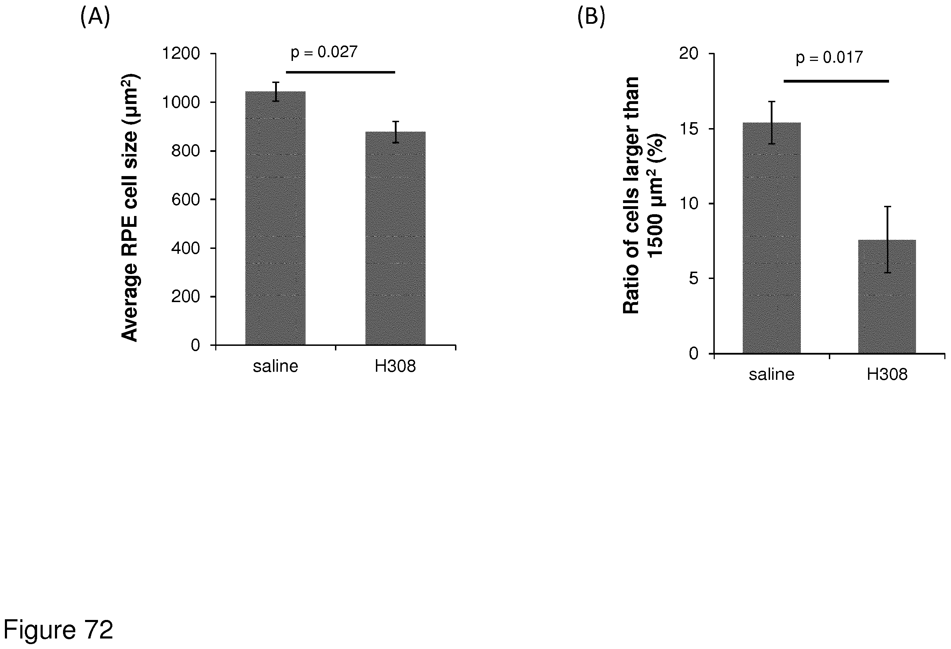

After 4 months from the start of feeding, RPE cell hypertrophy in the model animals was evaluated. The results are shown in Figure 72. The HTRA1 inhibitor exhibited a suppressive effect on the hypertrophy of RPE cells, as seen from both the indicators, i.e., the average area of RPE cells (Figure 72(A)) and the number of hypertrophied RPE cells having a cell area of 1500 µm² or larger (Figure 72(B)). As shown in Figure 71(E), an increase in HTRA1 was confirmed in the vitreous humor of the models, suggesting the involvement of HTRA1 in the process of damage on RPE cells due to HFD-HQ. Thus, the HTRA1-inhibiting peptide is useful as an anti-age-related macular degeneration agent. This test indicated that the HTRA1-inhibiting peptide is useful in the prevention of, particularly, dry age-related macular degeneration.

The presence of the HTRA1-inhibiting peptide was confirmed in the retina of a normal rabbit given the HTRA1-inhibiting peptide, indicating the high tissue penetration of the HTRA1-inhibiting peptide.

Example 12. Suppressive effect of HTRA1-inhibiting peptide in VEGF mRNA induction test using human retinal pigment epithelial cell ARPE-19

ARPE-19 cells were cultured until confluent in 12 mm Transwell with 0.4 µm Pore Polyester Membrane Insert, Sterile (Corning Inc.) under conditions of 37°C and 5% CO₂ using a DMEM/F-12 medium (Wako Pure Chemical Industries, Ltd.) containing 10% fetal bovine serum (FBS) and penicillin-streptomycin (Thermo Fisher Scientific Inc.). Then, the cells were cultured in FBS-free DMEM/F-12 for 5 days. H₂O₂ (final concentration: 500 µM) was added to the upper and lower layers of the chamber, and normal human serum complement (Quidel Corp.) (final concentration: 25%) was added to the upper layer of the chamber. Each of HTRA1 (Example 2-2), inactive HTRA1 protease mutant HTRA1 (S328A) (Example 2-3), or HTRA1-inhibiting peptide H308_D1G_S16A (Example 6) was further added at a final concentration of 1 µM to the upper and lower layers of the chamber. Four hours later, the culture supernatant was removed, and the cells were washed with PBS. Then, mRNA was extracted using SuperPrep(TM) Cell Lysis & RT Kit for qPCR (Toyobo Co., Ltd.) and subjected to a reverse transcription reaction. The mRNA level of VEGF was quantitatively analyzed by TaqMan Gene Expression Assays (Hs000900055_m1 and Hs02786624_g1; Thermo Fisher Scientific Inc.) using a 7900HT Fast Real-Time PCR System (Applied Biosystems, Inc.). GAPDH was used in the correction of the mRNA level.

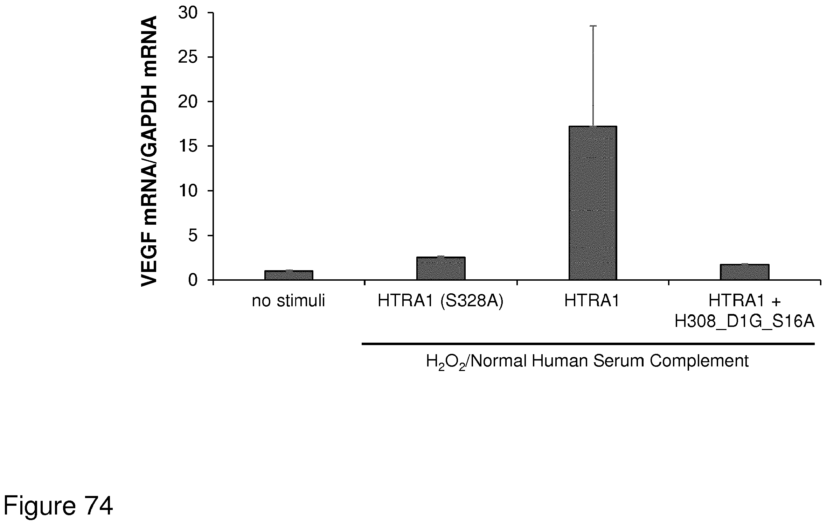

The results are shown in Figure 74. The addition of H₂O₂, normal human serum complement and HTRA1 markedly increased the mRNA level of VEGF as compared with the conditions involving adding H₂O₂, normal human serum complement and inactive HTRA1 mutant HTRA1 (S328A). The co-addition of the HTRA1-inhibiting peptide H308_D1G_S16A was confirmed to suppress the expression of VEGF. VEGF induction from retinal pigment epithelial cells was reportedly important for the pathogenesis of wet age-related macular degeneration (Klettner A. et al., (2009) Graefes Arch Clin Exp Ophthalmol., Vol. 247: p. 1487-1492). In addition, it is considered that such morbid VEGF induction is involved not only in the pathogenesis but in the maintenance of the pathological condition. Thus, the administration of the peptide of the present invention such as HTRA1-inhibiting peptide H308_D1G_S16A is effective for the prevention and treatment of wet age-related macular degeneration.

Example 13. Suppressive effect of HTRA1-inhibiting peptide H308_D1G_S16A on human umbilical vein endothelial cell (HUVEC) migration

### (13-1) HUVEC migration test

HUVEC (Kurabo Industries Ltd.) was cultured for 18 hours under conditions of 37°C and 5% CO₂ in a medium (0.1% BSA-containing serum-free EGM) in which EBM(TM)-2 basal medium (Lonza Walkersville, Inc.) containing 0.1% BSA was supplemented with an EGM(TM)-2 SingleQuots(TM) additive factor set except for serum and VEGF. Then, the cells were adjusted to 4 × 10⁵ cells/mL with 0.1% BSA-containing serum-free EGM. 4 × 10⁵ cells/mL of the HUVEC suspension was added at 50 µL/well to the upper layer of a chamber of a Corning FluoroBlok HTS 96 Well Multiwell Permeable Support System with a 3.0 µm High Density PET Membrane (Corning Inc.) having a gelatin-coated membrane. Then, each sample described below (medium 1, 2 or 3) was added at 210 µL/well to the lower layer of the chamber (n = 3). 0.1% BSA-containing serum-free EGM was added at 50 µL/well to the upper layer of a chamber without the addition of HUVEC, and 0.1% BSA-containing serum-free EGM was added at 210 µL/well to the lower layer of the chamber (n = 3).
Medium 1; 0.1% BSA-containing serum-free EGM
Medium 2; EBM(TM)-2 medium supplemented with all EGM(TM)-2 SingleQuots(TM) additive factors (EGM growth medium)
Medium 3; EGM growth medium containing 300 nM H308_D1G_S16A

A FluoroBlok HTS 96 Well Multiwell Support System supplemented with the cells and the sample was incubated for 2 hours under conditions of 37°C and 5% CO₂. HUVEC migrated to the lower layer was washed with PBS and then stained for 15 minutes with 0.1% BSA-containing serum-free EGM containing 4 µg/mL Calcein-AM (Thermo Fisher Scientific Inc.). Then, the medium was replaced with PBS. The fluorescence intensity (excitation wavelength/fluorescence wavelength: 485 nm/535 nm) of each well was measured using a plate reader (ARVO-MX, PerkinElmer, Inc.), and the migrated cells were counted for each well according to the following expression. Migrated cells = Mean fluorescence intensity of wells containing HUVEC (n = 3) - Mean fluorescence intensity of blank wells (n = 3).

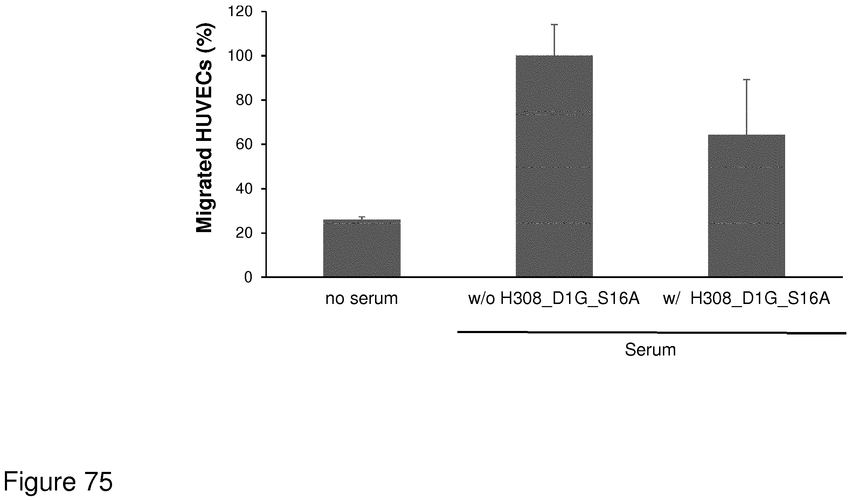

The results are shown in Figure 75. The HTRA1-inhibiting peptide H308_D1G_S16A was confirmed to have a suppressive effect on the migration of HUVEC induced in the serum-containing medium. Accordingly, the peptide of the present invention was found to exhibit a suppressive effect on angiogenesis, a feature of wet age-related macular degeneration.

### Example 14. Retinal protective effect of HTRA1-inhibiting peptide in rabbit retinal damage model (part 2)

HTRA1-inhibiting peptide H308 prepared in Example 1 or one of the three HTRA1-inhibiting peptides prepared in Example 6 is evaluated for its therapeutic effect on retinal damage using the rabbit retinal damage models prepared and evaluated in Examples (11-1) to (11-3). 50 µL of a 40 mg/mL inhibiting peptide solution is intravitreally administered to one eye of each model animal under anesthesia, and the animal is raised for 2 months. Normal saline is intravitreally administered to the fellow eye. n = 5 for all groups.

Increase in RPE cell area or increase in RPE cell count should be found in the normal saline administration group, whereas the increase in RPE cell area or the increase in RPE cell count should be suppressed in the HTRA1-inhibiting peptide administration group. Thus, the HTRA1-inhibiting peptide can be confirmed to be useful as an anti-age-related macular degeneration agent. In this Example, the HTRA1-inhibiting peptide can be confirmed to be useful in the treatment of dry age-related macular degeneration, in particular.

### Industrial Applicability

The peptide provided by the present invention, and a pharmaceutical composition comprising the peptide are useful in the treatment or prevention, etc. of age-related macular degeneration and the like.

### Sequence Listing Free Text

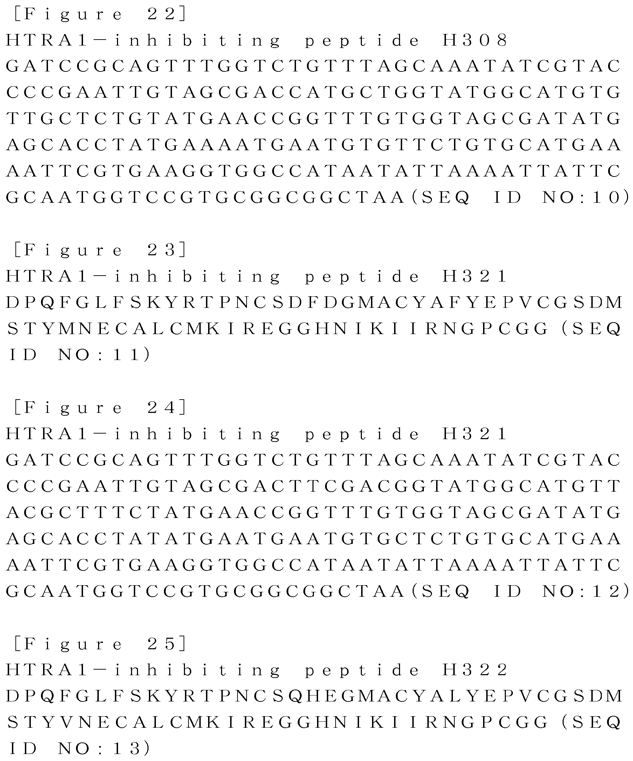

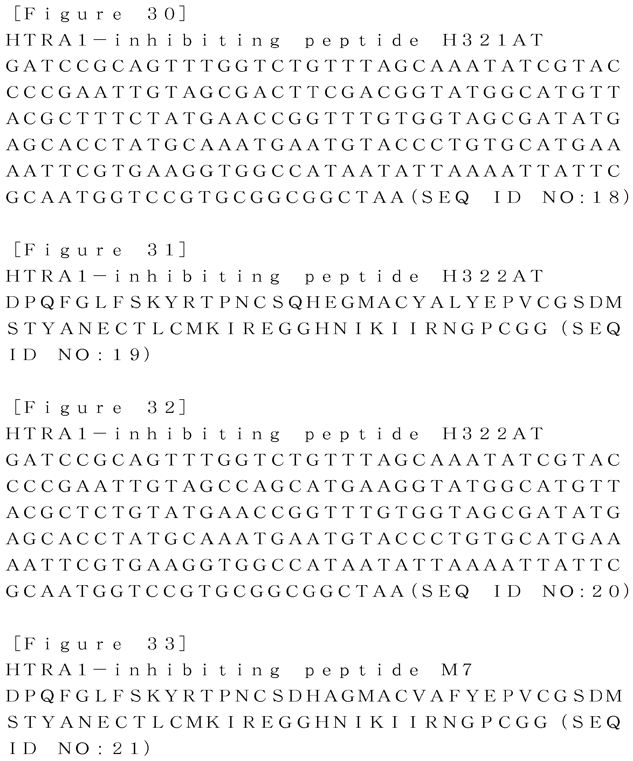

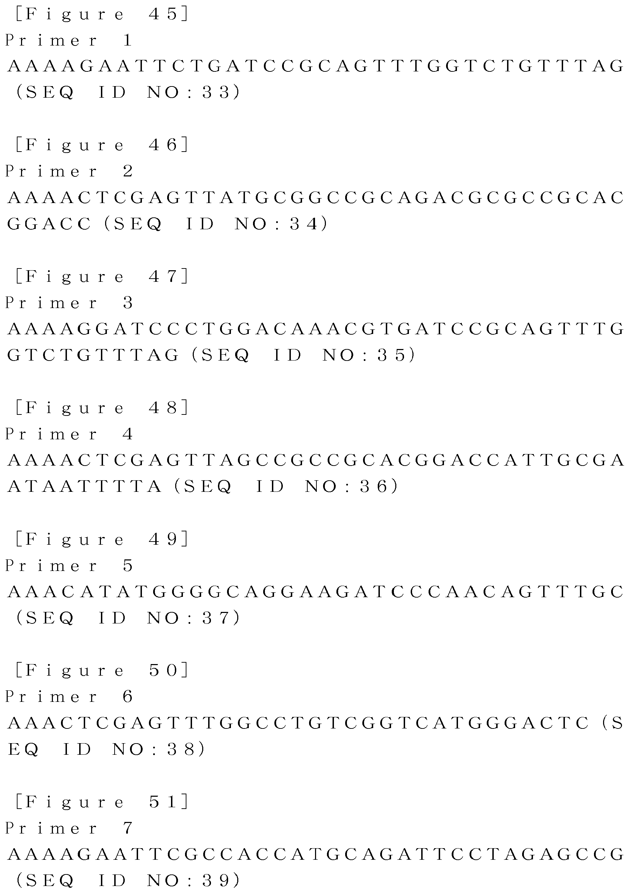

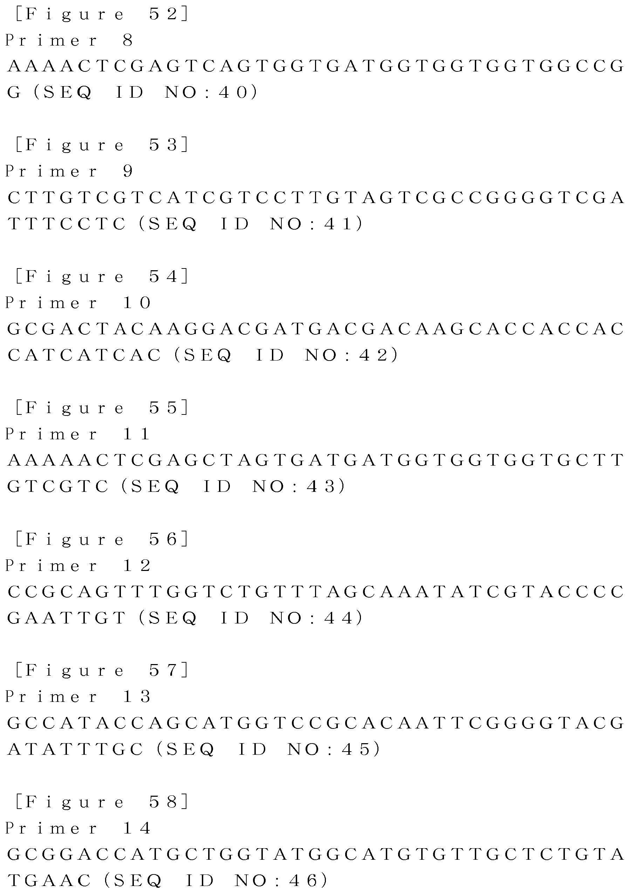

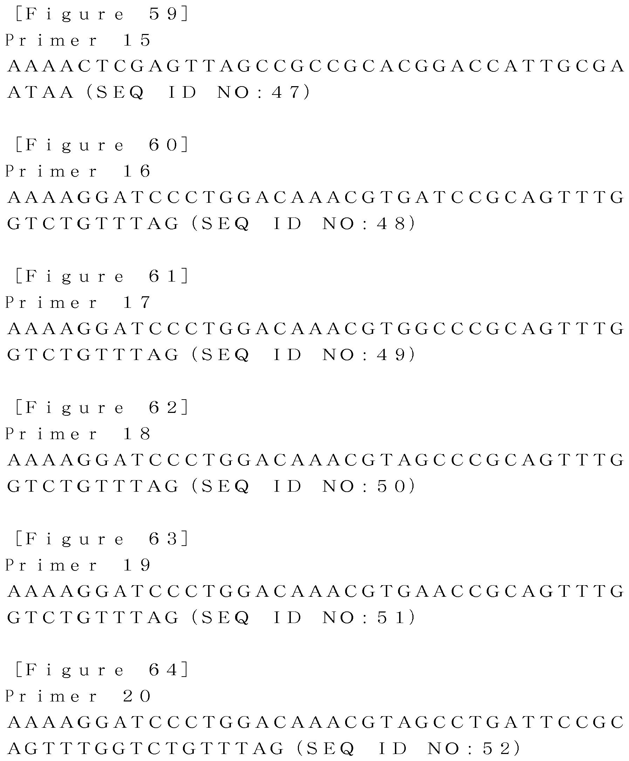

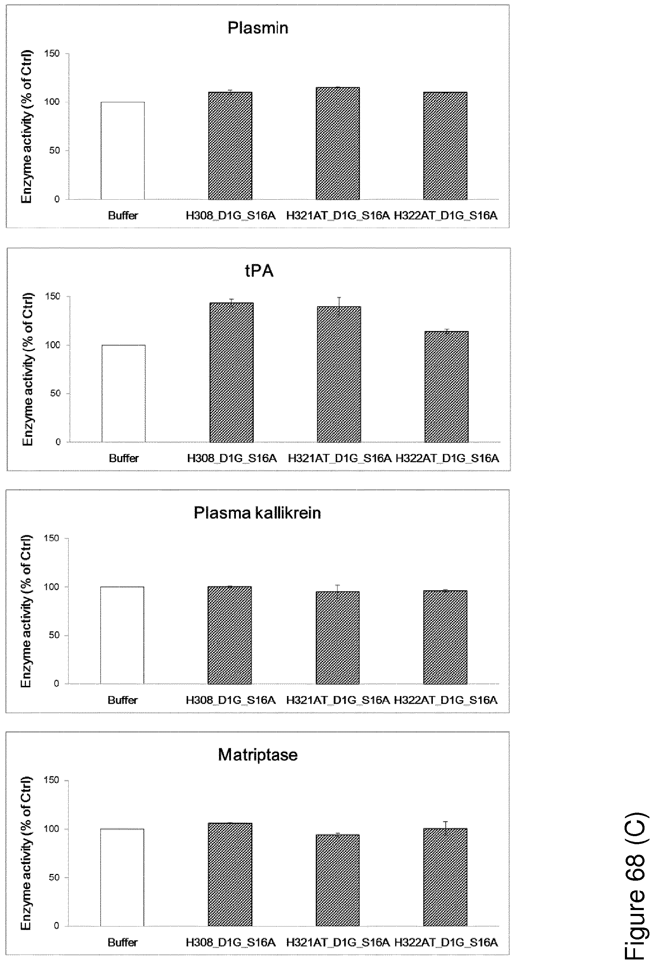

SEQ ID NO: 1 - Amino acid sequence of human SPINK2 (Figure 13)
SEQ ID NO: 2 - Nucleotide sequence encoding amino acid sequence of human SPINK2 (Figure 14)
SEQ ID NO: 3 - Amino acid sequence of peptide H218 (Figure 15)
SEQ ID NO: 4 - Nucleotide sequence encoding amino acid sequence of peptide H218 (Figure 16)
SEQ ID NO: 5 - Amino acid sequence of peptide H223 (Figure 17)
SEQ ID NO: 6 - Nucleotide sequence encoding amino acid sequence of peptide H223 (Figure 18)
SEQ ID NO: 7 - Amino acid sequence of peptide H228 (Figure 19)
SEQ ID NO: 8 - Nucleotide sequence encoding amino acid sequence of peptide H228 (Figure 20)
SEQ ID NO: 9 - Amino acid sequence of peptide H308 (Figure 21)
SEQ ID NO: 10 - Nucleotide sequence encoding amino acid sequence of peptide H308 (Figure 22)
SEQ ID NO: 11 - Amino acid sequence of peptide H321 (Figure 23)
SEQ ID NO: 12 - Nucleotide sequence encoding amino acid sequence of peptide H321 (Figure 24)
SEQ ID NO: 13 - Amino acid sequence of peptide H322 (Figure 25)
SEQ ID NO: 14 - Nucleotide sequence encoding amino acid sequence of peptide H322 (Figure 26)
SEQ ID NO: 15 - Amino acid sequence of peptide derivative H308AT (Figure 27)
SEQ ID NO: 16 - Nucleotide sequence encoding amino acid sequence of peptide derivative H308AT (Figure 28)
SEQ ID NO: 17 - Amino acid sequence of peptide derivative H321AT (Figure 29)
SEQ ID NO: 18 -Nucleotide sequence encoding amino acid sequence of peptide derivative H321AT (Figure 30)
SEQ ID NO: 19 - Amino acid sequence of peptide derivative H322AT (Figure 31)
SEQ ID NO: 20 - Nucleotide sequence encoding amino acid sequence of peptide derivative H322AT (Figure 32)
SEQ ID NO: 21 - Amino acid sequence of peptide M7 (Figure 33)
SEQ ID NO: 22 - Nucleotide sequence encoding amino acid sequence of peptide M7 (Figure 34)
SEQ ID NO: 23 - Amino acid sequence of peptide derivative H308_S16A (Figure 35)
SEQ ID NO: 24 - Amino acid sequence of peptide derivative H308_D1G_S16A (Figure 36)
SEQ ID NO: 25 - Amino acid sequence of peptide derivative H308_D1S_S16A (Figure 37)
SEQ ID NO: 26 - Amino acid sequence of peptide derivative H308_D1E_S16A (Figure 38)
SEQ ID NO: 27 - Amino acid sequence of peptide derivative H308_D1SLI_S16A (Figure 39)
SEQ ID NO: 28 - Amino acid sequence of peptide derivative H321AT_D1G_S16A (Figure 40)
SEQ ID NO: 29 - Amino acid sequence of peptide derivative H322AT_D1G_S16A (Figure 41)
SEQ ID NO: 30 - General formula of HTRA1-inhibiting peptide (Figure 42)
SEQ ID NO: 31 - Amino acid sequence consisting of S tag and linker (Figure 43)
SEQ ID NO: 32 - Amino acid sequence of C-terminal hexamer (Figure 44)
SEQ ID NO: 33 - Nucleotide sequence of primer 1 (Figure 45)
SEQ ID NO: 34 - Nucleotide sequence of primer 2 (Figure 46)
SEQ ID NO: 35 - Nucleotide sequence of primer 3 (Figure 47)
SEQ ID NO: 36 - Nucleotide sequence of primer 4 (Figure 48)
SEQ ID NO: 37 - Nucleotide sequence of primer 5 (Figure 49)
SEQ ID NO: 38 - Nucleotide sequence of primer 6 (Figure 50)
SEQ ID NO: 39 - Nucleotide sequence of primer 7 (Figure 51)
SEQ ID NO: 40 - Nucleotide sequence of primer 8 (Figure 52)
SEQ ID NO: 41 - Nucleotide sequence of primer 9 (Figure 53)
SEQ ID NO: 42 - Nucleotide sequence of primer 10 (Figure 54)
SEQ ID NO: 43 - Nucleotide sequence of primer 11 (Figure 55)
SEQ ID NO: 44 - Nucleotide sequence of primer 12 (Figure 56)
SEQ ID NO: 45 - Nucleotide sequence of primer 13 (Figure 57)
SEQ ID NO: 46 - Nucleotide sequence of primer 14 (Figure 58)
SEQ ID NO: 47 - Nucleotide sequence of primer 15 (Figure 59)
SEQ ID NO: 48 - Nucleotide sequence of primer 16 (Figure 60)
SEQ ID NO: 49 - Nucleotide sequence of primer 17 (Figure 61)
SEQ ID NO: 50 - Nucleotide sequence of primer 18 (Figure 62)
SEQ ID NO: 51 - Nucleotide sequence of primer 19 (Figure 63)
SEQ ID NO: 52 - Nucleotide sequence of primer 20 (Figure 64)
SEQ ID NO: 53 - Amino acid sequence of human HTRA1 (full) (Figure 65)
SEQ ID NO: 54 - Amino acid sequence of H2-Opt (Figure 8)
SEQ ID NO: 55 - Nucleotide sequence of primer 21 (Figure 76)
SEQ ID NO: 56 - Nucleotide sequence of primer 22 (Figure 77)

## Claims

1. A SPINK2 mutant peptide which inhibits the protease activity of human HTRA1 wherein the peptide comprises:
(a) an amino acid sequence shown in any one of SEQ ID NOs: 24, 23, 25 to 27, 9, 15, 3, 5, 7, 11, 13, 17, 19, 21, 28 and 29 (Figures 36, 35, 37 to 39, 21, 27, 15, 17, 19, 23, 25, 29, 31, 33, 40 and 41); or
(b) an amino acid sequence which is derived from the amino acid sequence of (a) by the deletion of 1 to 15 amino acids.

2. A polynucleotide comprising a nucleotide sequence encoding an amino acid sequence comprised in the peptide according to claim 1.

3. A vector comprising the polynucleotide according to claim 2.

4. A cell comprising the polynucleotide according to claim 2 or the vector according to claim 3 or producing the peptide according to claim 1.

5. A method for producing a SPINK2 mutant peptide which inhibits the protease activity of HTRA1, comprising the following steps (i) and (ii):
(i) culturing the cell according to claim 4; and
(ii) recovering the SPINK2 mutant peptide from the culture.

6. A conjugate comprising the peptide according to claim 1 or the peptide produced according to claim 5 linked to an additional moiety.

7. The conjugate according to claim 6, which is a polypeptide.

8. The method according to claim 5, wherein recovering the SPINK2 mutant peptide comprises an affinity purification using an antibody, or functional fragment thereof, which binds to the peptide.

9. A composition comprising the peptide according to claim 1, the peptide produced according to claim 5, the polynucleotide according to claim 2, the vector according to claim 3, the cell according to claim 4, and/or the conjugate according to claim 6 or 7.

10. A pharmaceutical composition comprising the peptide according to claim 1, the peptide produced according to claim 5, the polynucleotide according to claim 2, the vector according to claim 3, the cell according to claim 4, and/or the conjugate according to claim 6 or 7.

11. The pharmaceutical composition according to claim 10, for use in the treatment or prevention of a HTRA1-related disease, wherein the HTRA1-related disease is one or two or more diseases selected from the group consisting of wet age-related macular degeneration, dry age-related macular degeneration, geographic atrophy, diabetic retinopathy, retinopathy of prematurity, polypoidal choroidal vasculopathy, rheumatoid arthritis, and osteoarthritis.

12. The pharmaceutical composition according to claim 10 or the pharmaceutical composition for use according to claim 11, which comprises one or two or more additional medicaments.

13. The pharmaceutical composition according to claim 10 or the pharmaceutical composition for use according to claim 11, which is used in combination with one or two or more additional medicaments.

14. The pharmaceutical composition according to any one of claims 10, 12 or 13, or the pharmaceutical composition for use according to any one of claims 11 to 13, which is a retinal protection agent.

## Patentansprüche

1. SPINK2-Mutantenpeptid, das die Proteaseaktivität von menschlichem HTRA1 hemmt, wobei das Peptid umfasst:
(a) eine Aminosäuresequenz, dargestellt in einer von SEQ ID NOs: 24, 23, 25 bis 27, 9, 15, 3, 5, 7, 11, 13, 17, 19, 21, 28 und 29 (Figuren 36, 35, 37 bis 39, 21, 27, 15, 17, 19, 23, 25, 29, 31, 33, 40 und 41); oder
(b) eine Aminosäuresequenz, die von der Aminosäuresequenz aus (a) durch die Deletion von 1 bis 15 Aminosäuren abgeleitet ist.

2. Polynukleotid, das eine Nukleotidsequenz umfasst, die eine Aminosäuresequenz codiert, die im Peptid nach Anspruch 1 umfasst ist.

3. Vektor, umfassend das Polynukleotid nach Anspruch 2.

4. Zelle, die das Polynukleotid nach Anspruch 2 oder den Vektor nach Anspruch 3 umfasst oder die das Peptid nach Anspruch 1 produziert.

5. Verfahren zur Herstellung eines SPINK2-Mutantenpeptids, das die Proteaseaktivität von HTRA1 hemmt, umfassend die folgenden Schritte (i) und (ii):
(i) Kultivieren der Zelle nach Anspruch 4; und
(ii) Gewinnen des SPINK2-Mutantenpeptids aus der Kultur.

6. Konjugat, umfassend das Peptid nach Anspruch 1 oder das nach Anspruch 5 hergestellte Peptid, verknüpft mit einem zusätzlichen Teil.

7. Konjugat nach Anspruch 6, das ein Polypeptid ist.

8. Verfahren nach Anspruch 5, wobei Gewinnen des SPINK2-Mutantenpeptids eine Affinitätsreinigung unter Verwendung eines Antikörpers, oder eines funktionellen Fragments davon, der bzw. das an das Peptid bindet, umfasst.

9. Zusammensetzung, umfassend das Peptid nach Anspruch 1, das nach Anspruch 5 hergestellte Peptid, das Polynukleotid nach Anspruch 2, den Vektor nach Anspruch 3, die Zelle nach Anspruch 4, und/oder das Konjugat nach Anspruch 6 oder 7.

10. Pharmazeutische Zusammensetzung, umfassend das Peptid nach Anspruch 1, das nach Anspruch 5 hergestellte Peptid, das Polynukleotid nach Anspruch 2, den Vektor nach Anspruch 3, die Zelle nach Anspruch 4, und/oder das Konjugat nach Anspruch 6 oder 7.

11. Pharmazeutische Zusammensetzung nach Anspruch 10, zur Verwendung bei der Behandlung oder Verhütung einer mit HTRA1 zusammenhängenden Erkrankung, wobei die mit HTRA1 zusammenhängende Erkrankung in einer oder zwei oder mehr Erkrankungen besteht, die aus der Gruppe ausgewählt sind, bestehend aus feuchter altersbedingter Makuladegeneration, trockener altersbedingter Makuladegeneration, geographischer Atrophie, diabetischer Retinopathie, Frühgeborenen-Retinopathie, polypoider choroidaler Vaskulopathie, rheumatoider Arthritis und Arthrose.

12. Pharmazeutische Zusammensetzung nach Anspruch 10 oder pharmazeutische Zusammensetzung zur Verwendung nach Anspruch 11, die ein oder zwei oder mehr zusätzliche Medikamente umfasst.

13. Pharmazeutische Zusammensetzung nach Anspruch 10 oder pharmazeutische Zusammensetzung zur Verwendung nach Anspruch 11, die in Kombination mit einem oder zwei oder mehr zusätzlichen Medikamenten verwendet wird.

14. Pharmazeutische Zusammensetzung nach einem der Ansprüche 10, 12 oder 13, oder pharmazeutische Zusammensetzung zur Verwendung nach einem der Ansprüche 11 bis 13, die ein Netzhautschutzmittel ist.

## Revendications

1. Peptide SPINK2 mutant qui inhibe l'activité de protéase d'une HTRA1 humaine où le peptide comprend :
(a) une séquence d'acides aminés représentée dans l'une quelconque des SEQ ID NOs : 24, 23, 25 à 27, 9, 15, 3, 5, 7, 11, 13, 17, 19, 21, 28 et 29 (Figures 36, 35, 37 à 39, 21, 27, 15, 17, 19, 23, 25, 29, 31, 33, 40 et 41) ; ou
(b) une séquence d'acides aminés qui est dérivée de la séquence d'acides aminés de (a) par la délétion de 1 à 15 acides aminés.

2. Polynucléotide comprenant une séquence de nucléotides codant pour une séquence d'acides aminés comprise dans le peptide selon la revendication 1.

3. Vecteur comprenant le polynucléotide selon la revendication 2.

4. Cellule comprenant le polynucléotide selon la revendication 2 ou le vecteur selon la revendication 3 ou produisant le peptide selon la revendication 1.

5. Procédé pour la production d'un peptide SPINK2 mutant qui inhibe l'activité de protéase de HTRA1, comprenant les étapes (i) et (ii) suivantes :
(i) culture de la cellule selon la revendication 4 ; et
(ii) récupération du peptide SPINK2 mutant à partir de la culture.

6. Conjugué comprenant le peptide selon la revendication 1 ou le peptide produit selon la revendication 5 lié à une fraction supplémentaire.

7. Conjugué selon la revendication 6, qui est un polypeptide.

8. Procédé selon la revendication 5, dans lequel la récupération du peptide SPINK2 mutant comprend une purification par affinité en utilisant un anticorps, ou un fragment fonctionnel de celui-ci, qui se lie au peptide.

9. Composition comprenant le peptide selon la revendication 1, le peptide produit selon la revendication 5, le polynucléotide selon la revendication 2, le vecteur selon la revendication 3, la cellule selon la revendication 4, et/ou le conjugué selon la revendication 6 ou 7.

10. Composition pharmaceutique comprenant le peptide selon la revendication 1, le peptide produit selon la revendication 5, le polynucléotide selon la revendication 2, le vecteur selon la revendication 3, la cellule selon la revendication 4 et/ou le conjugué selon la revendication 6 ou 7.

11. Composition pharmaceutique selon la revendication 10, pour utilisation dans le traitement ou la prévention d'une maladie liée à HTRA1, où la maladie liée à HTRA1 est une ou deux maladies ou plus choisies dans le groupe constitué de : une dégénérescence maculaire liée à l'âge humide, une dégénérescence maculaire liée à l'âge sèche, une atrophie géographique, une rétinopathie diabétique, une rétinopathie de prématurité, une vasculopathie polypoïdale choroïdienne, une polyarthrite rhumatoïde et une ostéoarthrite.

12. Composition pharmaceutique selon la revendication 10 ou composition pharmaceutique pour utilisation selon la revendication 11, qui comprend un ou deux médicaments supplémentaires ou plus.

13. Composition pharmaceutique selon la revendication 10 ou composition pharmaceutique pour utilisation selon la revendication 11, qui est utilisée en combinaison avec un ou deux médicaments supplémentaires ou plus.

14. Composition pharmaceutique selon l'une quelconque des revendications 10, 12 ou 13 ou composition pharmaceutique pour utilisation selon l'une quelconque des revendications 11 à 13, qui est un agent de protection rétinien.
